# EUROPEAN PATENT SPECIFICATION

(11) **EP 3 458 457 B1**
(45) Date of publication and mention of the grant of the patent: **28.10.2020**
(21) Application number: 17799568.5
(22) Date of filing: 12.04.2017
(51) Int. Cl.: C07D 487/04, C09K 11/06, H01L 51/54, H05B 3/14

(54) **ORGANIC ELECTROLUMINESCENT COMPOUND, ORGANIC ELECTROLUMINESCENT MATERIAL AND ORGANIC ELECTROLUMINESCENT DEVICE COMPRISING THE SAME**
ORGANISCHE ELEKTROLUMINESZENTE VERBINDUNG, ORGANISCHES ELEKTROLUMINESZENTES MATERIAL UND ORGANISCHE ELEKTROLUMINESZENTE VORRICHTUNG DAMIT
COMPOSÉ ORGANIQUE ÉLECTROLUMINESCENT, MATÉRIAU ORGANIQUE ÉLECTROLUMINESCENT ET DISPOSITIF ORGANIQUE ÉLECTROLUMINESCENT LES COMPRENANT

(30) Priority: 17.05.2016 KR 20160060247; 03.02.2017 KR 20170015372
(43) Date of publication of application: 27.03.2019
(73) Proprietor: Rohm And Haas Electronic Materials Korea Ltd., Cheonan-si, Chungcheongnam-do 31093 (KR)
(72) Inventor: LEE, Hyo-Jung, Hwaseong-si Gyeonggi-do 18449 (KR); LIM, Young-Mook, Hwaseong-si Gyeonggi-do 18449 (KR); KIM, Bitnari, Hwaseong-si Gyeonggi-do 18449 (KR)
(74) Representative: Houghton, Mark Phillip
(86) International application number: PCT/KR2017/003959
(87) International publication number: WO 2017/200210

(56) References cited:
- WO-A1-2015/046916
- WO-A1-2015/142036
- WO-A1-2015/160224
- WO-A1-2015/174738
- WO-A1-2015/178732
- WO-A1-2016/013867
- WO-A1-2016/148390
- WO-A2-2010/107244
- KR-A- 20140 006 708
- KR-A- 20140 009 019

## Description

### Technical Field

The present disclosure relates to an organic electroluminescent compound, an organic electroluminescent material and an organic electroluminescent device comprising the same.

### Background Art

Among display devices, an electroluminescent device (EL device) is a self-light-emitting display device which has advantages in that it provides a wider viewing angle, a greater contrast ratio, and a faster response time. The first organic EL device was developed by Eastman Kodak in 1987, by using small aromatic diamine molecules and aluminum complexes as materials for forming a light-emitting layer [Appl. Phys. Lett. 51, 913, 1987].

The most important factor determining luminous efficiency in an organic electroluminescent device is light-emitting materials. Until now, fluorescent materials have been widely used as the light-emitting material. However, in view of electroluminescent mechanisms, since phosphorescent light-emitting materials theoretically enhance luminous efficiency by four (4) times compared to fluorescent light-emitting materials, phosphorescent light-emitting materials have been widely researched. Iridium(III) complexes have been widely known as phosphorescent light-emitting materials, including bis(2-(2'-benzothienyl)-pyridinato-N,C-3')iridium(acetylacetonate) [(acac)Ir(btp)₂], tris(2-phenylpyridine)iridium [Ir(ppy)₃] and bis(4,6-difluorophenylpyridinato-N,C2)picolinato iridium (Firpic) as red-, green- and blue-emitting materials, respectively.

In conventional technology, 4,4'-N,N'-dicarbazol-biphenyl (CBP) is the most widely known phosphorescent host material. Recently, Pioneer (Japan) et al., developed a high performance organic electroluminescent device using bathocuproine (BCP) and aluminum(III) bis(2-methyl-8-quinolinate)(4-phenylphenolate) (BAlq), etc., as host materials, which were known as hole blocking materials.

Although these materials provide good luminous characteristics, they have the following disadvantages: (1) Due to their low glass transition temperature and poor thermal stability, their degradation may occur during a high-temperature deposition process in a vacuum, and the lifespan of the device may be shortened. (2) The power efficiency of the organic electroluminescent device is given by [(π/voltage) × current efficiency], and the power efficiency is inversely proportional to the voltage. Although the organic electroluminescent device comprising phosphorescent host materials provides higher current efficiency (cd/A) than one comprising fluorescent materials, a significantly high driving voltage is necessary. Thus, there is no merit in terms of power efficiency (lm/W). (3) Also, the operational lifespan of the organic electroluminescent device is short, and luminous efficiency is still necessary to improve. Accordingly, the materials constituting the organic layer in the device, in particular a host or a dopant constituting the light-emitting material, should be selected appropriately in order to realize the excellent characteristics of the organic EL device.

Korean Patent No. 1313730 discloses an organic electroluminescent device using a substituted indolocarbazole compound as a host material.

Korean Patent No. 1529164 discloses an organic electroluminescent device using a compound, in which a pyridine, pyrimidine or triazine substituted with phenyls is linked to a benzoindolocarbazole via two consecutive naphthylenes as a linker, or a pyridine, pyrimidine or triazine substituted with phenyls is linked to an indolocarbazole via a naphthylene as a linker, only as a phosphorescent green host material.

Korean Patent Application Laid-Open No. 2015-0077513 discloses a compound in which an indolocarbazole derivative is linked to a quinazoline or a quinoxaline via an arylene as a linker.

The International Publications WO 2015/142036 A1 and WO 2010/107244 A2 disclose further materials with indolocarbazole groups linked to triazine moieties.

### Disclosure of Invention

### Technical Problem

The object of the present disclosure is firstly, to provide an organic electroluminescent compound capable of improving lifespan properties of an organic electroluminescent device, secondly, to provide an organic electroluminescent material comprising the organic electroluminescent compound, and thirdly, to provide an organic electroluminescent device having improved lifespan properties, comprising the organic electroluminescent compound.

### Solution to Problem

As a result of intensive studies to solve the technical problem above, the present inventors found that the above objective can be achieved by an organic electroluminescent compound represented by the following formula 1: 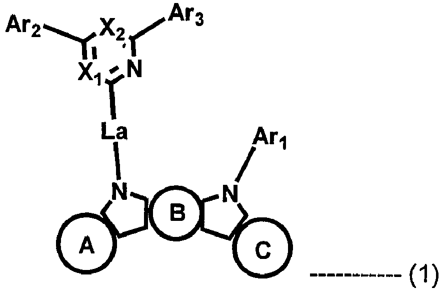 wherein
X₁ and X₂, each independently, represent N or CH, with the proviso, at least one of X ₁ and X₂ represents N,
La represents a substituted or unsubstituted divalent (C10-C30) aromatic ring, which is one condensed ring,
A ring and C ring, each independently, represent a substituted or unsubstituted benzene ring, or a substituted or unsubstituted naphthalene ring, with the proviso, at least one of A ring and C ring represents a substituted or unsubstituted naphthalene ring,
B ring represents a substituted or unsubstituted benzene ring,
Ar₁, Ar₂ and Ar₃, each independently, represent hydrogen, deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino, and
the heteroaryl contains at least one heteroatom selected from B, N, O, S, Si, and P.

### Advantageous Effects of Invention

By using the organic electroluminescent compound of the present disclosure, it is possible to provide an organic electroluminescent device having long driving lifespan.

### Mode for the Invention

Hereinafter, the present disclosure will be described in detail. However, the following description is intended to explain the disclosure, and is not meant in any way to restrict the scope of the disclosure.

The term "an organic electroluminescent compound" in the present disclosure means a compound that may be used in an organic electroluminescent device, and may be comprised in any layers constituting an organic electroluminescent device, if necessary.

The term "an organic electroluminescent material" in the present disclosure means a material that may be used in an organic electroluminescent device, and may comprise at least one compound. If necessary, the organic electroluminescent material may be comprised in any layers constituting an organic electroluminescent device. For example, the organic electroluminescent material may be a hole injection material, a hole transport material, a hole auxiliary material, a light-emitting auxiliary material, an electron blocking material, a light-emitting material, an electron buffer material, a hole blocking material, an electron transport material, an electron injection material, etc.

In formula 1, La represents a substituted or unsubstituted divalent (C10-C30) aromatic ring, which is one condensed ring, preferably, a substituted or unsubstituted divalent (C10-C25) aromatic ring, and more preferably, an unsubstituted divalent (C10-C18) aromatic ring. Specifically, La may be a naphthylene, an anthracenylene, a triphenylenylene, a dimethylbenzofluorenylene, or phenanthrenylene, in which the naphthylene may be 1,3-naphthylene, 1,4-naphthylene, 1,5-naphthylene, 1,6-naphthylene, or 2,6-naphthylene. According to one embodiment of the present disclosure, the substituent of the substituted divalent (C10-C30) aromatic ring may be not a condensed aromatic ring.

In formula 1, A ring and C ring, each independently, represent a substituted or unsubstituted benzene ring, or a substituted or unsubstituted naphthalene ring, with the proviso, at least one of A ring and C ring represents a substituted or unsubstituted naphthalene ring. Preferably, one of A ring and C ring represents a substituted or unsubstituted naphthalene ring, and the other represents a substituted or unsubstituted benzene ring.

In formula 1, B ring represents a substituted or unsubstituted benzene ring, and preferably, an unsubstituted benzene ring.

In formula 1, Ar₁, Ar₂ and Ar₃, each independently, represent hydrogen, deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino, preferably, a substituted or unsubstituted (C6-C25)aryl, or a substituted or unsubstituted (5- to 25-membered)heteroaryl, and more preferably, a substituted or unsubstituted (C6-C18)aryl, or a substituted or unsubstituted (5- to 18-membered)heteroaryl. Also, Ar₁, Ar₂ and Ar₃, each independently, may represent an unsubstituted (C6-C15)aryl, or an unsubstituted (5- to 15-membered)heteroaryl, for example, an unsubstituted phenyl, an unsubstituted naphthyl, an unsubstituted biphenyl, or an unsubstituted pyridyl.

In formula 1, the heteroaryl contains at least one heteroatom selected from B, N, O, S, Si, and P, preferably, at least one heteroatom selected from N, O and S, and more preferably, at least one N.

According to one embodiment of the present disclosure, formula 1 may be represented by any one of the following formulas 2 to 5:

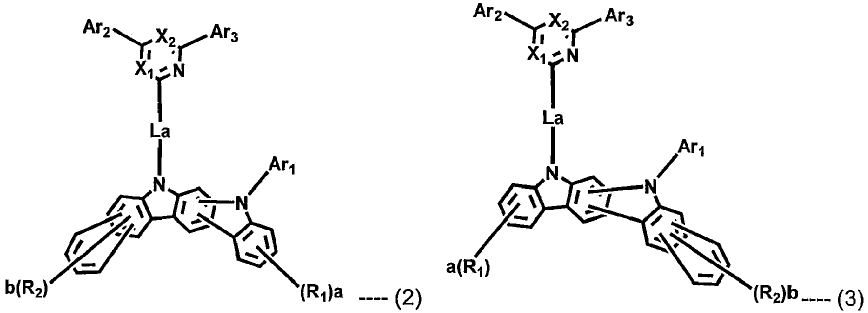

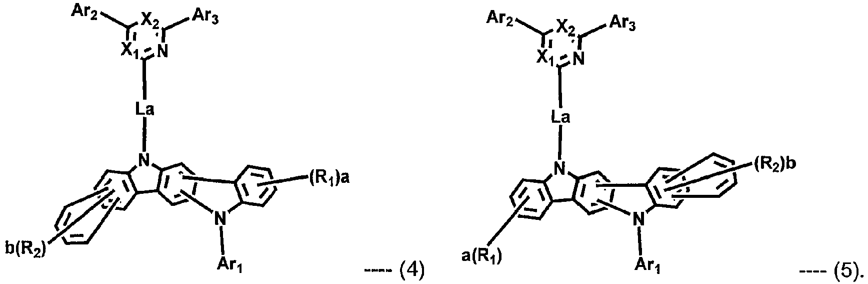

According to another embodiment of the present disclosure, formula 1 may be represented by any one of the following formulas 6 to 17:

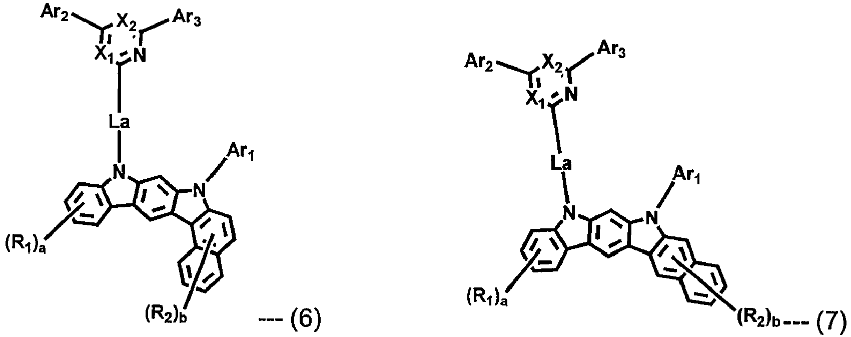

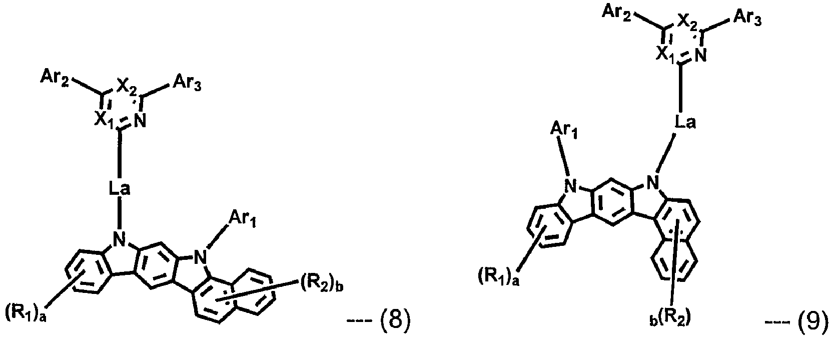

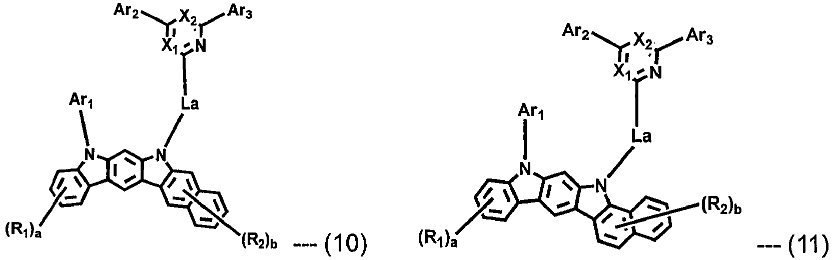

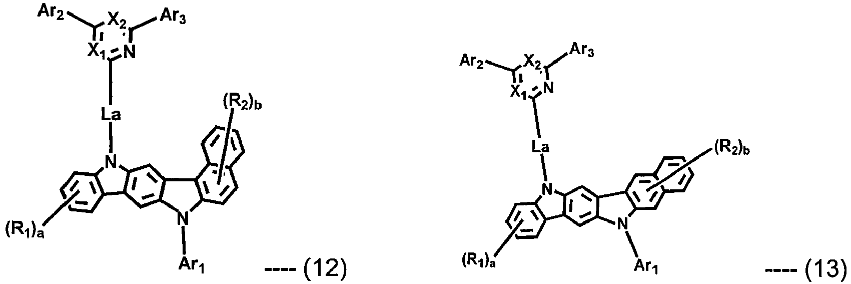

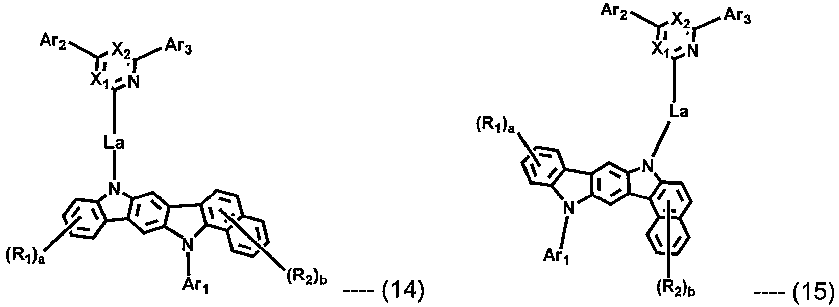

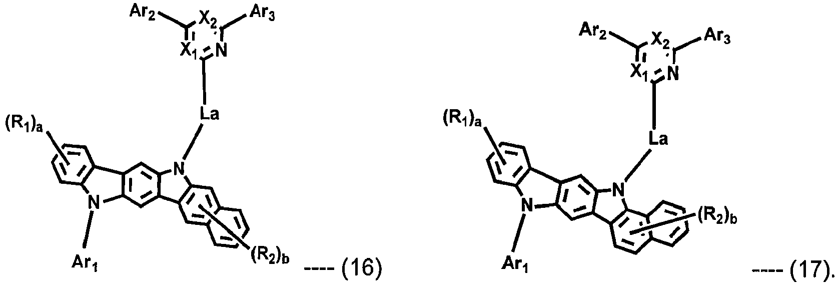

In formulas 2 to 17, X₁, X₂, La, Ar₁, Ar₂, and Ar₃ are as defined in formula 1.

In formulas 2 to 17, R₁ and R₂, each independently, represent deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to adjacent R₁ and R₂ to form a substituted or unsubstituted, mono- or polycyclic, (C3-C30) alicyclic or aromatic ring, or a combination thereof, whose carbon atom(s) may be replaced with at least one heteroatom selected from nitrogen, oxygen, and sulfur; preferably, represent a substituted or unsubstituted (C1-C20)alkyl, a substituted or unsubstituted (C6-C25)aryl, or a substituted or unsubstituted (5- to 25-membered)heteroaryl, more preferably, represent a substituted or unsubstituted (C6-C18)aryl, or a substituted or unsubstituted (5- to 18-membered)heteroaryl, even more preferably, represent an unsubstituted (C6-C15)aryl, and for example, may be an unsubstituted phenyl. The heteroaryl contains at least one heteroatom selected from B, N, O, S, Si, and P.

In formulas 2 to 17, a represents an integer of 0 to 4, and b represents an integer of 0 to 6; where if a and b, each independently, represent an integer of 2 or more, each of R₁ and R₂ may be the same or different. Preferably, a and b, each independently, represent 0 or 1.

Herein, the term "(C1-C30)alkyl" is meant to be a linear or branched alkyl having 1 to 30 carbon atoms constituting the chain, in which the number of carbon atoms is preferably 1 to 20, and more preferably 1 to 10. The above alkyl may include methyl, ethyl, n-propyl, isopropyl, n-butyl, isobutyl, tert-butyl, etc. The term "(C2-C30)alkenyl" is meant to be a linear or branched alkenyl having 2 to 30 carbon atoms constituting the chain, in which the number of carbon atoms is preferably 2 to 20, and more preferably 2 to 10. The above alkenyl may include vinyl, 1-propenyl, 2-propenyl, 1-butenyl, 2-butenyl, 3-butenyl, 2-methylbut-2-enyl, etc. The term "(C2-C30)alkynyl" is meant to be a linear or branched alkynyl having 2 to 30 carbon atoms constituting the chain, in which the number of carbon atoms is preferably 2 to 20, and more preferably 2 to 10. The above alkynyl may include ethynyl, 1-propynyl, 2-propynyl, 1-butynyl, 2-butynyl, 3-butynyl, 1-methylpent-2-ynyl, etc. The term "(C3-C30)cycloalkyl" is a mono- or polycyclic hydrocarbon having 3 to 30 ring backbone carbon atoms, in which the number of carbon atoms is preferably 3 to 20, and more preferably 3 to 7. The above cycloalkyl may include cyclopropyl, cyclobutyl, cyclopentyl, cyclohexyl, etc. The term "(3- to 7- membered) heterocycloalkyl" is a cycloalkyl having 3 to 7, preferably 5 to 7, ring backbone atoms, including at least one heteroatom selected from B, N, O, S, Si, and P, and preferably O, S, and N. The above heterocycloalkyl may include tetrahydrofuran, pyrrolidine, thiolan, tetrahydropyran, etc. The term "(C6-C30)aryl" is a monocyclic or fused ring radical derived from an aromatic hydrocarbon having 6 to 30 ring backbone carbon atoms, in which the number of the ring backbone carbon atoms is preferably 6 to 20, more preferably 6 to 15, may be partially saturated, and may comprise a spiro structure. The above aryl may include phenyl, biphenyl, terphenyl, naphthyl, binaphthyl, phenylnaphthyl, naphthylphenyl, fluorenyl, phenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, spirobifluorenyl, etc. The term "(3- to 30-membered)heteroaryl" is an aryl having 3 to 30 ring backbone atoms, including at least one, preferably 1 to 4 heteroatoms selected from the group consisting of B, N, O, S, Si, and P. The above heteroaryl may be a monocyclic ring, or a fused ring condensed with at least one benzene ring; may be partially saturated; may be one formed by linking at least one heteroaryl or aryl group to a heteroaryl group via a single bond(s); and may comprise a spiro structure. The above heteroaryl may include a monocyclic ring-type heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, and pyridazinyl, and a fused ring-type heteroaryl such as benzofuranyl, benzothiophenyl, isobenzofuranyl, dibenzofuranyl, dibenzothiophenyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, benzocarbazolyl, phenoxazinyl, phenothiazinyl, phenanthridinyl, benzodioxolyl, and dihydroacridinyl. The term "condensed ring" is a ring in which two or more rings are bonded by sharing two or more atoms, wherein a carbon atom(s) of the condensed ring may be replaced with at least one heteroatom selected from B, N, O, S, Si, and P. The above condensed ring may include a naphthalene, an anthracene, a triphenylene, a benzofluorene, phenanthrene, etc., in which the naphthalene may be 1,2-naphthalene, 1,3-naphthalene, 1,4-naphthalene, 1,5-naphthalene, 1,6-naphthalene, 1,7-naphthalene, 1,8-naphthalene, 2,3-naphthalene, 2,6-naphthalene, or 2,7-naphthalene. Furthermore, "halogen" includes F, Cl, Br, and I.

Herein, "substituted" in the expression "substituted or unsubstituted" means that a hydrogen atom in a certain functional group is replaced with another atom or another functional group, i.e. a substituent. The substituents of the substituted (C1-C30)alkyl, the substituted (C6-C30)aryl, the substituted (3- to 30-membered)heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di-(C6-C30)arylamino, the substituted (C1-C30)alkyl(C6-C30)arylamino, the substituted divalent (C10-C30) aromatic ring, the substituted benzene ring, the substituted naphthalene ring, and the substituted mono- or polycyclic, (C3-C30) alicyclic or aromatic ring, or the combination thereof, in La, Ar₁, Ar₂, Ar₃, A ring, B ring, C ring, R ₁ and R₂, each independently, are at least one selected from the group consisting of deuterium; a halogen; a cyano; a carboxyl; a nitro; a hydroxyl; a (C1-C30)alkyl; a halo(C1-C30)alkyl; a (C2-C30)alkenyl; a (C2-C30)alkynyl; a (C1-C30)alkoxy; a (C1-C30)alkylthio; a (C3-C30)cycloalkyl; a (C3-C30)cycloalkenyl; a (3- to 7-membered)heterocycloalkyl; a (C6-C30)aryloxy; a (C6-C30)arylthio; a (5- to 30-membered)heteroaryl unsubstituted or substituted with a (C6-C30)aryl; a (C6-C30)aryl unsubstituted or substituted with a (5- to 30-membered)heteroaryl; a tri(C1-C30)alkylsilyl; a tri(C6-C30)arylsilyl; a di(C1-C30)alkyl(C6-C30)arylsilyl; a (C1-C30)alkyldi(C6-C30)arylsilyl; an amino; a mono- or di- (C1-C30)alkylamino; a mono- or di- (C6-C30)arylamino unsubstituted or substituted with a (C1-C30)alkyl; a (C1-C30)alkyl(C6-C30)arylamino; a (C1-C30)alkylcarbonyl; a (C1-C30)alkoxycarbonyl; a (C6-C30)arylcarbonyl; a di(C6-C30)arylboronyl; a di(C1-C30)alkylboronyl; a (C1-C30)alkyl(C6-C30)arylboronyl; a (C6-C30)aryl(C1-C30)alkyl; and a (C1-C30)alkyl(C6-C30)aryl; preferably, are at least one selected from the group consisting of a (C1-C20)alkyl and a (C6-C25)aryl; more preferably, are at least one selected from the group consisting of a (C1-C10)alkyl and a (C6-C18)aryl; and for example, may be at least one selected from the group consisting of a methyl and a phenyl.

The organic electroluminescent compound represented by formula 1 includes the following compounds, but is not limited thereto:

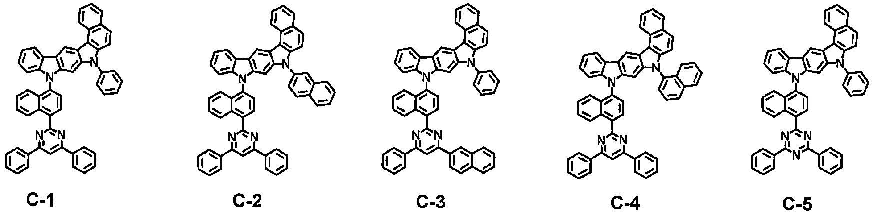

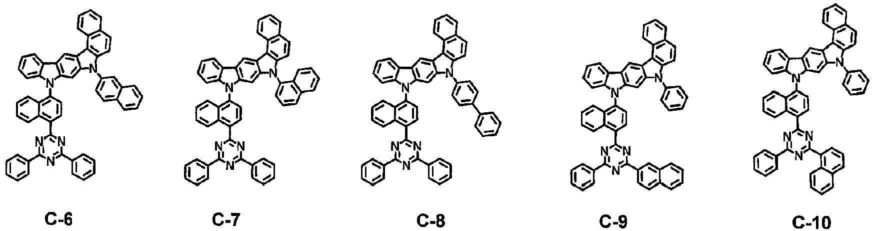

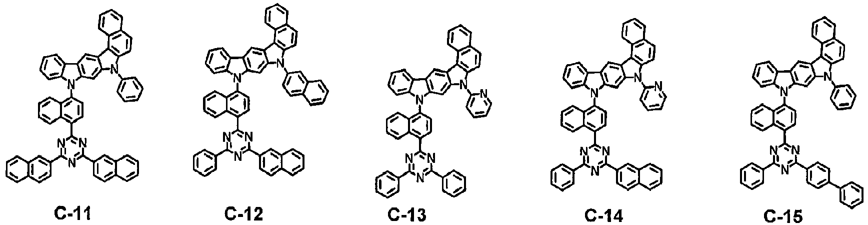

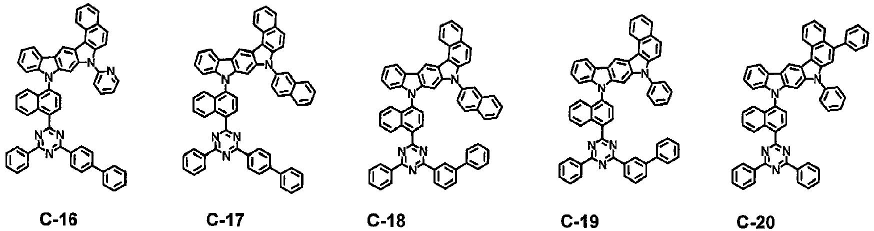

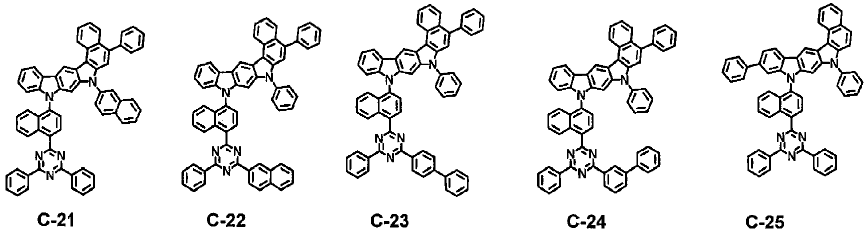

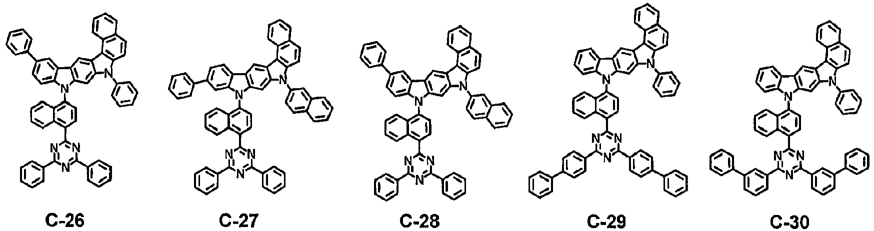

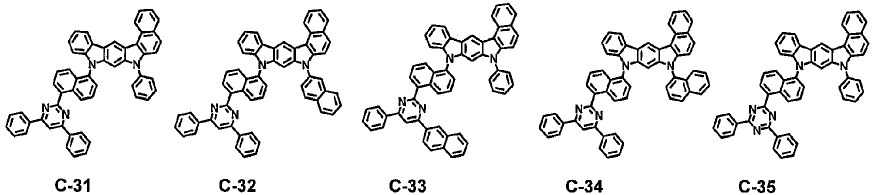

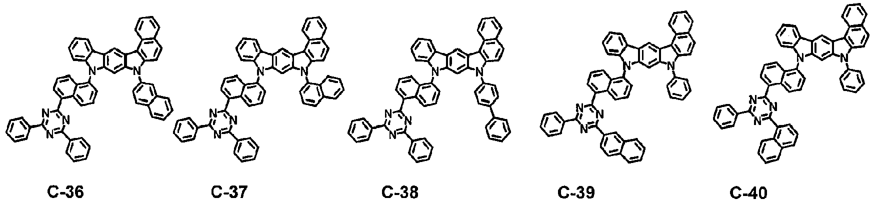

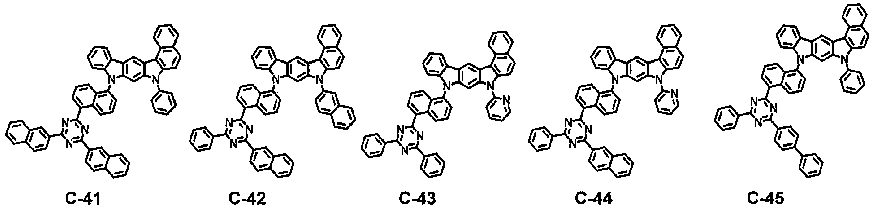

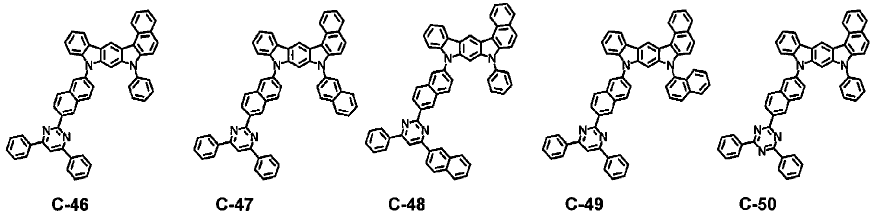

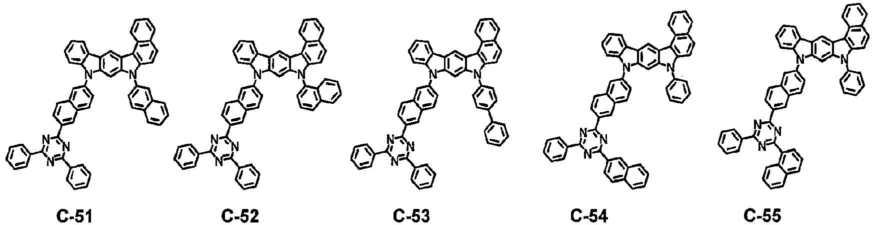

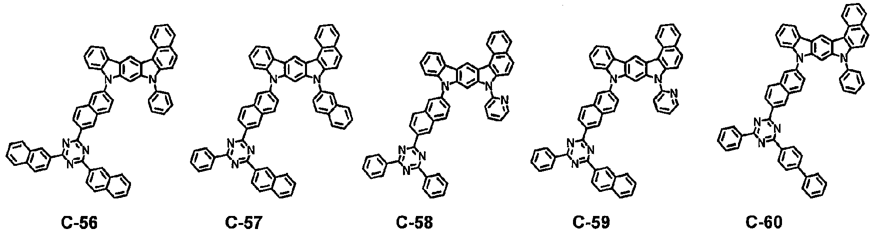

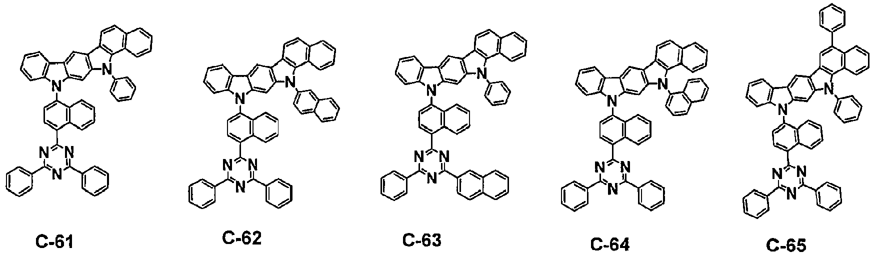

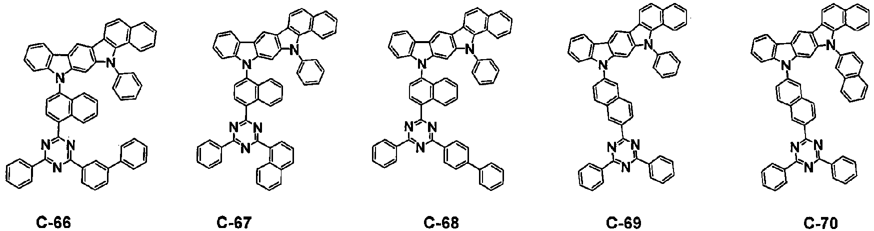

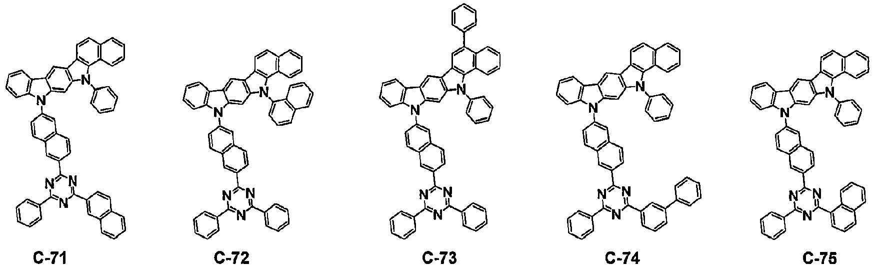

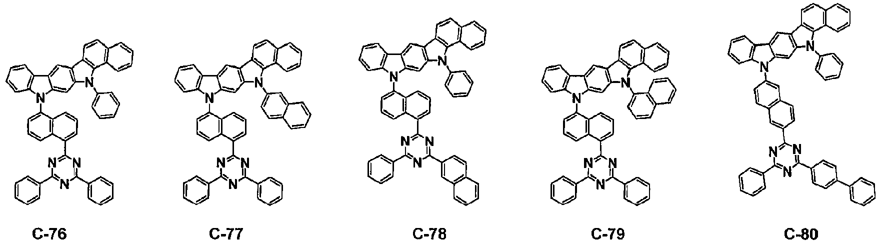

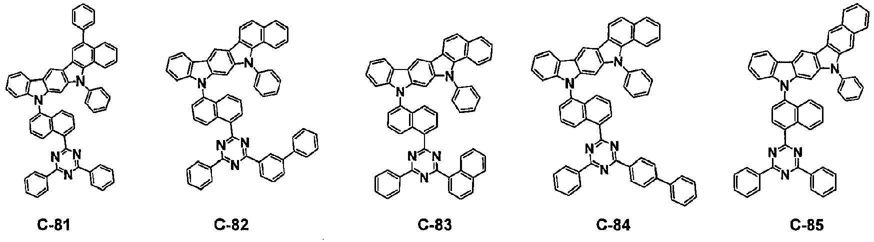

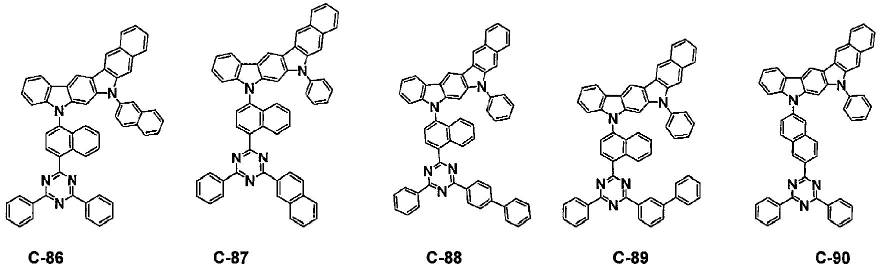

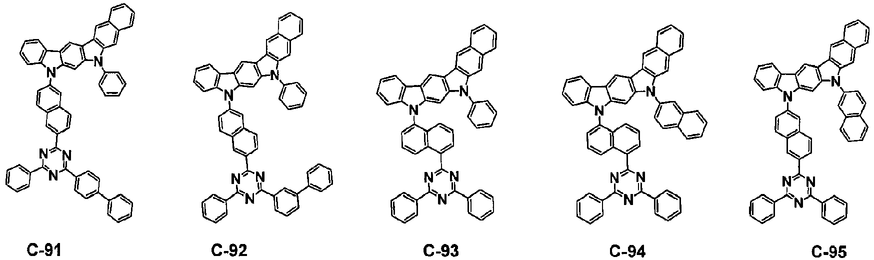

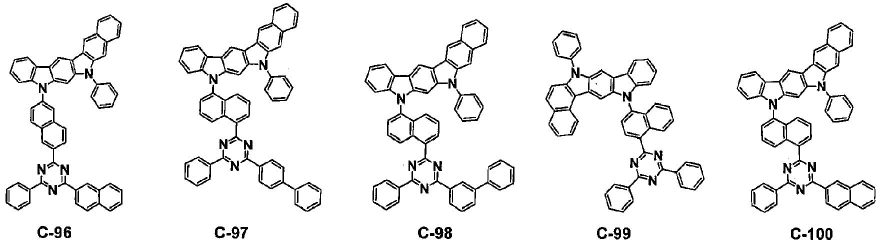

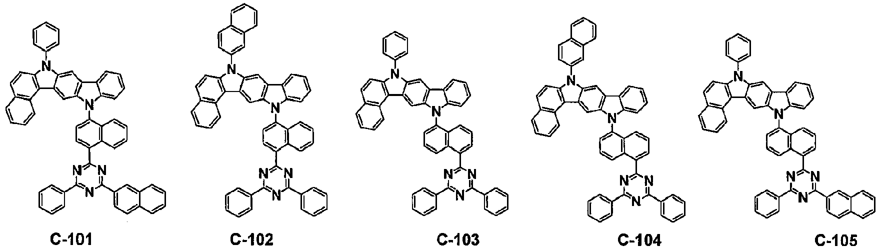

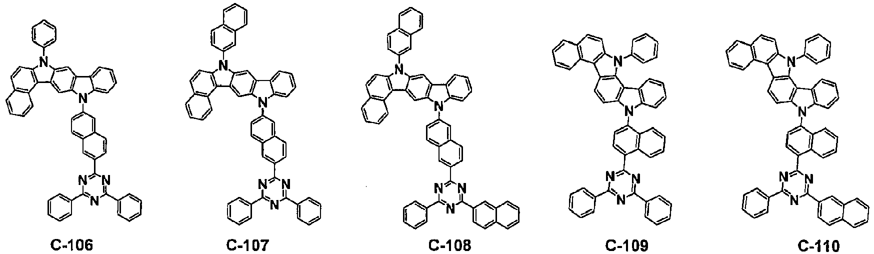

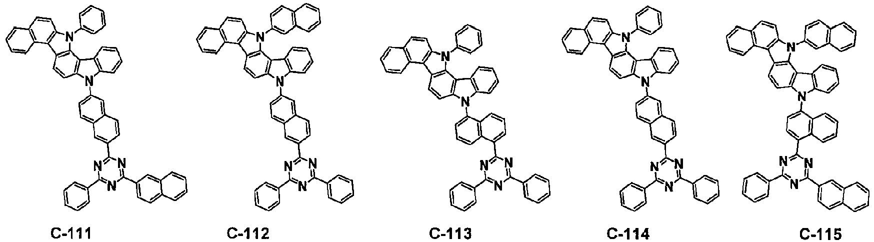

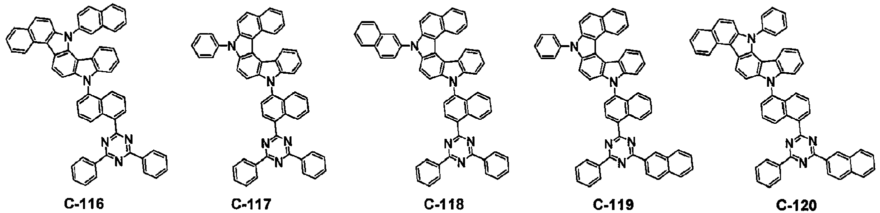

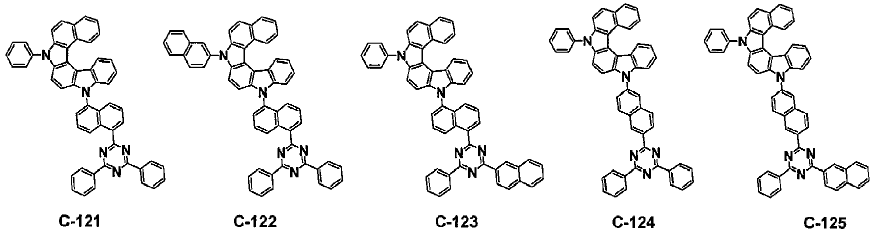

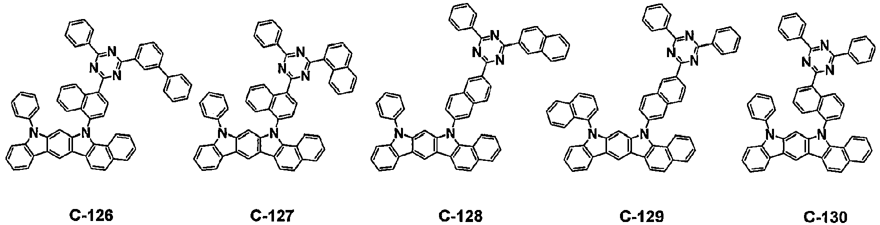

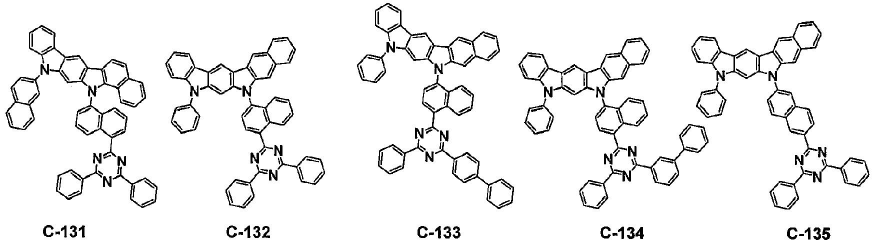

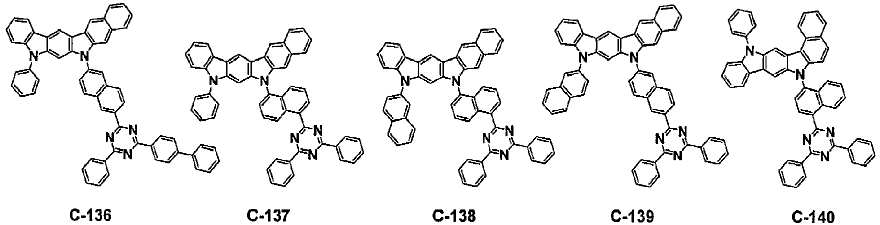

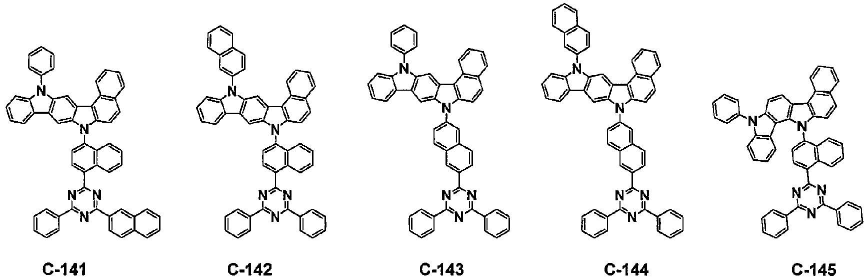

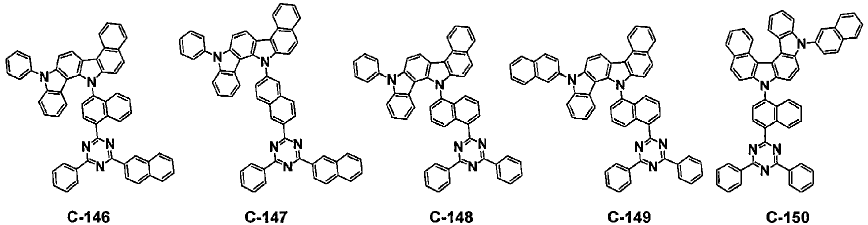

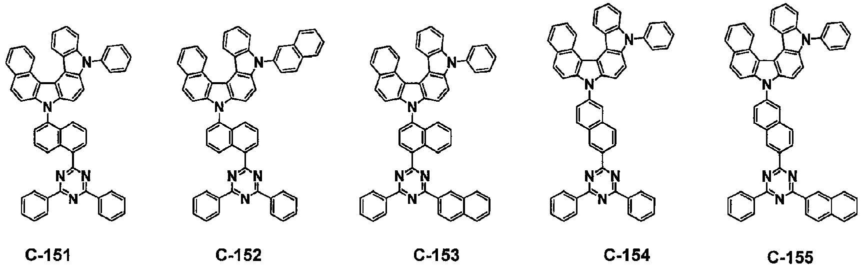

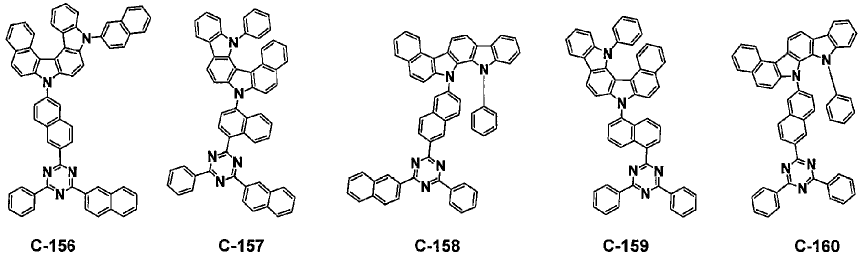

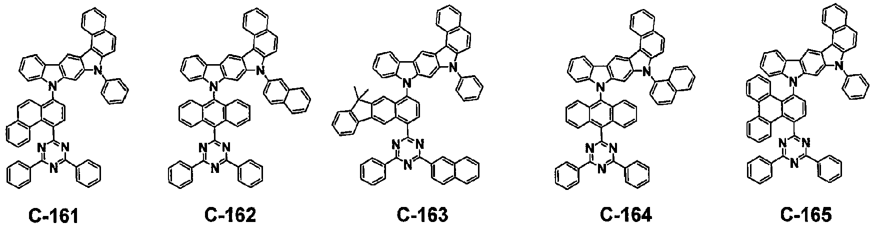

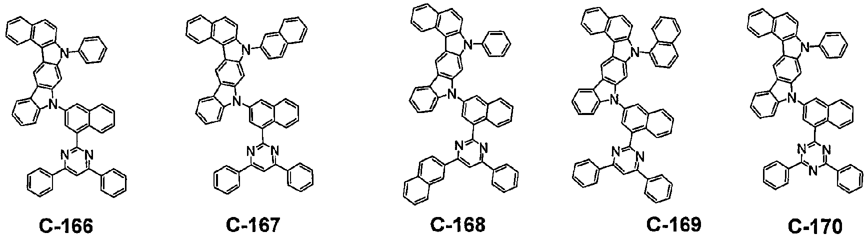

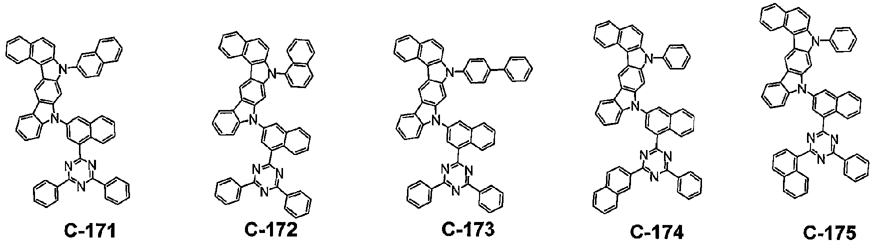

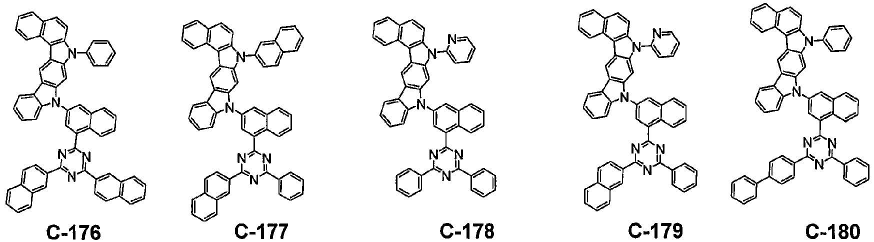

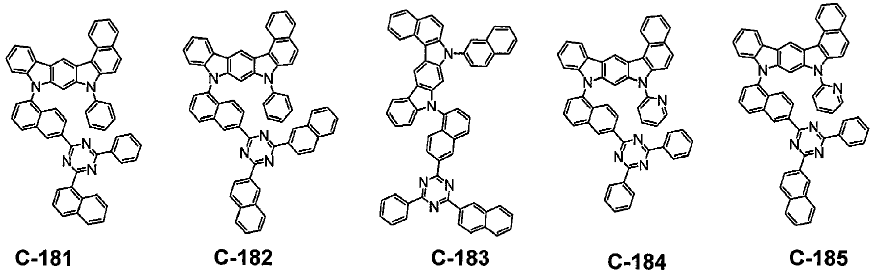

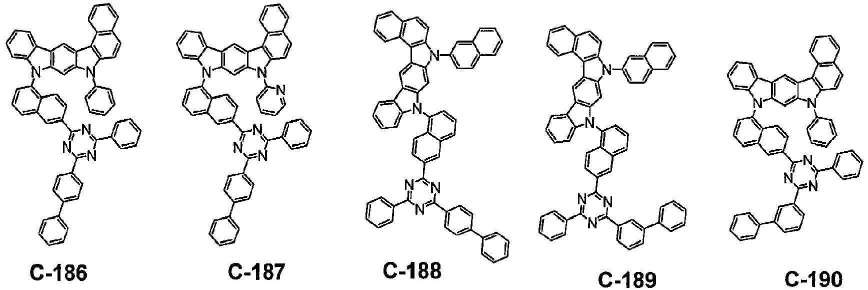

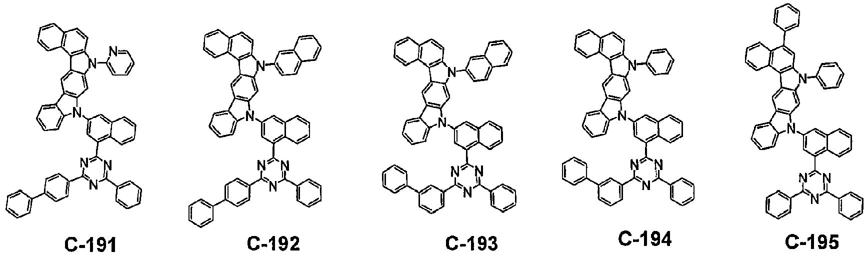

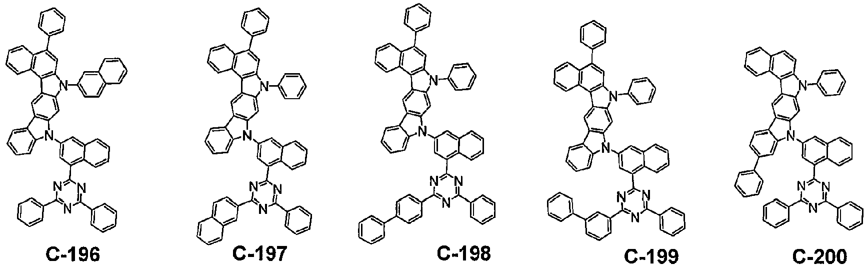

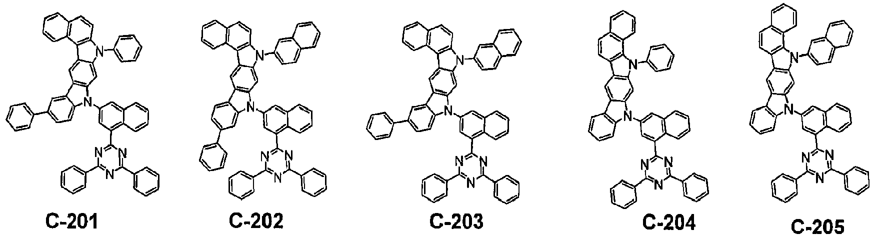

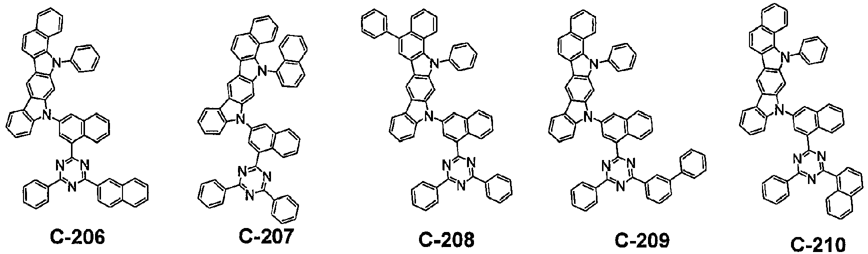

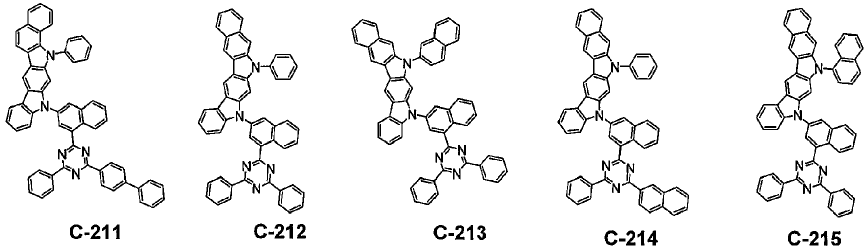

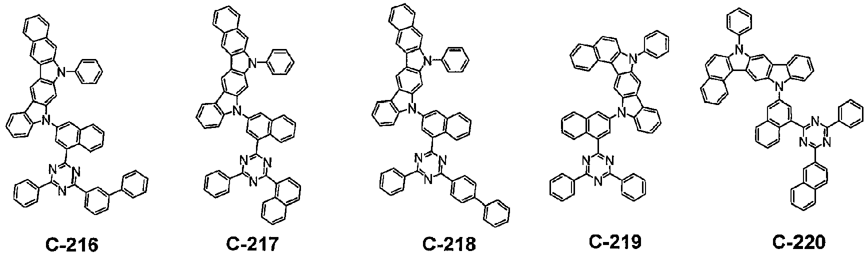

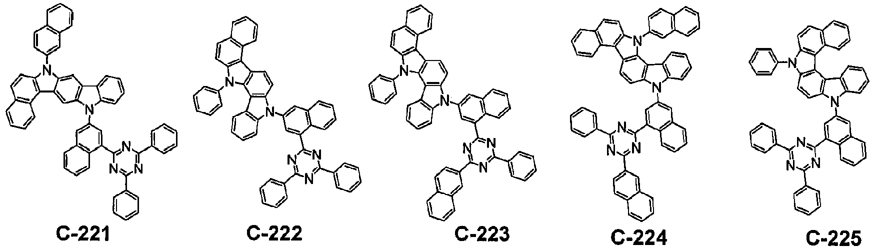

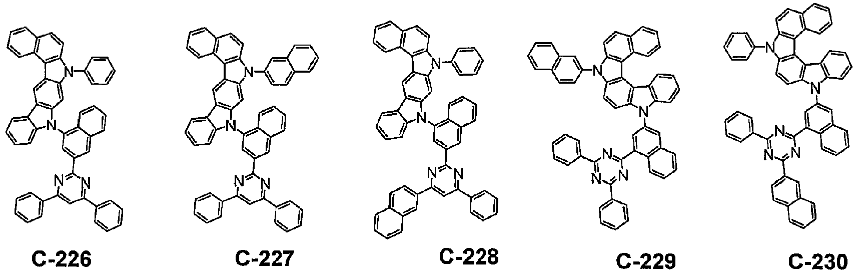

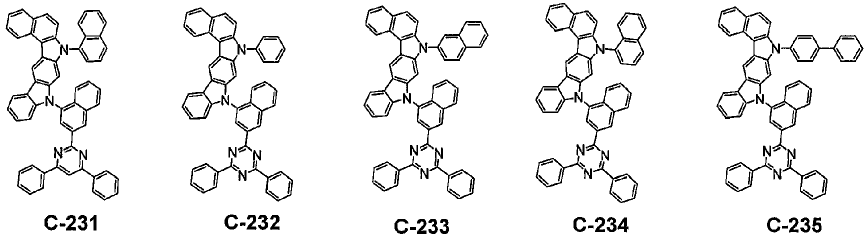

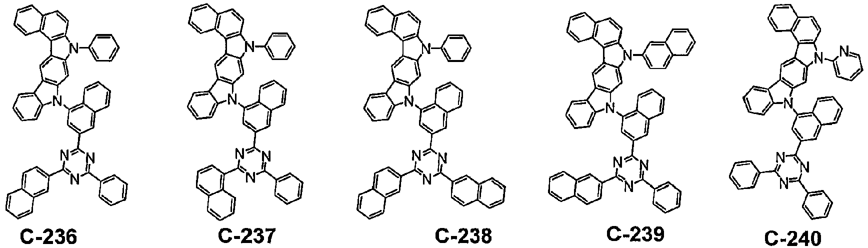

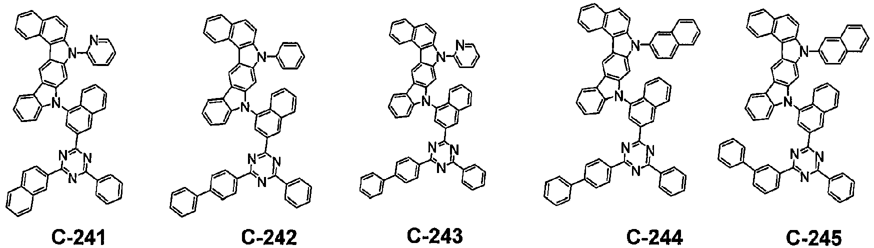

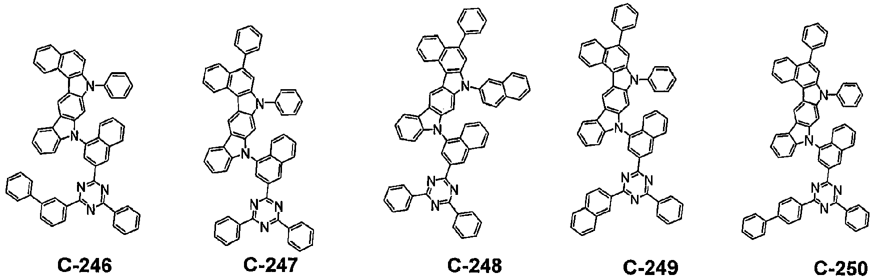

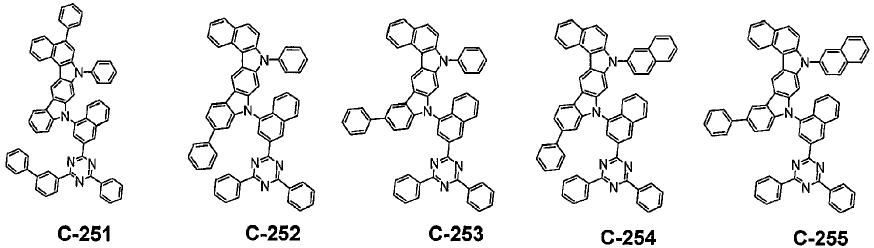

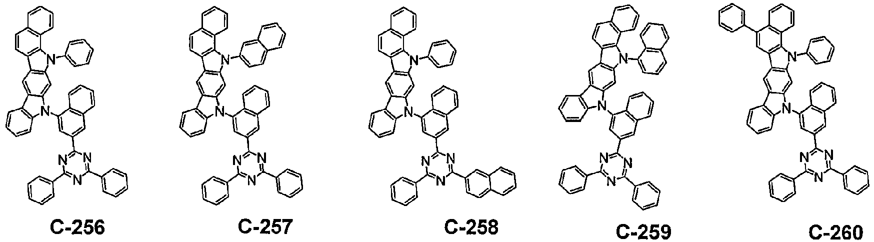

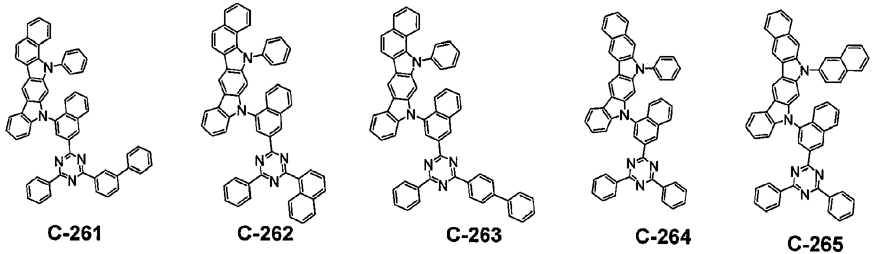

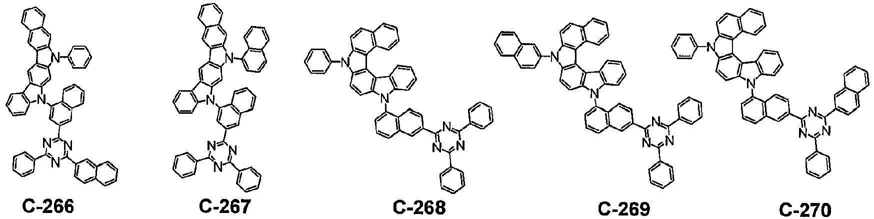

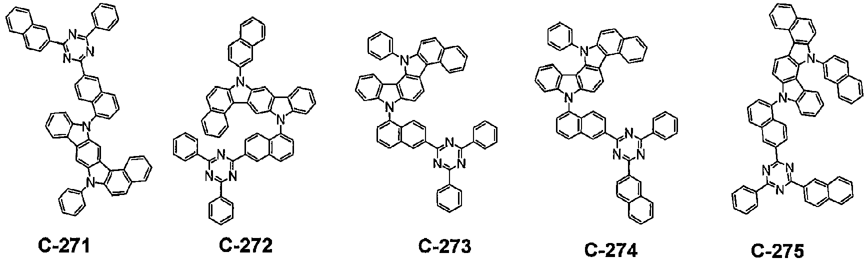

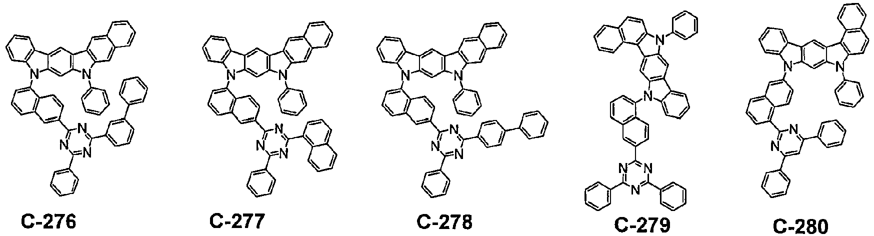

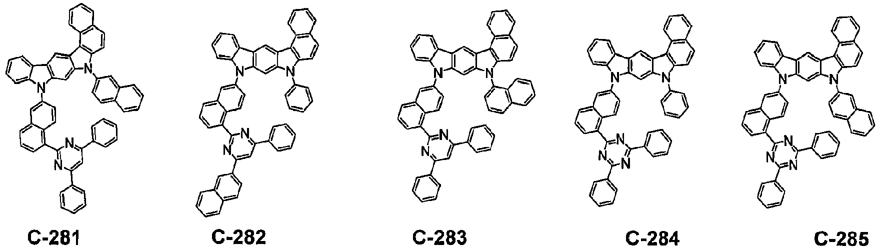

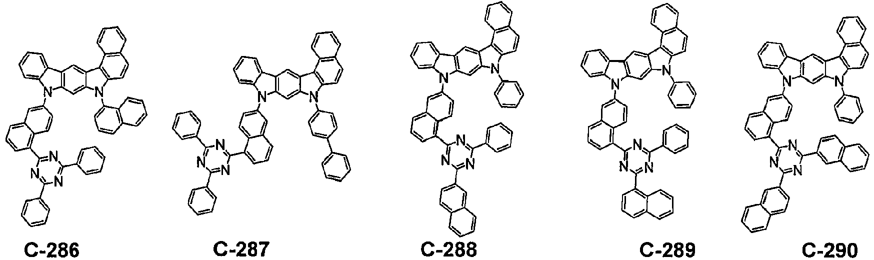

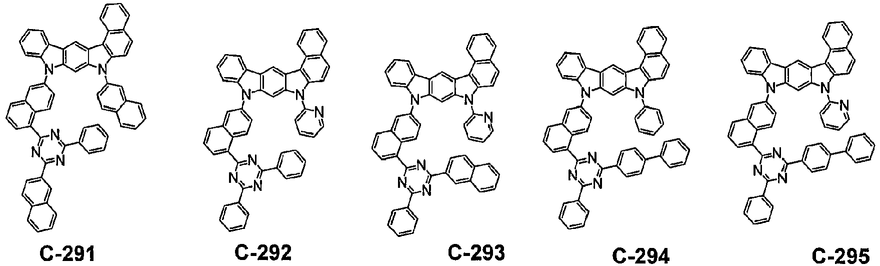

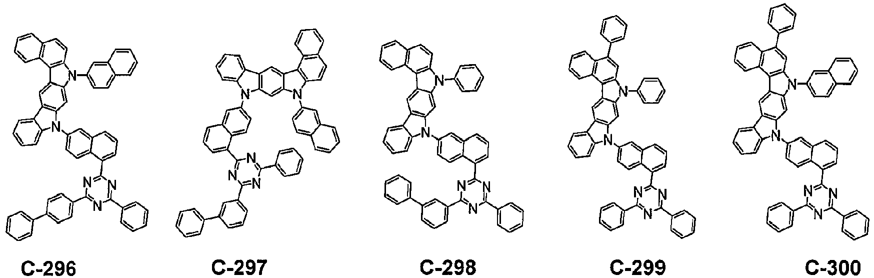

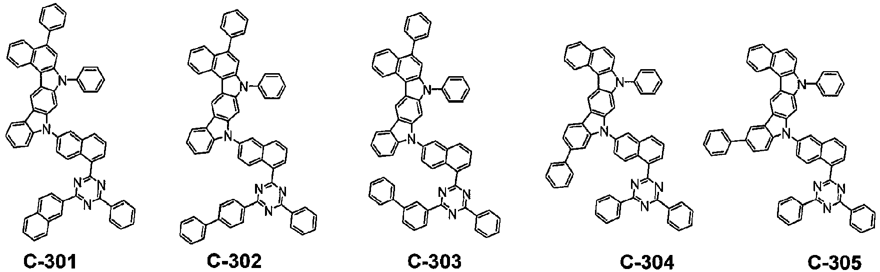

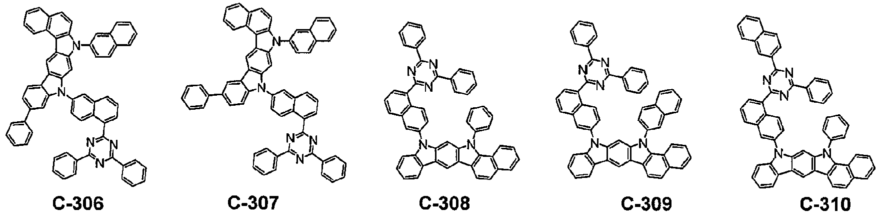

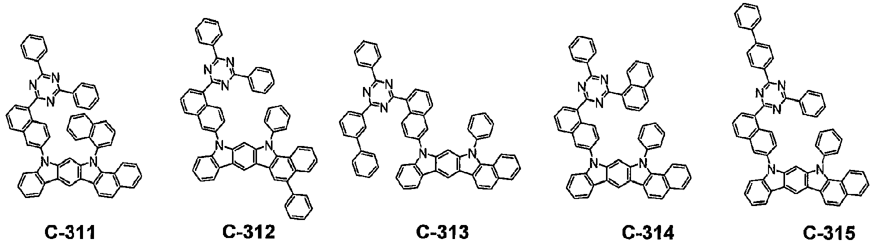

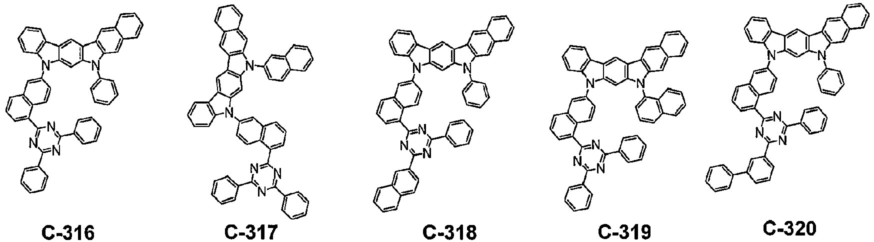

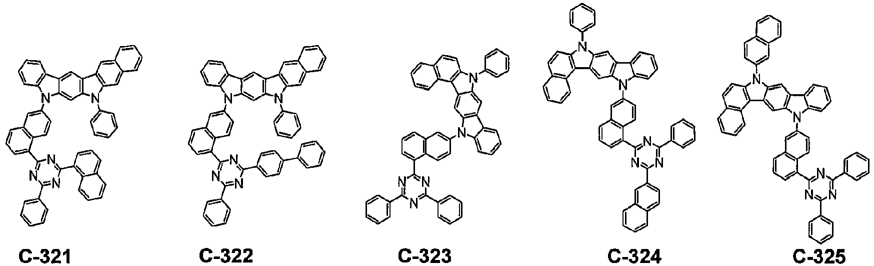

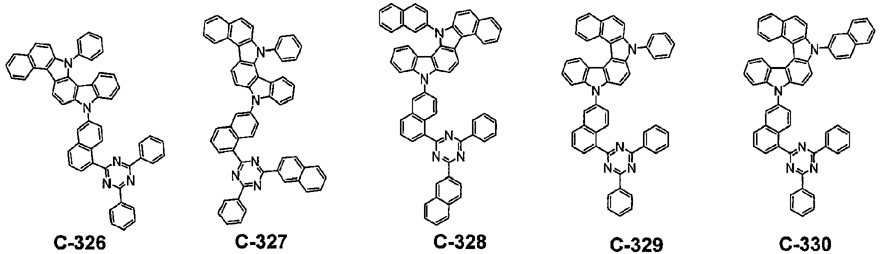

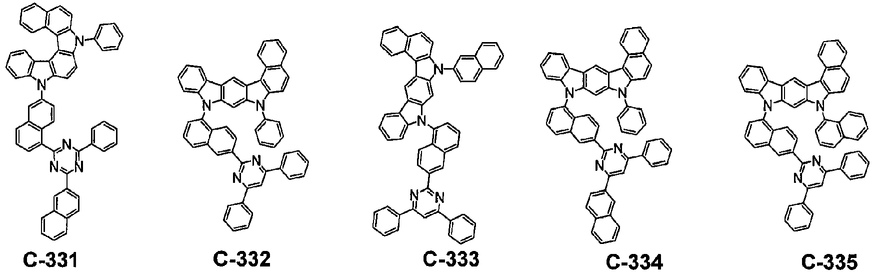

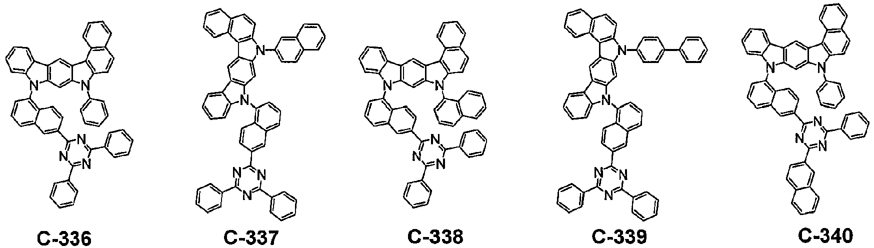

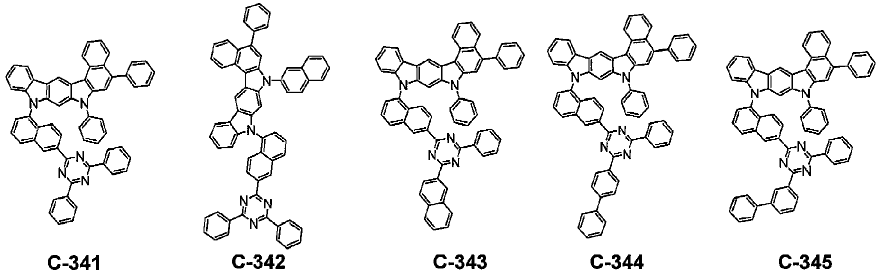

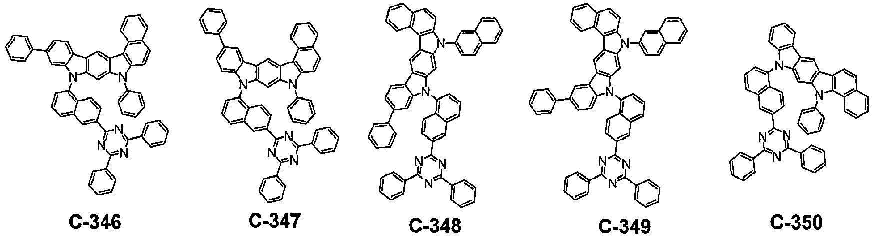

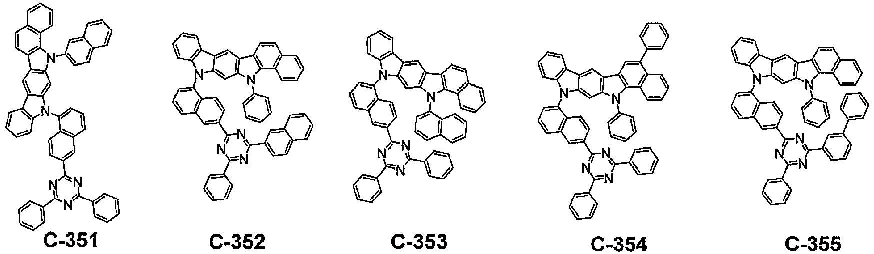

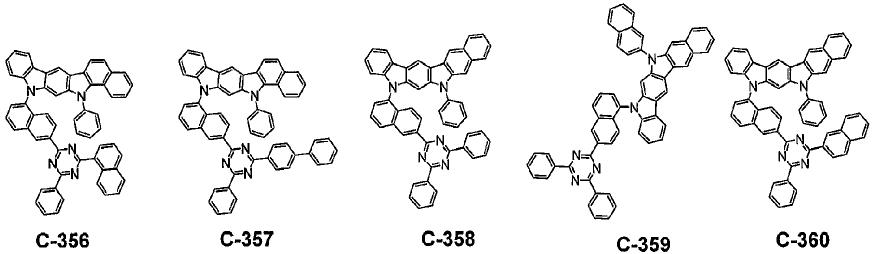

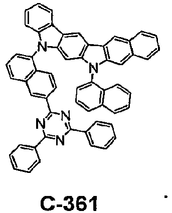

The organic electroluminescent compound of formula 1 according to the present disclosure may be produced by a synthetic method known to a person skilled in the art referring to the following reaction scheme 1, but is not limited thereto: 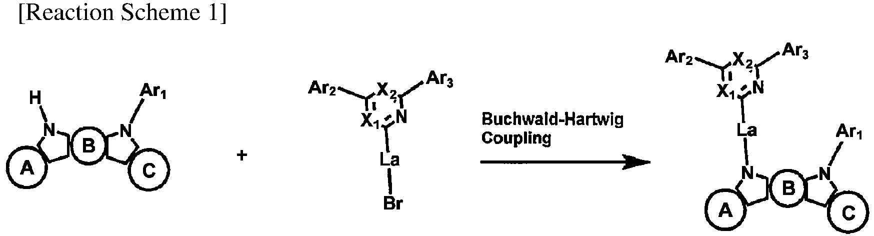 wherein, La, X₁, X₂, Ar₁, Ar₂, Ar₃, A ring, B ring, and C ring are as defined in formula 1.

The present disclosure may provide an organic electroluminescent material comprising the organic electroluminescent compound of formula 1, and an organic electroluminescent device comprising the material.

The organic electroluminescent material may consist of the organic electroluminescent compound of the present disclosure as a sole compound, or may further comprise conventional materials generally used in organic electroluminescent materials.

Meanwhile, the organic electroluminescent device of the present disclosure may comprise a first electrode, a second electrode, and at least one organic layer between the first and second electrodes. The organic layer may comprise at least one organic electroluminescent compound of formula 1. The organic layer may further comprise at least one compound selected from the group consisting of arylamine-based compounds and styrylarylamine-based compounds. Also, the organic layer may further comprise at least one metal selected from the group consisting of metals of Group 1, metals of Group 2, transition metals of the 4^{th} period, transition metals of the 5^{th} period, lanthanides, and organic metals of the d-transition elements of the Periodic Table, or at least one complex compound comprising the metal.

One of the first and second electrodes may be an anode, and the other may be a cathode. The organic layer may comprise a light-emitting layer, and may further comprise at least one layer selected from a hole injection layer, a hole transport layer, a hole auxiliary layer, a light-emitting auxiliary layer, an electron transport layer, an electron injection layer, an interlayer, a hole blocking layer, an electron blocking layer, and an electron buffer layer.

A hole injection layer, a hole transport layer, an electron blocking layer, or a combination thereof can be used between the anode and the light-emitting layer. The hole injection layer may be multi-layers in order to lower the hole injection barrier (or hole injection voltage) from the anode to the hole transport layer or the electron blocking layer, wherein each of the multi-layers may use two compounds simultaneously. The electron blocking layer may be placed between the hole transport layer (or the hole injection layer) and the light-emitting layer, and can confine the excitons within the light-emitting layer by blocking the overflow of electrons from the light-emitting layer to prevent a light-emitting leakage. The hole transport layer or the electron blocking layer may also be multi-layers, wherein each layer may use a plurality of compounds.

An electron buffer layer, a hole blocking layer, an electron transport layer, an electron injection layer, or a combination thereof can be used between the light-emitting layer and the cathode. The electron buffer layer may be multi-layers in order to control the injection of the electron and improve the interfacial properties between the light-emitting layer and the electron injection layer, wherein each of the multi-layers may use two compounds simultaneously. The hole blocking layer or the electron transport layer may also be multi-layers, wherein each layer may use a plurality of compounds.

The light-emitting auxiliary layer may be placed between the anode and the light-emitting layer, or between the cathode and the light-emitting layer. When the light-emitting auxiliary layer is placed between the anode and the light-emitting layer, it can be used for promoting the hole injection and/or the hole transport, or for preventing the overflow of electrons. When the light-emitting auxiliary layer is placed between the cathode and the light-emitting layer, it can be used for promoting the electron injection and/or the electron transport, or for preventing the overflow of holes. Also, the hole auxiliary layer may be placed between the hole transport layer (or hole injection layer) and the light-emitting layer, and may be effective to promote or block the hole transport rate (or the hole injection rate), thereby enabling the charge balance to be controlled. When an organic electroluminescent device includes two or more hole transport layers, the hole transport layer, which is further included, may be used as a hole auxiliary layer or an electron blocking layer. The light-emitting auxiliary layer, the hole auxiliary layer or the electron blocking layer may have an effect of improving the efficiency and/or the lifespan of the organic electroluminescent device.

In the organic electroluminescent device of the present disclosure, preferably, at least one layer selected from a chalcogenide layer, a metal halide layer, and a metal oxide layer (hereinafter, "a surface layer") may be placed on an inner surface(s) of one or both electrode(s). Specifically, a chalcogenide (including oxides) layer of silicon or aluminum is preferably placed on an anode surface of an electroluminescent medium layer, and a metal halide layer or a metal oxide layer is preferably placed on a cathode surface of an electroluminescent medium layer. The operation stability for the organic electroluminescent device may be obtained by the surface layer. Preferably, the chalcogenide includes SiOₓ(1≤X≤2), AlOₓ(1≤X≤1.5), SiON, SiAlON, etc.; the metal halide includes LiF, MgF₂, CaF₂, a rare earth metal fluoride, etc.; and the metal oxide includes Cs₂O, Li₂O, MgO, SrO, BaO, CaO, etc.

In the organic electroluminescent device of the present disclosure, a mixed region of an electron transport compound and a reductive dopant, or a mixed region of a hole transport compound and an oxidative dopant may be placed on at least one surface of a pair of electrodes. In this case, the electron transport compound is reduced to an anion, and thus it becomes easier to inject and transport electrons from the mixed region to an electroluminescent medium. Furthermore, the hole transport compound is oxidized to a cation, and thus it becomes easier to inject and transport holes from the mixed region to the electroluminescent medium. Preferably, the oxidative dopant includes various Lewis acids and acceptor compounds, and the reductive dopant includes alkali metals, alkali metal compounds, alkaline earth metals, rare-earth metals, and mixtures thereof. A reductive dopant layer may be employed as a charge generating layer to prepare an organic electroluminescent device having two or more light-emitting layers and emitting white light.

The organic electroluminescent compound represented by formula 1 may be comprised in the light-emitting layer. When used in the light-emitting layer, the organic electroluminescent compound of formula 1 may be comprised as a host material. Preferably, the light-emitting layer may further comprise at least one dopant. If necessary, another compound besides the organic electroluminescent compound of formula 1 may be further comprised as a second host material. Herein, the weight ratio of the first host material to the second host material is in the range of 1:99 to 99:1.

The second host material can use any of the known phosphorescent hosts. In terms of luminous efficiency, the second host material may be preferably selected from the group consisting of the compounds represented by the following formulas 18 to 23:

H-(Cz-L₄)ₕ-M --- (18)

H-(Cz)ᵢ-L₄-M --- (19)

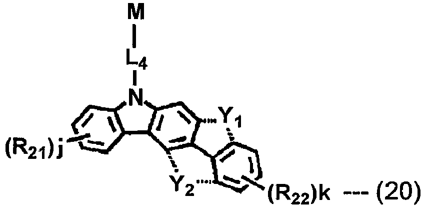

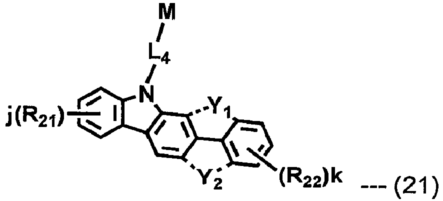

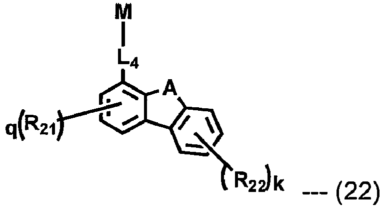

wherein
Cz represents the following structure: 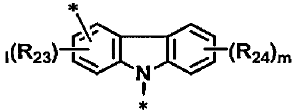
A represents -O- or -S-; and
R₂₁ to R₂₄, each independently, represent hydrogen, deuterium, a halogen, a substituted or unsubstituted (C1-C30)alkyl, a substituted or unsubstituted (C6-C30)aryl, a substituted or unsubstituted (5- to 30-membered)heteroaryl, or -SiR₂₅R₂₆R₂₁, in which R₂₅ to R₂₇, each independently, represent a substituted or unsubstituted (C1-C30)alkyl, or a substituted or unsubstituted (C6-C30)aryl; L₄ represents a single bond, a substituted or unsubstituted (C6-C30)arylene, or a substituted or unsubstituted (5- to 30-membered)heteroarylene; M represents a substituted or unsubstituted (C6-C30)aryl, or a substituted or unsubstituted (5- to 30-membered)heteroaryl; Y₁ and Y₂, each independently, represent -O-, -S-, -NR₃₁- or -CR₃₂R₃₃-, with the proviso that Y₁ and Y₂ are not present simultaneously; R₃₁ to R₃₃, each independently, represent a substituted or unsubstituted (C1-C30)alkyl, a substituted or unsubstituted (C6-C30)aryl, or a substituted or unsubstituted (5- to 30-membered)heteroaryl; R₃₂ and R₃₃ may be the same or different; h and i, each independently, represent an integer of 1 to 3; j, k, l and m, each independently, represent an integer of 0 to 4; q represents an integer of 0 to 3; where if h, i, j, k, l, m or q represents an integer of 2 or more, each (Cz-L₄), each (Cz), each R₂₁, each R₂₂, each R₂₃ or each R₂₄ may be the same or different;

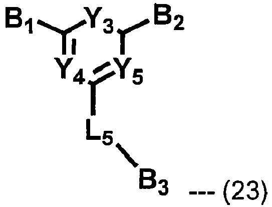

wherein
Y₃ to Y₅, each independently, represent CR₃₄ or N, in which R₃₄ represents hydrogen, a substituted or unsubstituted (C1-C30)alkyl, a substituted or unsubstituted (C6-C30)aryl, or a substituted or unsubstituted (5- to 30-membered)heteroaryl;
B₁ and B₂, each independently, represent hydrogen, a substituted or unsubstituted (C6-C30)aryl, or a substituted or unsubstituted (5- to 30-membered)heteroaryl;
B₃ represents a substituted or unsubstituted (C6-C30)aryl, or a substituted or unsubstituted (5- to 30-membered)heteroaryl; and
L₅ represents a single bond, a substituted or unsubstituted (C6-C30)arylene, or a substituted or unsubstituted (5- to 30-membered)heteroarylene.

Specifically, the preferred examples of the second host material are as follows. 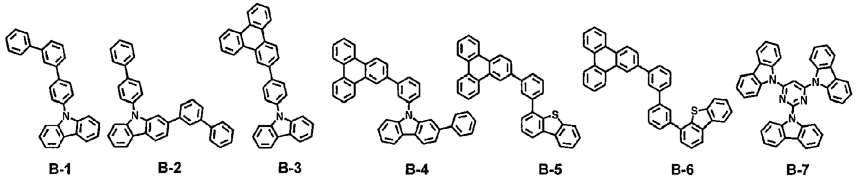 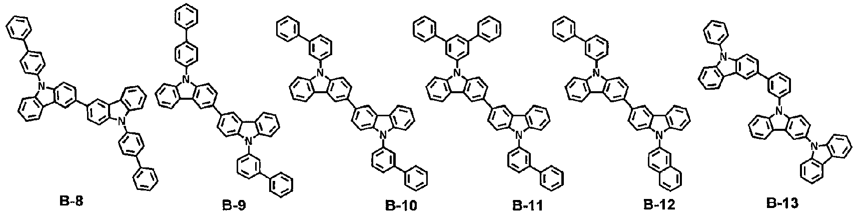 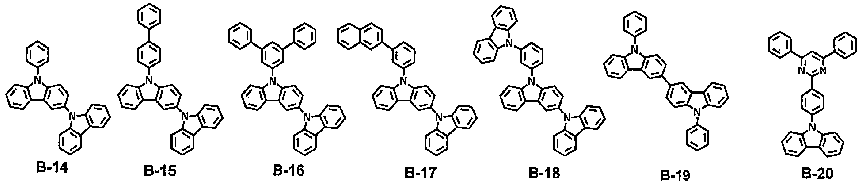 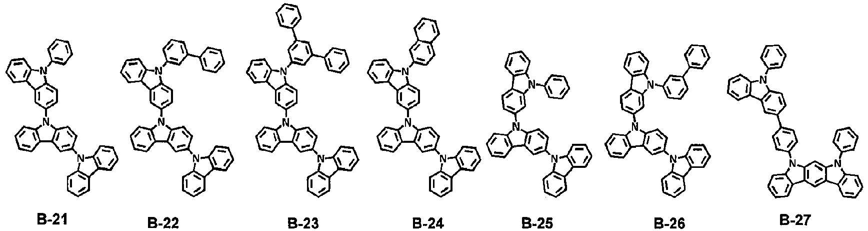 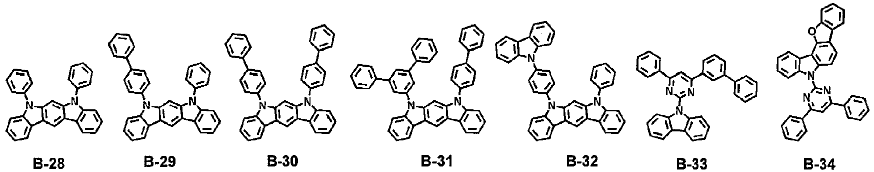 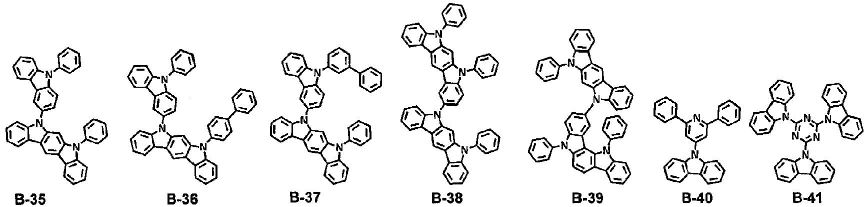 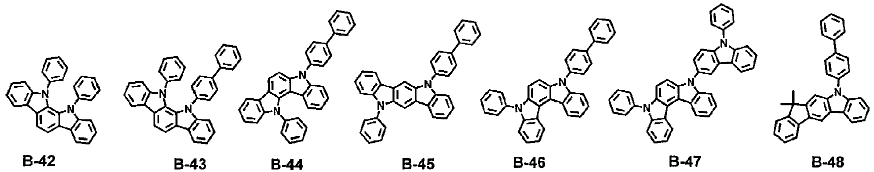 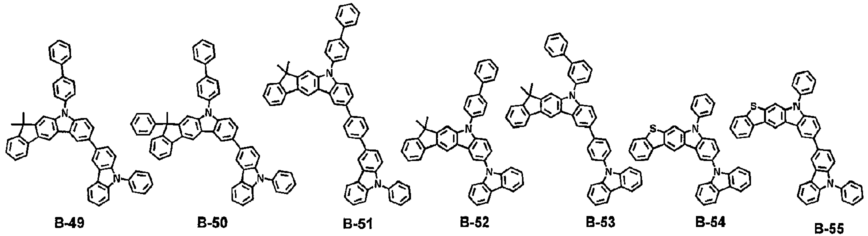 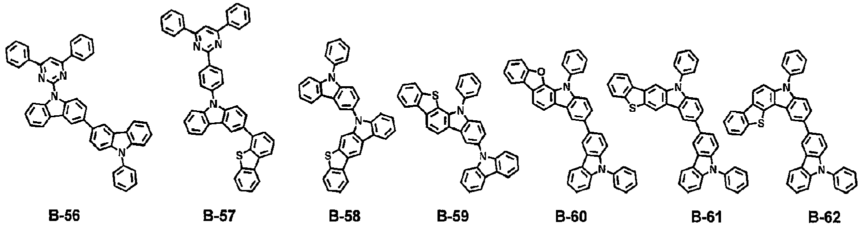 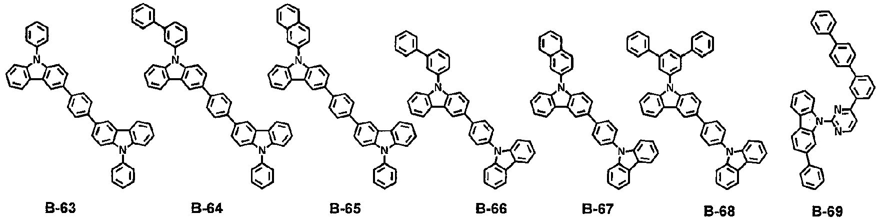 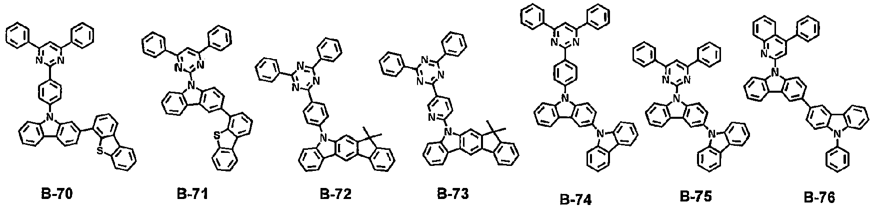 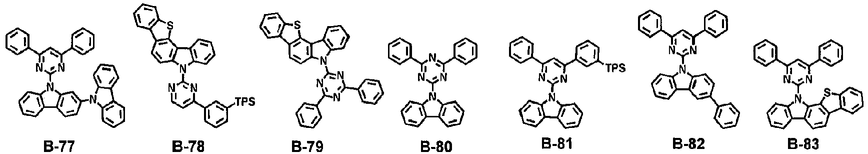 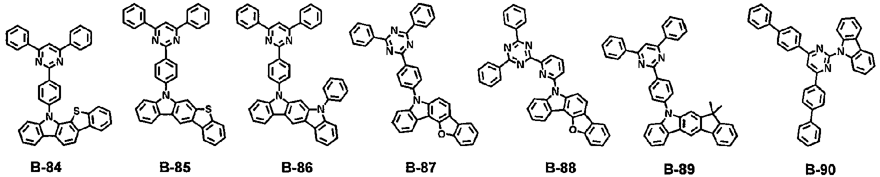 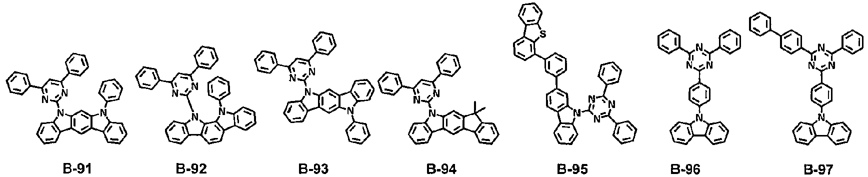 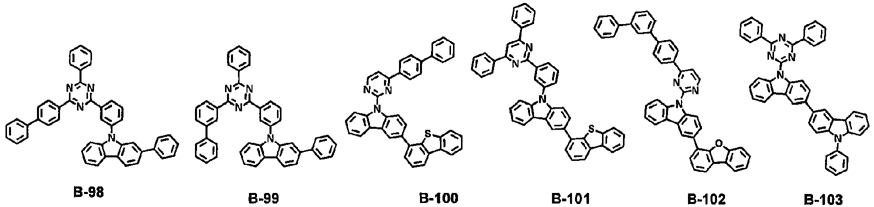 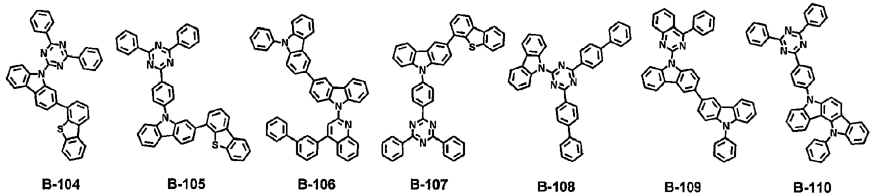 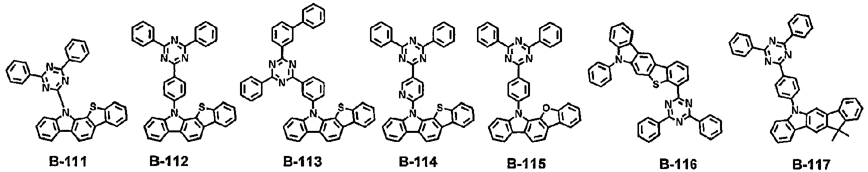 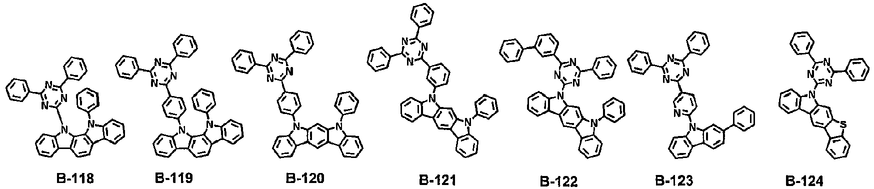 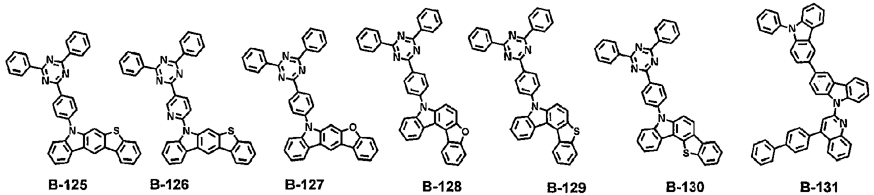 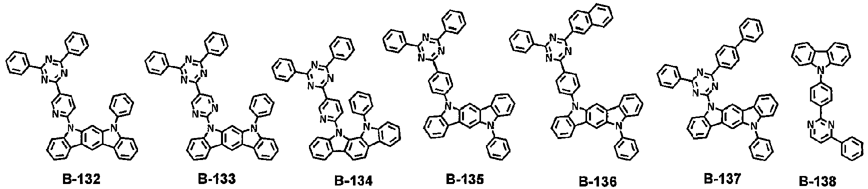 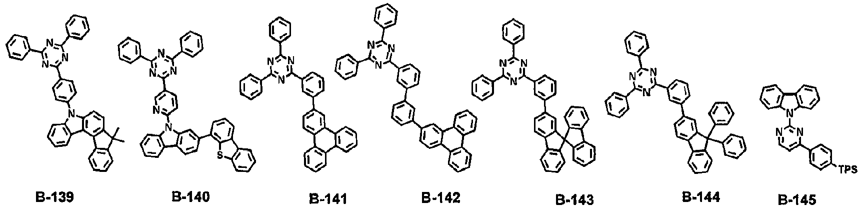 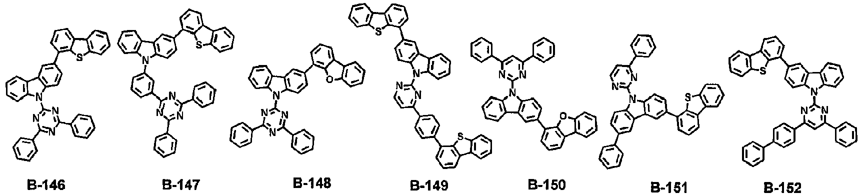 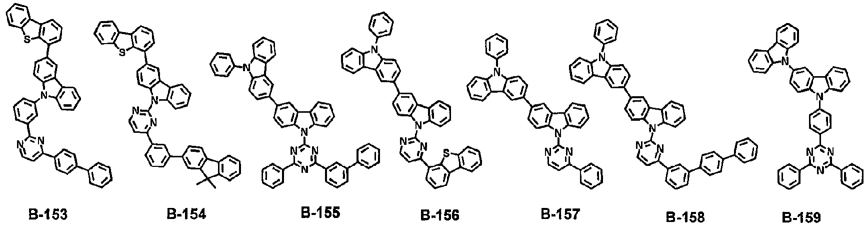 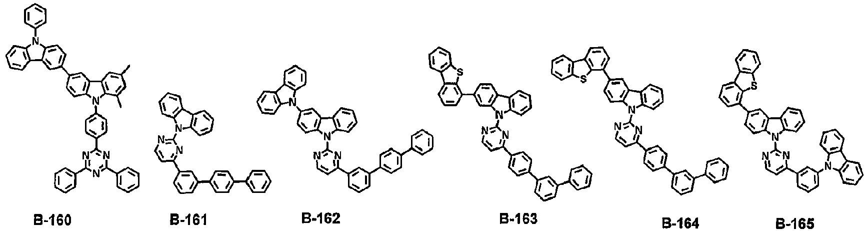 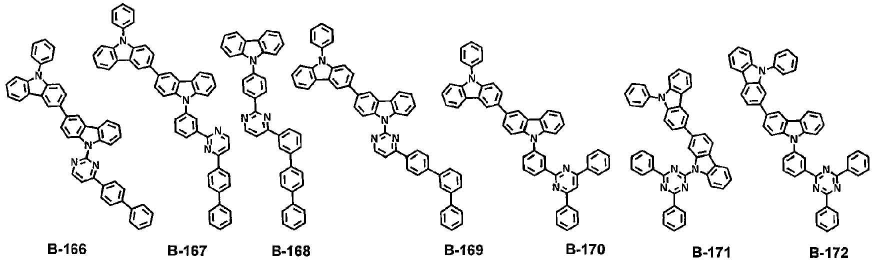 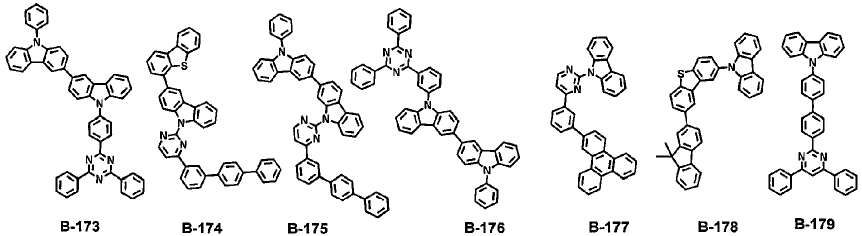 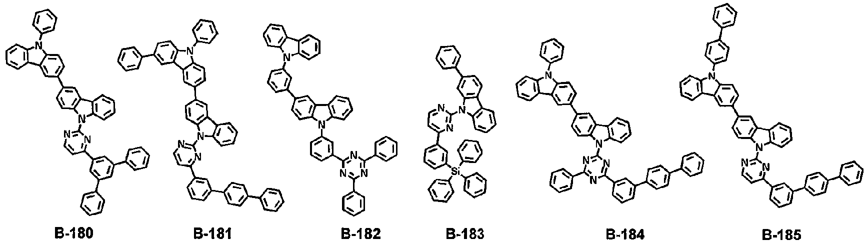 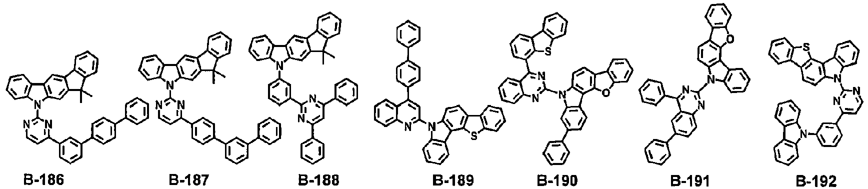 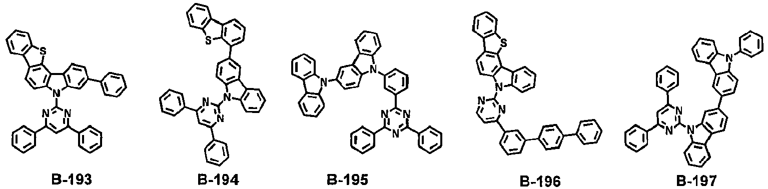 [Wherein, TPS represents a triphenylsilyl group.]

The dopant comprised in the organic electroluminescent device of the present disclosure is preferably at least one phosphorescent dopant. The phosphorescent dopant material applied to the organic electroluminescent device of the present disclosure is not particulary limited, but may be preferably selected from the metallated complex compounds of iridium (Ir), osmium (Os), copper (Cu), and platinum (Pt), more preferably selected from ortho-metallated complex compounds of iridium (Ir), osmium (Os), copper (Cu), and platinum (Pt), and even more preferably ortho-metallated iridium complex compounds.

The dopant comprised in the organic electroluminescent device of the present disclosure may comprise the compound selected from the group consisting of the compounds represented by the following formulas 101 to 104: 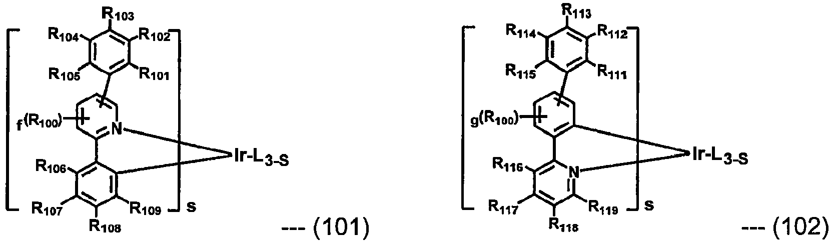 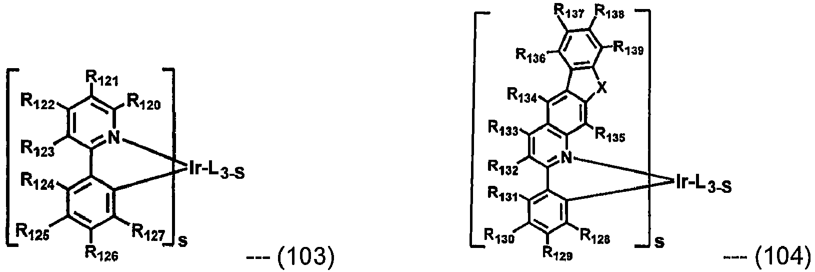 wherein, L is selected from the following structures: 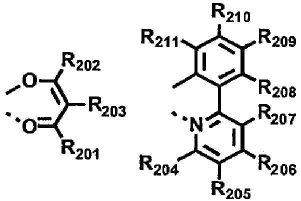
R₁₀₀, R₁₃₄ and R₁₃₅, each independently, represent hydrogen, deuterium, a substituted or unsubstituted (C1-C30)alkyl, or a substituted or unsubstituted (C3-C30)cycloalkyl;
R₁₀₁ to R₁₀₉ and R₁₁₁ to R₁₂₃, each independently, represent hydrogen, deuterium, a halogen, a (C1-C30)alkyl unsubstituted or substituted with a halogen, a substituted or unsubstituted (C3-C30)cycloalkyl, a substituted or unsubstituted (C6-C30)aryl, a cyano, or a substituted or unsubstituted (C1-C30)alkoxy; R₁₀₆ to R₁₀₉ may be linked to adjacent R₁₀₆ to R₁₀₉, respectively, to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl; and R₁₂₀ to R₁₂₃ may be linked to adjacent R₁₂₀ to R₁₂₃, respectively, to form a substituted or unsubstituted fused ring, e.g., a quinoline unsubstituted or substituted with at least one of an alkyl, an aryl, an arylalkyl and an alkylaryl;
R₁₂₄ to R₁₃₃ and R₁₃₆ to R₁₃₉, each independently, represent hydrogen, deuterium, a halogen, a substituted or unsubstituted (C1-C30)alkyl, or a substituted or unsubstituted (C6-C30)aryl; and R₁₂₄ to R₁₂₇ may be linked to adjacent R₁₂₄ to R₁₂₇, respectively, to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl;
X represents CR₁₁R₁₂, O, or S;
R₁₁ and R₁₂, each independently, represent a substituted or unsubstituted (Cl-ClO)alkyl, or a substituted or unsubstituted (C6-C30)aryl;
R₂₀₁ to R₂₁₁, each independently, represent hydrogen, deuterium, a halogen, a (C1-C30)alkyl unsubstituted or substituted with deuterium or a halogen, a substituted or unsubstituted (C3-C30)cycloalkyl, or a (C6-C30)aryl unsubstituted or substituted with an alkyl or deuterium; and R₂₀₈ to R₂₁₁ may be linked to adjacent R₂₀₈ to R₂₁₁, respectively, to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl;
f and g, each independently, represent an integer of 1 to 3; where if f or g is an integer of 2 or more, each R₁₀₀ may be the same or different; and
s represents an integer of 1 to 3.

The specific examples of the compound used as a dopant are as follows.

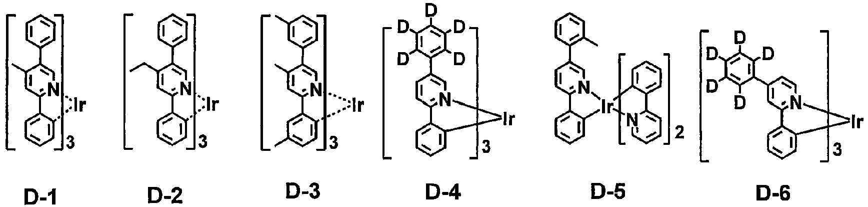

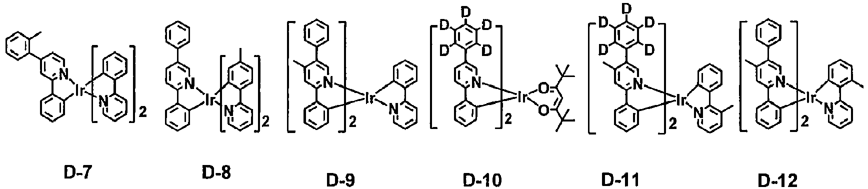

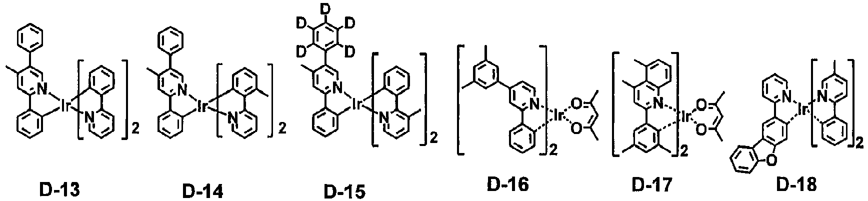

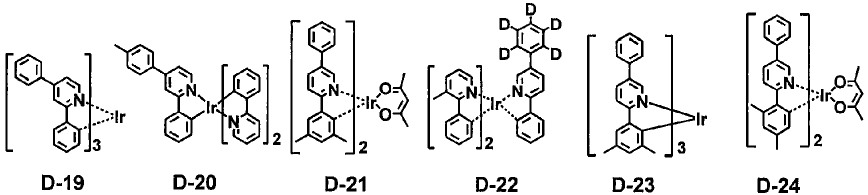

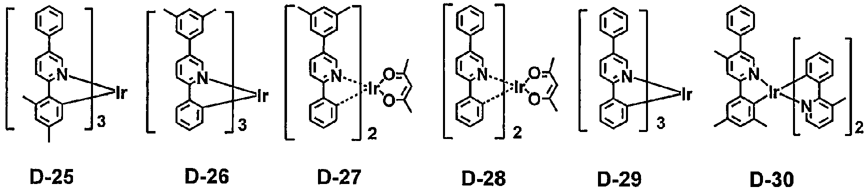

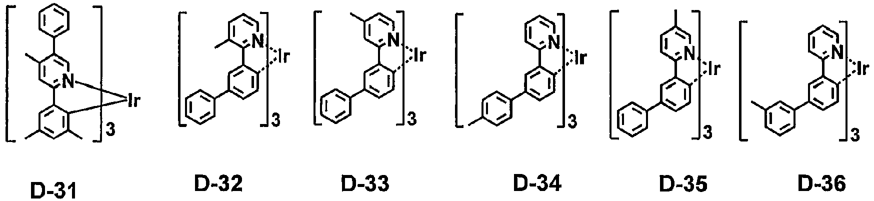

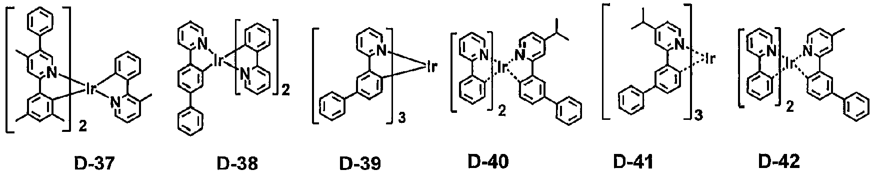

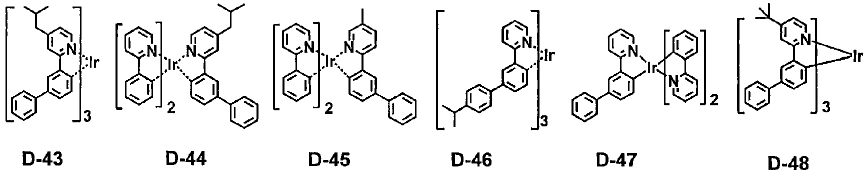

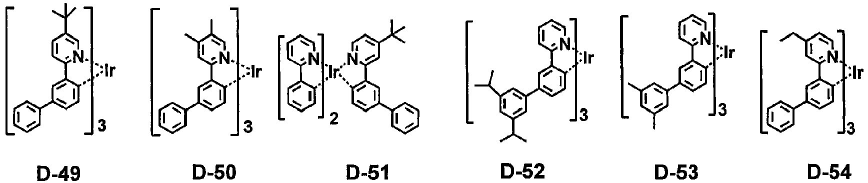

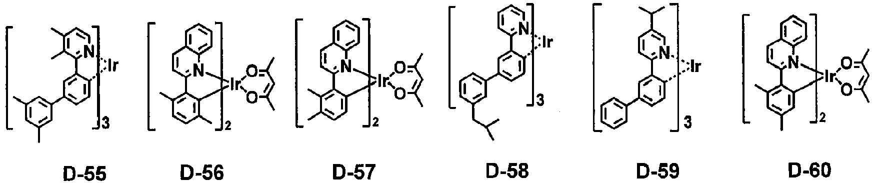

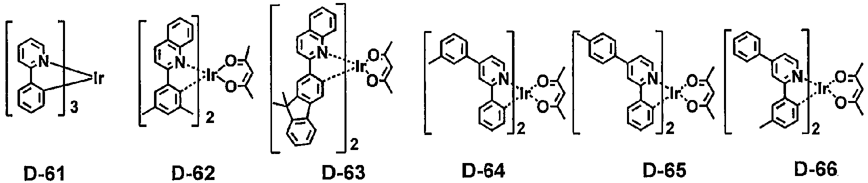

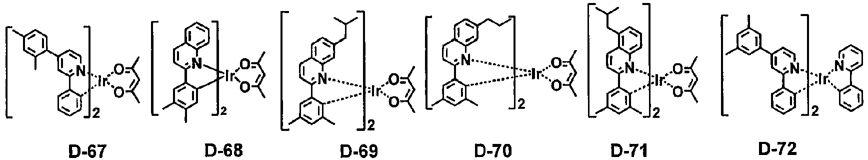

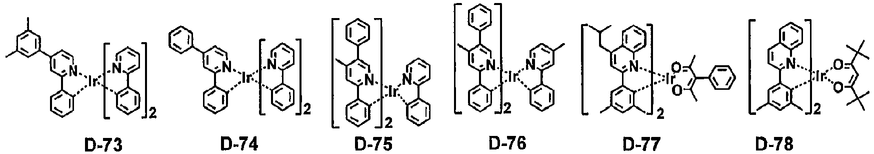

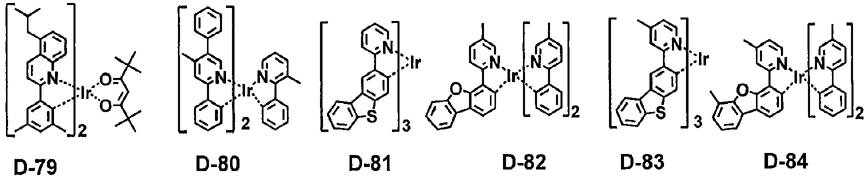

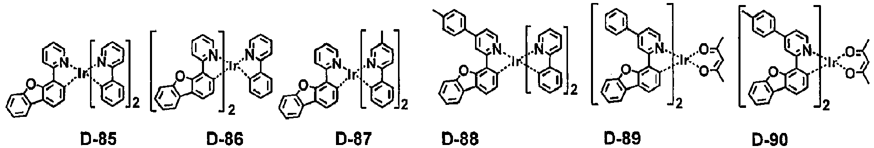

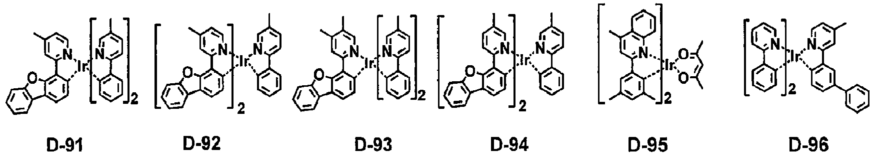

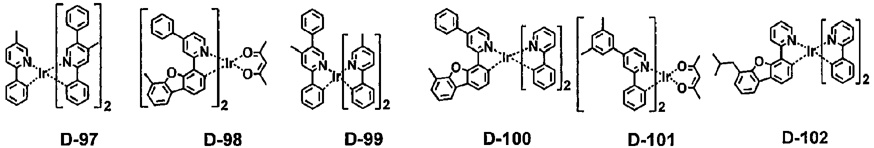

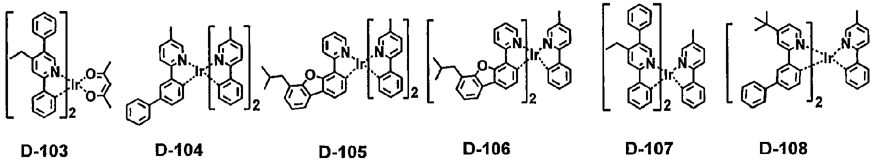

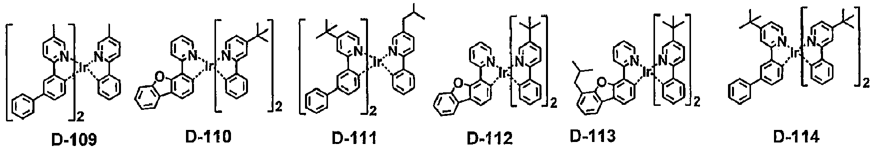

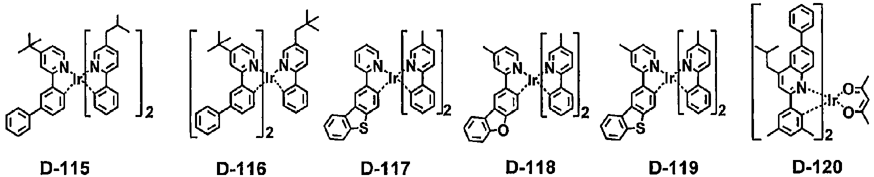

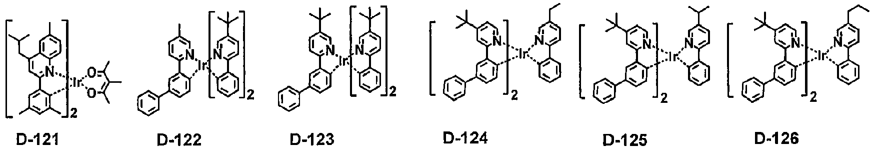

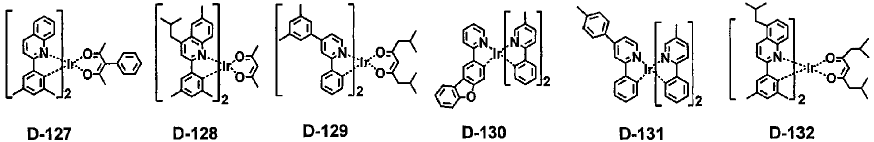

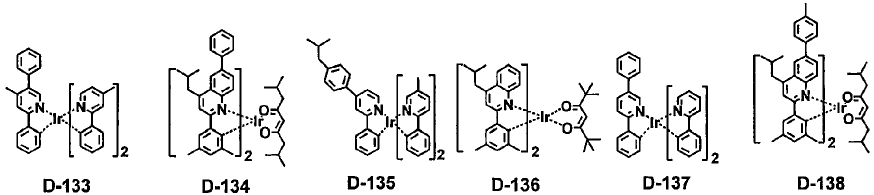

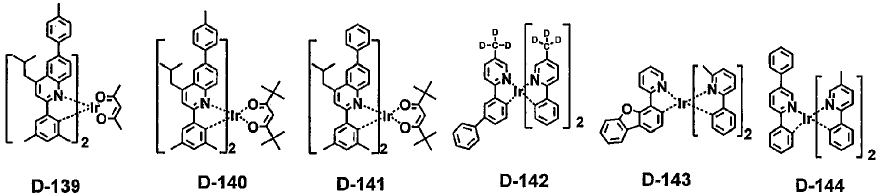

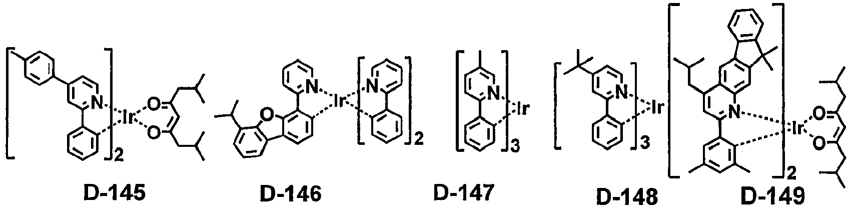

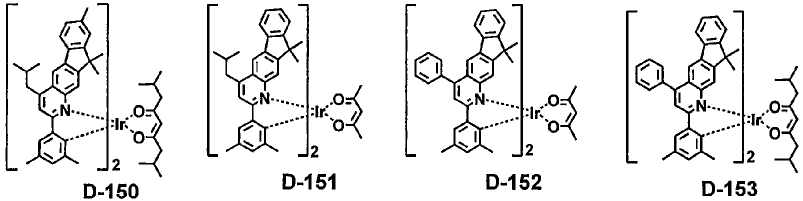

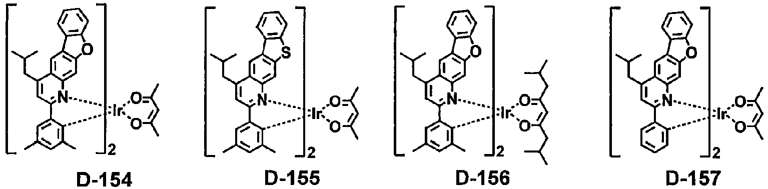

In order to form each layer of the organic electroluminescent device of the present disclosure, dry film-forming methods such as vacuum evaporation, sputtering, plasma, ion plating methods, etc., or wet film-forming methods such as ink jet printing, nozzle printing, slot coating, spin coating, dip coating, flow coating methods, etc., can be used. The first and the second host compounds of the present disclosure may be film-formed by a co-evaporation process or a mixture-evaporation process.

When using a wet film-forming method, a thin film can be formed by dissolving or diffusing materials forming each layer into any suitable solvent such as ethanol, chloroform, tetrahydrofuran, dioxane, etc. The solvent can be any solvent where the materials forming each layer can be dissolved or diffused, and where there are no problems in film-formation capability.

Also, the organic electroluminescent device of the present disclosure can be used for the manufacture of a display device or a lighting device.

Hereinafter, the preparation method of the organic electroluminescent compounds of the present disclosure, and the properties of the device comprising the compounds will be explained in detail with reference to the representative compounds of the present disclosure. However, the present disclosure is not limited by the following examples.

### Example 1: Preparation of compound C-5

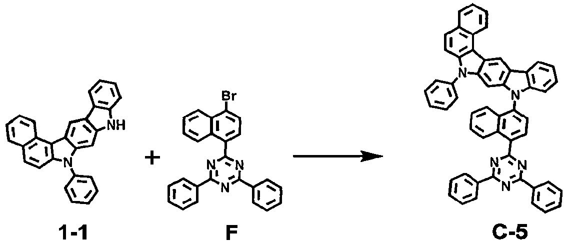

3 g of compound **1-1** (0.8 mmol), 4.1 g of compound **F** (0.9 mmol), 88 mg of palladium(II) acetate (Pd(OAc)₂) (0.392 mmol), 322 mg of 2-dicyclohexylphosphino-2',6'-dimethoxybiphenyl (S-phos) (0.784 mmol), 1.9 g of sodium tert-butoxide (NaOtBu) (2 mmol), and 80 mL of o-xylene were poured into a flask, and dissolved, and then stirred under reflux for 5 hours. After completion of the reaction, the reaction product was extracted with EA(ethyl acetate)/H₂O. The organic extract was purified by column chromatography to obtain 1.5 g of compound **C-5** (yield: 25.8 %).

¹H NMR (600MHz, CDCl₃, δ) 9.429 (s, 1H), 9.236-9.222 (d, *J* = 8.4Hz, 1H), 9.130-9.117 (d, *J* = 7.8Hz, 1H), 8.834-8.820 (d, *J* = 7.8Hz, 4H), 8.661-8.649 (d, *J* = 7.2Hz, 1H), 8.485-8.473 (d, *J* = 7.2HZ, 1H), 8.055-8.042 (d, *J* = 7.8Hz, 1H), 7.879-7.864 (m, 2H), 7.823-7.808 (d, *J* = 9Hz, 1H), 7.691-7.653 (m, 3H), 7.634-7.608 (m, 4H), 7.560-7.547 (m, 2H), 7.505-7.490 (m, 5H), 7.422-7.361 (m, 4H), 7.055 (s, 1H), 7.025-7.012 (d, *J* = 7.8Hz, 1H)

**[Table 1]**

| **Compound** | **MW** | **UV** | **PL** | **M.P.** |
|---|---|---|---|---|
| **C-5** | **739.86** | **458 nm** | **519 nm** | **299°C** |

### Example 2: Preparation of compound C-6

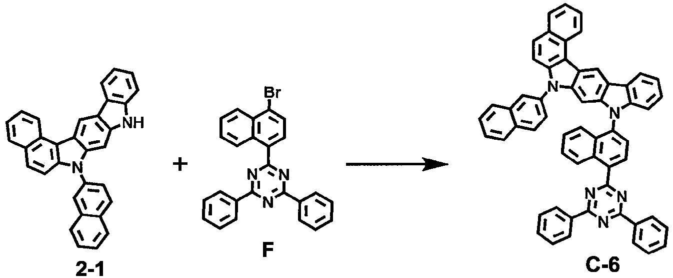

4 g of compound **2-1** (9.25 mmol), 4.5 g of compound **F** (10.17 mmol), 104 mg of palladium(II) acetate (Pd(OAc)₂) (0.46 mmol), 380 mg of 2-dicyclohexylphosphino-2',6'-dimethoxybiphenyl (S-phos) (0.92 mmol), 2.3 g of sodium tert-butoxide (NaOtBu) (23.57 mmol), and 46 mL of o-xylene were poured into a flask, and dissolved, and then stirred under reflux for 3 hours. After completion of the reaction, the reaction product was extracted with EA/H₂O. The organic extract was purified by column chromatography to obtain 4.0 g of compound **C-6** (yield: 56 %).
¹H NMR (600MHz, CDCl₃, δ) 9.443 (s, 1H), 9.18-9.17 (d, *J* = 6Hz, 1H), 9.141-9.127 (d, *J* = 9.3Hz, 1H), 8.778-8.764 (d, *J* = 8.1 Hz, 4H), 8.602-8.589 (d, *J* = 7.5Hz, 1H), 8.487-8.474 (d, *J* = 7.74Hz, 1H), 8.050-8.037 (d, *J* = 7.9Hz, 1H), 7.96 (s, 2H), 7.865-7.837 (m, 3H), 7.814-7.800 (d, *J* = 8.76Hz, 1H), 7.665-7.624 (m, 3H), 7.597-7.475 (m, 10H), 7.433-7.387 (m, 4H), 7.06 (s, 1H), 7.050-7.001 (m, 1H)

**[Table 2]**

| **Compound** | **MW** | **UV** | **PL** | **M.P.** |
|---|---|---|---|---|
| **C-6** | **789.92** | **454 nm** | **515 nm** | **199.5°C** |

### Example 3: Preparation of compound C-9

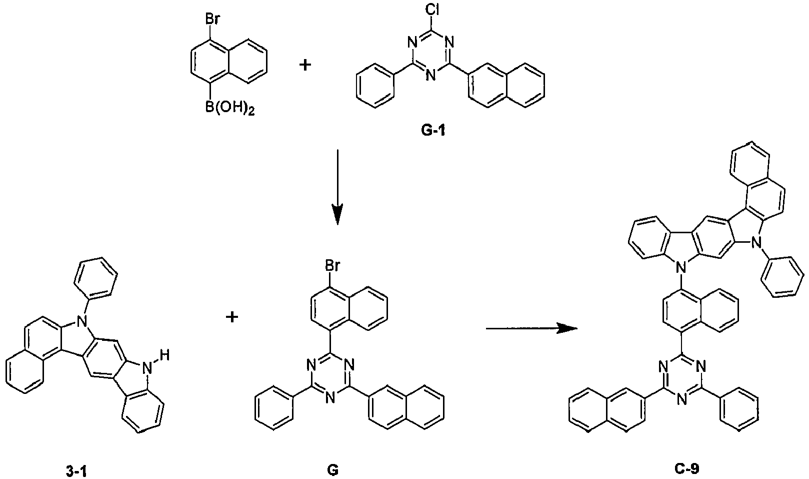

### Preparation of compound G

10 g of compound **G-1** (31 mmol), 4.47 g of (4-bromonaphthalene-1-yl)boronic acid (37 mmol), 1.82 g of tetrakis(triphenylphosphine)palladium (O) (Pd(PPh₃)₄) (1.6 mmol), 10 g of calcium carbonate (K₂CO₃) (94 mmol), 141 mL of toluene, 47 mL of ethanol, and 47 mL of water were poured into a flask, and dissolved, and then stirred under reflux for 3 hours. After completion of the reaction, the reaction product was extracted with EA/H₂O. The organic extract was purified by column chromatography to obtain 10.6 g of compound **G** (yield: 64 %).

### Preparation of compound C-9

7 g of compound **3-1** (18.3 mmol), 10.7 g of compound **G** (21.9 mmol), 200 mg of palladium(II) acetate (Pd(OAc)₂) (0.9 mmol), 0.75 g of 2-dicyclohexylphosphino-2',6'-dimethoxybiphenyl (S-phos) (1.8 mmol), 4.4 g of sodium tert-butoxide (NaOtBu) (45.7 mmol), and 183 mL of o-xylene were poured into a flask, and dissolved, and then stirred under reflux for 3 hours. After completion of the reaction, the reaction product was extracted with EA/H₂O. The organic extract was purified by column chromatography to obtain 5.5 g of compound **C-9** (yield: 38 %).
[²⁴¹] ¹H NMR (600MHz, CDCl₃) 9.427 (s, 1H), 9.379 (s, 1H), 9.254-9.239 (d, *J* = 9HZ, 1H) , 9.126-9.112 (d, *J* = 8.4Hz, 1H), 8.874-8.846 (m, 3H), 8.690-8.677 (d, *J* = 7.8HZ, 1H), 8.482-8.470 (d, *J* = 7.2Hz, 1H), 8.117-8.104 (d, *J* = 7.8Hz, 1H), 8.063-8.036 (m, 2H), 7.965-7.952 (d, *J* = 7.8Hz, 1H), 7.899-7.887 (d, *J* = 7.2Hz, 1H), 7.86-7.827 (m, 1H), 7.817-7.802 (d, *J* = 9Hz*,* 1H), 7.689-7.593 (m, 6H), 7.568-7.485 (m, 7H), 7.441-7.361 (m, 4H), 7.061 (s, 1H), 7.034-7.020 (d, *J* = 8.4Hz, 1H)

**[Table 3]**

| **Compound** | **MW** | **UV** | **PL** | **M.P.** |
|---|---|---|---|---|
| **C-9** | **789.94** | **354 nm** | **516 nm** | **280°C** |

### Example 4: Preparation of compound C-61

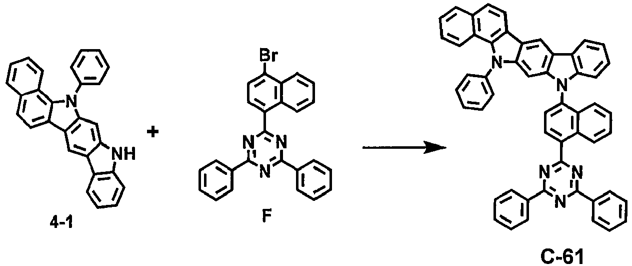

4.0 g of compound **4-1** (10 mmol), 5 g of compound **F** (12 mmol), 479 mg of tris(dibenzylideneacetone)dipalladium(O) (Pd₂(dba)₃) (0.523 mmol), 429 mg of 2-dicyclohexylphosphino-2',6'-dimethoxybiphenyl (S-phos) (1 mmol), 2.5 g of sodium tert-butoxide (NaOtBu) (26 mmol), and 87 mL of o-xylene were poured into a flask, and dissolved, and then stirred under reflux for 3 hours. After completion of the reaction, the reaction product was extracted with EA/H₂O. The organic extract was purified by column chromatography to obtain 1.3 g of compound **C-61** (yield: 16 %).
¹H NMR (600MHz, CDCl₃) 9.206-9.192 (d, *J* = 8.4Hz, 1H), 8.974 (s, 1H), 8.824-8.807 (m, 4H), 8.630-8.618 (d, *J* = 7.2Hz, 1H), 8.413-8.398 (d, *J* = 9Hz, 2H), 7.999-7.985 (d, *J* = 8.4Hz, 1H), 7.827-7.815 (d, *J* = 7.2Hz, 1H) 7.781-7.767 (d, *J* = 8.4Hz, 1H), 7.664-7.600 (m, 8H), 7.501-7.474 (m, 4H), 7.384-7.313 (m, 5H), 7.233-7.218 (d, *J* = 9Hz, 1H), 6.947-6.934 (d, 1H), 6.752 (s, 2H)

**[Table 4]**

| **Compound** | **MW** | **UV** | **PL** | **M.P.** |
|---|---|---|---|---|
| **C-61** | **739.88** | **344 nm** | **517 nm** | **192°C** |

### Example 5: Preparation of compound C-50

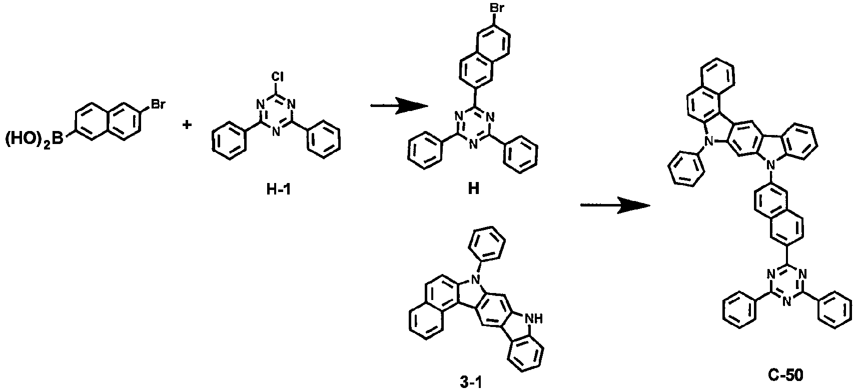

### Preparation of compound H

13.3 g of compound **H-1** (50 mmol), 15 g of (2-bromonaphthalene-6-yl)boronic acid (60 mmol), 3.4 g of tetrakis(triphenylphosphine)palladium (O) (Pd(PPh₃)₄) (3 mmol), 25 g of calcium carbonate (K₂CO₃) (179 mmol), 150 mL of toluene, 73 mL of ethanol, and 73 mL of water were poured into a flask, and dissolved, and then stirred under reflux for 3 hours. After completion of the reaction, the reaction product was extracted with EA/H₂O. The organic extract was purified by column chromatography to obtain 4 g of compound **H** (yield: 15 %).

### Preparation of compound C-50

2.4 g of compound **3-1** (6 mmol), 3 g of compound **H** (7 mmol), 114 mg of tris(dibenzylideneacetone)dipalladium(O) (Pd₂(dba)₃) (0.126 mmol), 103 mg of 2-dicyclohexylphosphino-2',6'-dimethoxybiphenyl (S-phos) (0.25 mmol), 1.5 g of sodium tert-butoxide (NaOtBu) (16 mmol), and 53 mL of o-xylene were poured into a flask, and dissolved, and then stirred under reflux for 3 hours. After completion of the reaction, the reaction product was extracted with EA/H₂O. The organic extract was purified by column chromatography to obtain 2 g of compound **C-50** (yield: 43 %).
¹H NMR (600MHz, CDCl₃) 9.418 (s, 1H), 9.353 (s, 1H), 9.087-9.073 (d, *J* = 8.4Hz, 1H), 8.915-8.901 (d, *J* = 8.4Hz, 1H), 8.850-8.834 (sd, *J* = 6Hz, 4H), 8.414-8.000 (d, *J* = 8.4Hz, 1H), 8.291-8.277 (d, *J* = 8.4Hz, 1H), 8.165 (s, 1H), 8.046-8.031 (d, *J* = 9Hz, 2H), 7.825-7.808 (m, 3H), 7.646-7.530 (m, 13H), 7.460-7.397 (m, 4H)

**[Table 5]**

| **Compound** | **MW** | **UV** | **PL** | **M.P.** |
|---|---|---|---|---|
| **C-50** | **739.88** | **326 nm** | **477 nm** | **315°C** |

Hereinafter, the luminescent properties of the organic light-emitting diode (OLED) device comprising the compound of the present disclosure will be explained in detail.

### Device Example 1: Producing an OLED device bv evaporating the compound of the present disclosure as a host

An OLED device was produced by using the organic electroluminescent compound according to the present disclosure. A transparent electrode indium tin oxide (ITO) thin film (10 Ω/sq) on a glass substrate for an OLED device (GEOMATEC CO., LTD., Japan) was subjected to an ultrasonic washing with acetone, ethanol, and distilled water, sequentially, and then was stored in isopropanol. The ITO substrate was then mounted on a substrate holder of a vacuum vapor deposition apparatus. Compound **HI-1** was introduced into a cell of the vacuum vapor deposition apparatus, and then the pressure in the chamber of the apparatus was controlled to 10⁶ torr. Thereafter, an electric current was applied to the cell to evaporate the above-introduced material, thereby forming a first hole injection layer having a thickness of 80 nm on the ITO substrate. Next, compound **HI-2** was introduced into another cell of the vacuum vapor deposition apparatus, and was evaporated by applying an electric current to the cell, thereby forming a second hole injection layer having a thickness of 5 nm on the first hole injection layer. Compound **HT-1** was then introduced into another cell of the vacuum vapor deposition apparatus, and was evaporated by applying an electric current to the cell, thereby forming a first hole transport layer having a thickness of 10 nm on the second hole injection layer. Compound **HT-3** was then introduced into another cell of the vacuum vapor deposition apparatus, and was evaporated by applying an electric current to the cell, thereby forming a second hole transport layer having a thickness of 60 nm on the first hole transport layer. After forming the hole injection layer and the hole transport layer, a light-emitting layer was formed thereon as follows: Compound **C-5** was introduced into one cell of the vacuum vapor depositing apparatus as a host, and compound **D-71** was introduced into another cell as a dopant. The dopant was deposited in a doping amount of 3 wt% based on the total amount of the host and dopant to form a light-emitting layer having a thickness of 40 nm on the second hole transport layer. Compound **ET-1** and compound **EI-1** were then introduced into the other two cells, and respectively evaporated at a rate of 1:1 to form an electron transport layer having a thickness of 30 nm on the light-emitting layer. After depositing compound **EI-1** as an electron injection layer having a thickness of 2 nm on the electron transport layer, an A1 cathode having a thickness of 80 nm was deposited on the electron injection layer by another vacuum vapor deposition apparatus. Thus, an OLED device was produced.

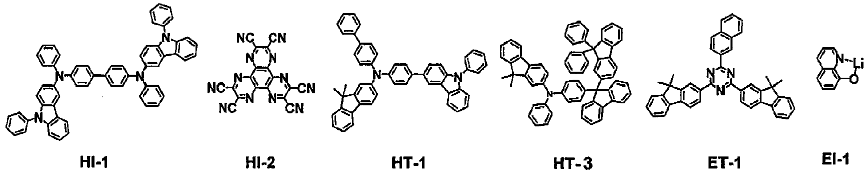

The lifespan (T97) was measured as the time taken to be reduced from 100% to 97% of the luminance at 5,000 nits and a constant current.

### Device Example 2: Producing an OLED device bv evaporating the compound of the present disclosure as a host

An OLED device was produced in the same manner as in Device Example 1, except for using compound **C-6** as a host.

### Device Example 3: Producing an OLED device bv evaporating the compound of the present disclosure as a host

An OLED device was produced in the same manner as in Device Example 1, except for using compound **C-9** as a host, and forming an electron transport layer having a thickness of 35 nm.

### Device Example 4: Producing an OLED device by evaporating the compound of the present disclosure as a host

An OLED device was produced in the same manner as in Device Example 1, except for using compound **C-61** as a host.

### Device Example 5: Producing an OLED device by evaporating the compound of the present disclosure as a host

An OLED device was produced in the same manner as in Device Example 1, except for using compound **C-50** as a host.

### Comparative Examples 1-1 to 1-5: Producing an OLED device using a conventional host

An OLED device was produced in the same manner as in Device Example 1, except for using the compounds of Comparative Examples 1-1 to 1-5 shown in Table 6 below as a host.

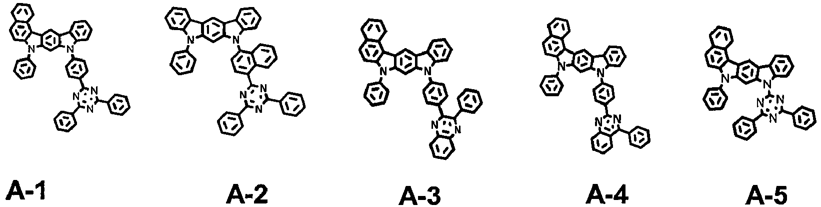

**[Table 6]**

| | **Host** | **Lifespan T97 [hr]** |
|---|---|---|
| **Device Example 1** | **C-5** | **193** |
| **Device Example 2** | **C-6** | **198** |
| **Device Example 3** | **C-9** | **188** |
| **Device Example 4** | **C-61** | **98** |
| **Device Example 5** | **C-50** | **105** |
| **Comparative Example 1-1** | **A-1** | **79** |
| **Comparative Example 1-2** | **A-2** | **95** |
| **Comparative Example 1-3** | **A-3** | **32** |
| **Comparative Example 1-4** | **A-4** | **67** |
| **Comparative Example 1-5** | **A-5** | **9** |

From the Device Examples and the Comparative Examples above, it can be seen that the OLED device comprising the organic electroluminescent compound of the present disclosure has improved lifespan properties, compared to the OLED device comprising a conventional organic electroluminescent compound.

## Claims

1. An organic electroluminescent compound represented by the following formula 1: 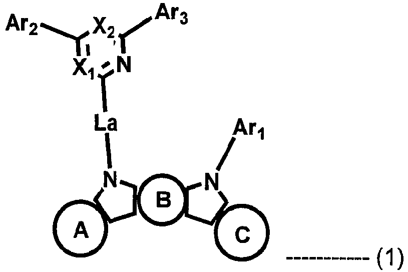 wherein
X₁ and X₂, each independently, represent N or CH, with the proviso, at least one of X₁ and X₂ represents N,
La represents a substituted or unsubstituted divalent (C10-C30) aromatic ring, which is one condensed ring,
A ring and C ring, each independently, represent a substituted or unsubstituted benzene ring, or a substituted or unsubstituted naphthalene ring, with the proviso, at least one of A ring and C ring represents a substituted or unsubstituted naphthalene ring,
B ring represents a substituted or unsubstituted benzene ring,
Ar₁, Ar₂ and Ar₃, each independently, represent hydrogen, deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino, and
the heteroaryl contains at least one heteroatom selected from B, N, O, S, Si, and P.

2. The organic electroluminescent compound according to claim 1,
wherein the substituents of the substituted (C1-C30)alkyl, the substituted (C6-C30)aryl, the substituted (3- to 30-membered)heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di- (C6-C30)arylamino, the substituted (C1-C30)alkyl(C6-C30)arylamino, the substituted divalent (C10-C30) aromatic ring, the substituted benzene ring, and the substituted naphthalene ring in La, Ar₁, Ar₂, Ar₃, A ring, B ring, and C ring, each independently, are at least one selected from the group consisting of deuterium; a halogen; a cyano; a carboxyl; a nitro; a hydroxyl; a (C1-C30)alkyl; a halo(C1-C30)alkyl; a (C2-C30)alkenyl; a (C2-C30)alkynyl; a (C1-C30)alkoxy; a (C1-C30)alkylthio; a (C3-C30)cycloalkyl; a (C3-C30)cycloalkenyl; a (3- to 7-membered)heterocycloalkyl; a (C6-C30)aryloxy; a (C6-C30)arylthio; a (5- to 30-membered)heteroaryl unsubstituted or substituted with a (C6-C30)aryl; a (C6-C30)aryl unsubstituted or substituted with a (5- to 30-membered)heteroaryl; a tri(C1-C30)alkylsilyl; a tri(C6-C30)arylsilyl; a di(C1-C30)alkyl(C6-C30)arylsilyl; a (C1-C30)alkyldi(C6-C30)arylsilyl; an amino; a mono- or di-(C1-C30)alkylamino; a mono- or di- (C6-C30)arylamino unsubstituted or substituted with a (C1-C30)alkyl; a (C1-C30)alkyl(C6-C30)arylamino; a (C1-C30)alkylcarbonyl; a (C1-C30)alkoxycarbonyl; a (C6-C30)arylcarbonyl; a di(C6-C30)arylboronyl; a di(C1-C30)alkylboronyl; a (C1-C30)alkyl(C6-C30)arylboronyl; a (C6-C30)aryl(C1-C30)alkyl; and a (C1-C30)alkyl(C6-C30)aryl.

3. The organic electroluminescent compound according to claim 1,
wherein formula 1 is represented by any one of the following formulas 2 to 5: 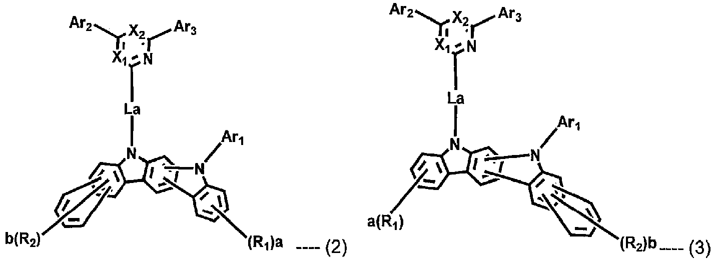 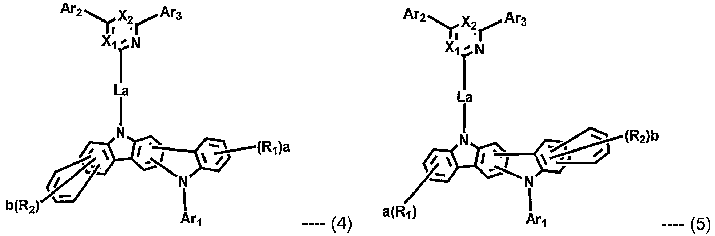 wherein
R₁ and R₂, each independently, represent deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino,
a represents an integer of 0 to 4, and b represents an integer of 0 to 6; where if a and b, each independently, represent an integer of 2 or more, each of R₁ and R₂ may be the same or different,
the heteroaryl contains at least one heteroatom selected from B, N, O, S, Si, and P, and
X₁, X₂, La, Ar₁, Ar₂, and Ar₃ are as defined in claim 1.

4. The organic electroluminescent compound according to claim 1,
wherein formula 1 is represented by any one of the following formulas 6 to 17: 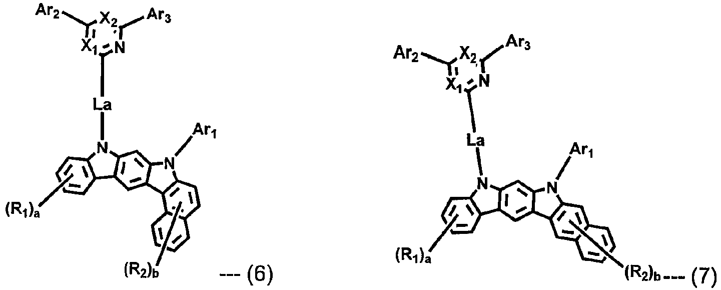 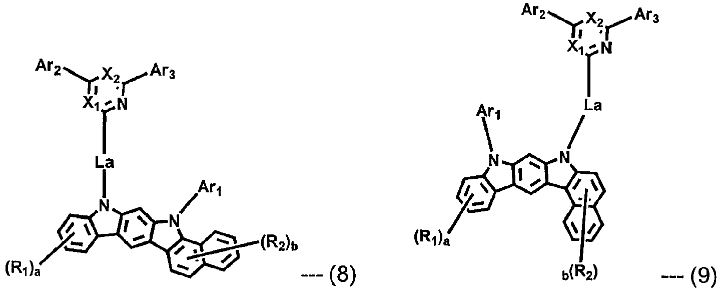 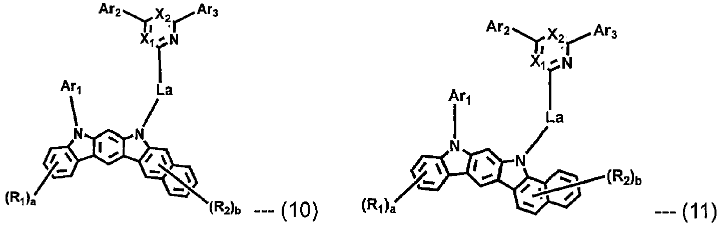 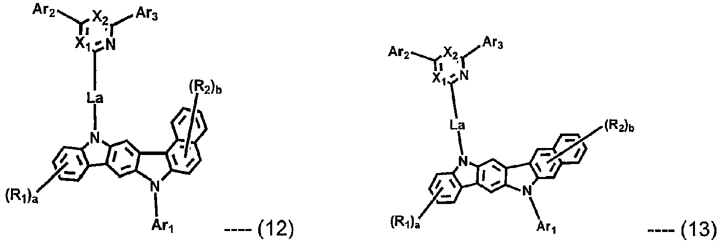 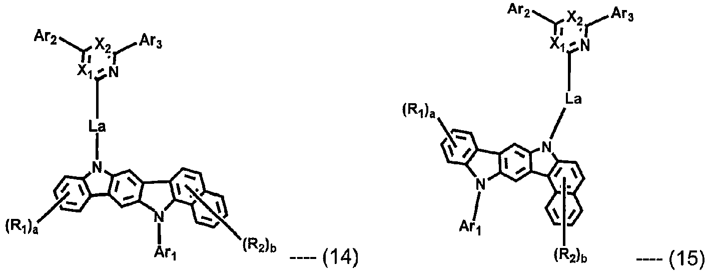 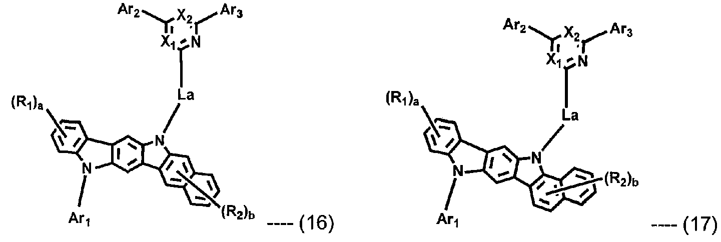 wherein
R₁ and R₂, each independently, represent deuterium, a halogen, a cyano, a substituted or unsubstituted (C1-C30)alkyl, a substituted or unsubstituted (C6-C30)aryl, a substituted or unsubstituted (3- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino,
a represents an integer of 0 to 4, and b represents an integer of 0 to 6; where if a and b, each independently, represent an integer of 2 or more, each of R₁ and R₂ may be the same or different,
the heteroaryl contains at least one heteroatom selected from B, N, O, S, Si, and P, and
X₁, X₂, La, Ar₁, Ar₂, and Ar₃ are as defined in claim 1.

5. The organic electroluminescent compound according to claim 1,
wherein La represents a naphthylene, an anthracenylene, a triphenylenylene, a dimethylbenzofluorenylene, or a phenanthrenylene.

6. The organic electroluminescent compound according to claim 1,
wherein any one of A ring and C ring represents a substituted or unsubstituted naphthalene ring, and the other represents a substituted or unsubstituted benzene ring; and Ar₁ to Ar₃, each independently, represent a substituted or unsubstituted (C6-C25)aryl, or a substituted or unsubstituted (5- to 25-membered)heteroaryl.

7. The organic electroluminescent compound according to claim 3,
wherein R₁ and R₂, each independently, represent a substituted or unsubstituted (C1-C20)alkyl, a substituted or unsubstituted (C6-C25)aryl, or a substituted or unsubstituted (5- to 25-membered)heteroaryl; and a and b, each independently, represent 0 or 1.

8. The organic electroluminescent compound according to claim 1,
wherein the compound represented by formula 1 is selected from the group consisting of:

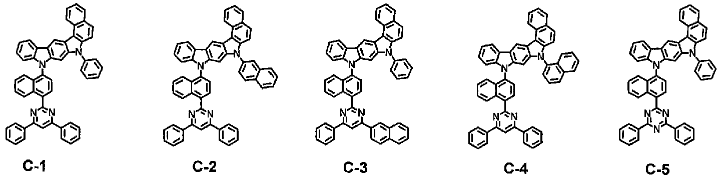

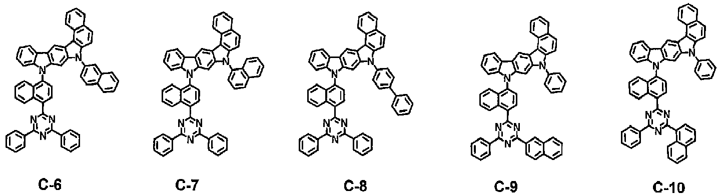

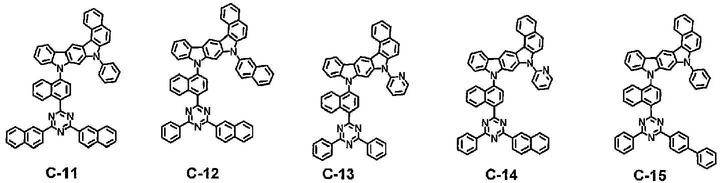

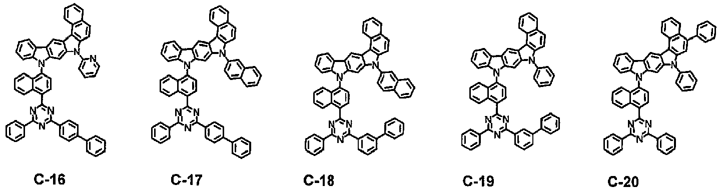

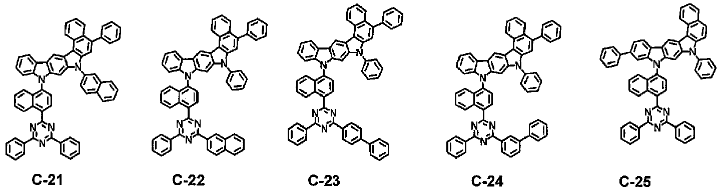

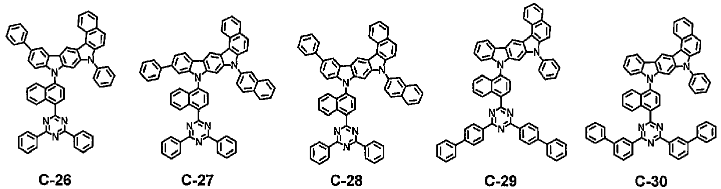

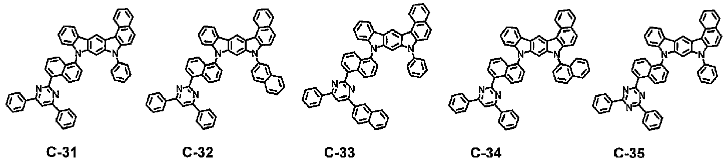

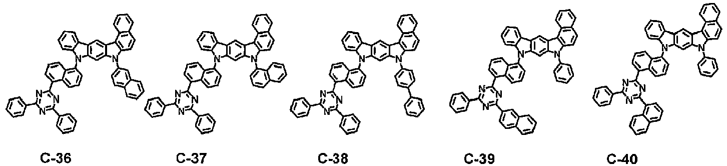

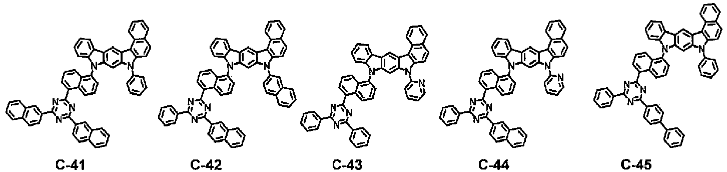

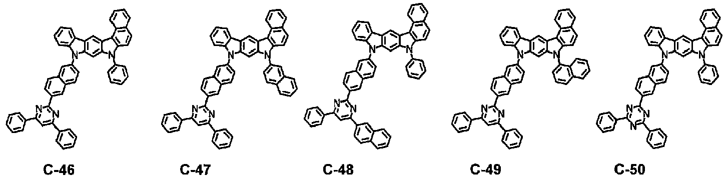

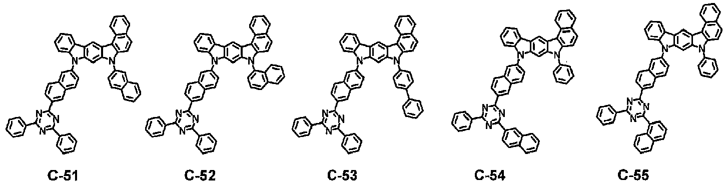

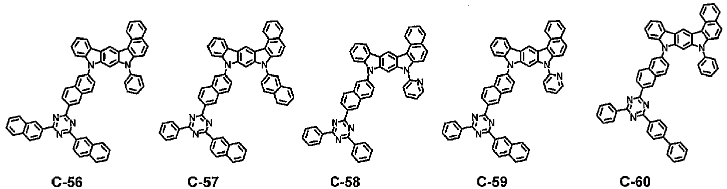

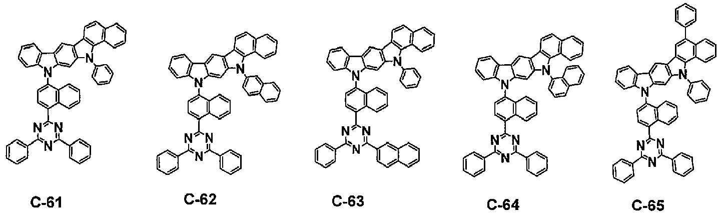

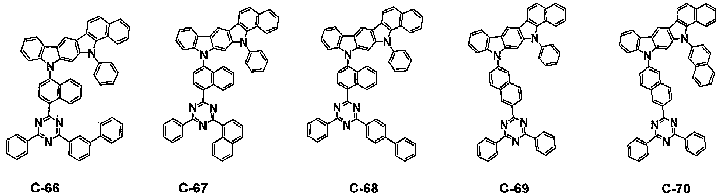

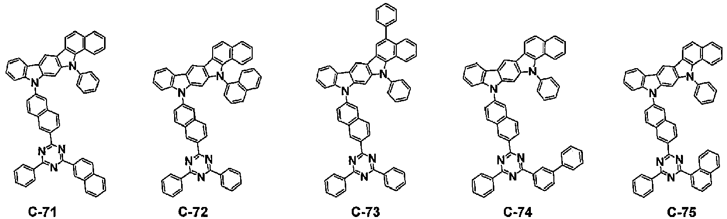

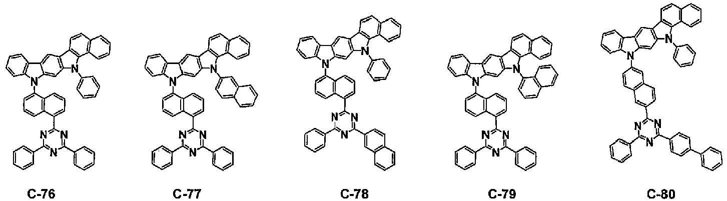

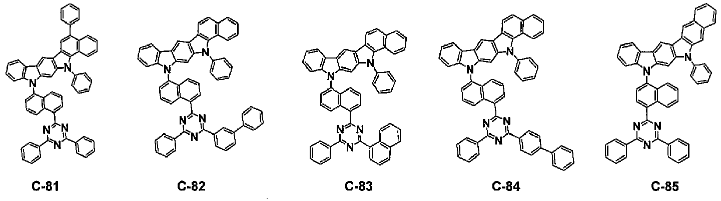

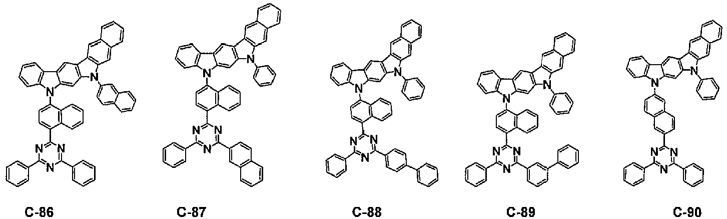

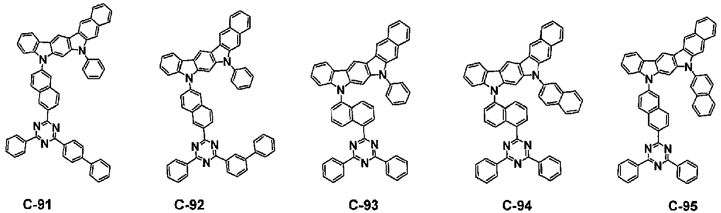

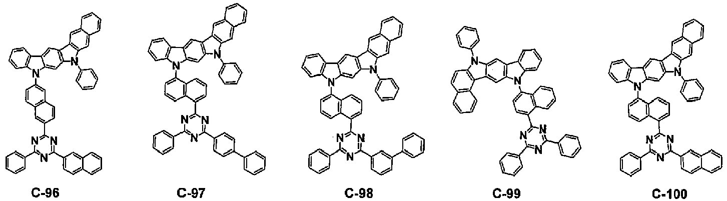

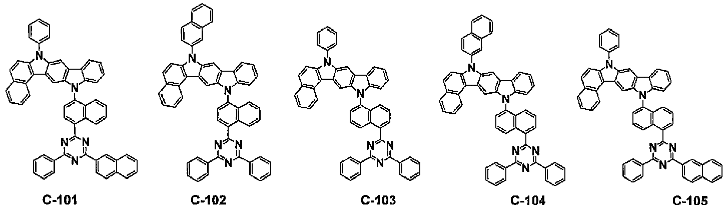

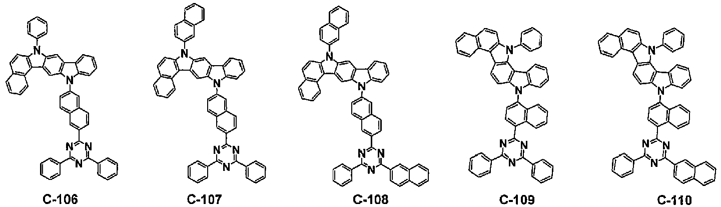

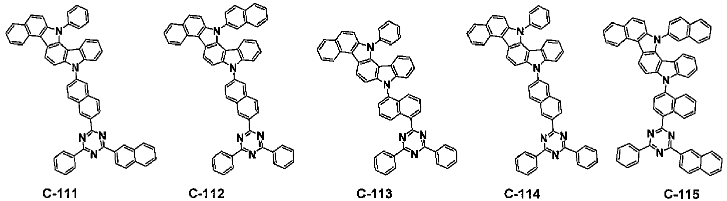

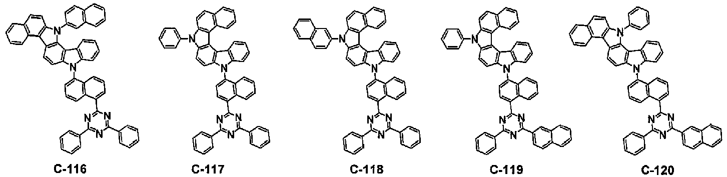

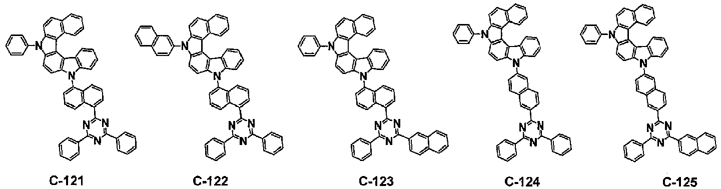

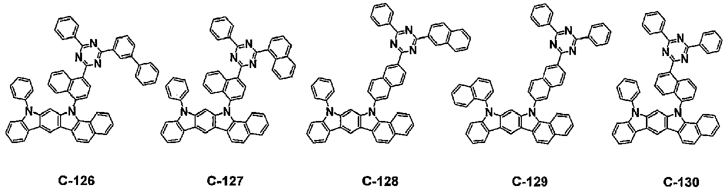

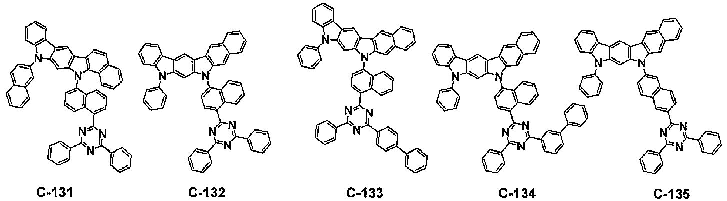

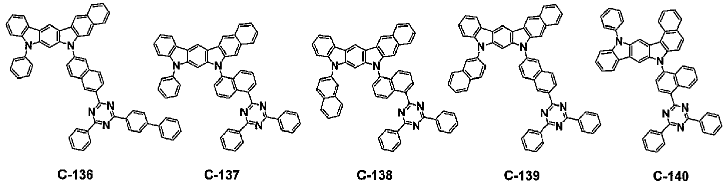

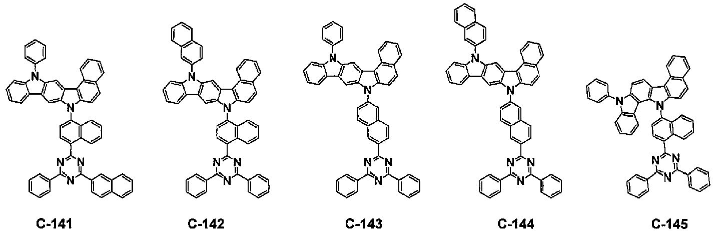

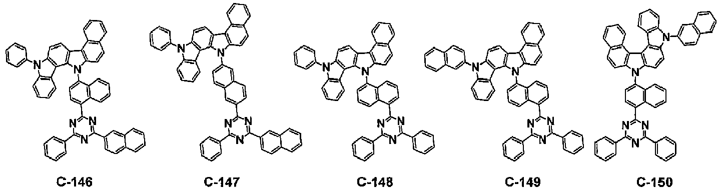

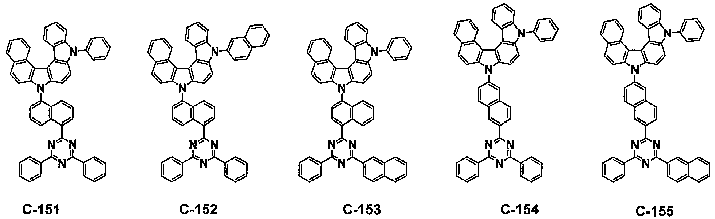

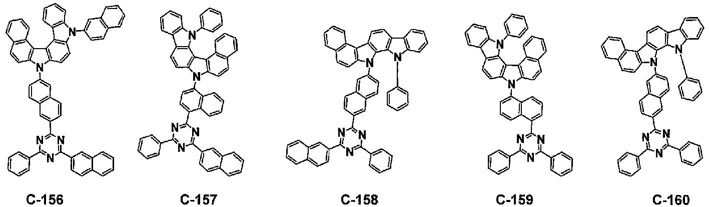

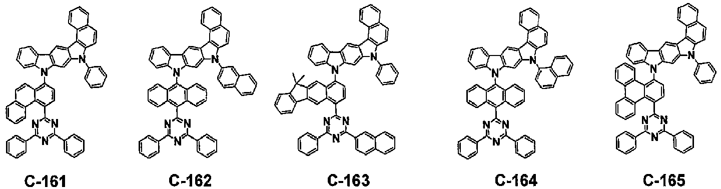

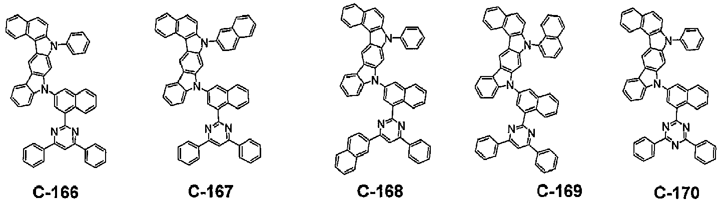

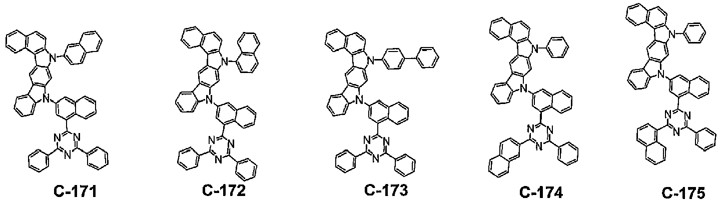

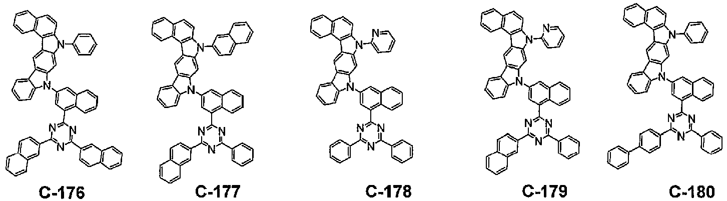

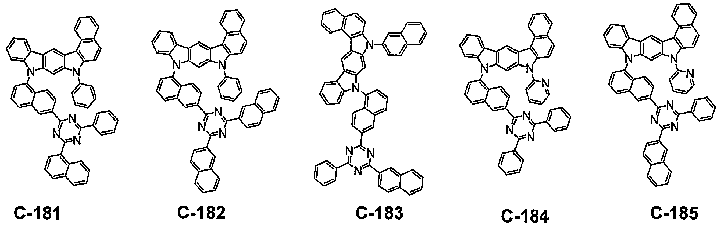

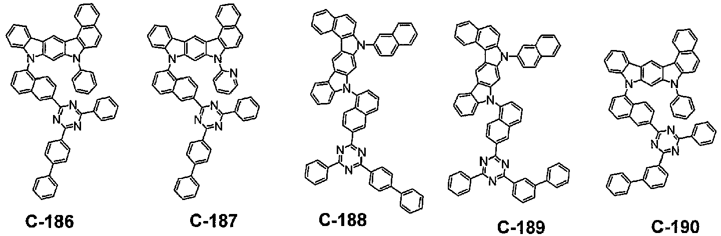

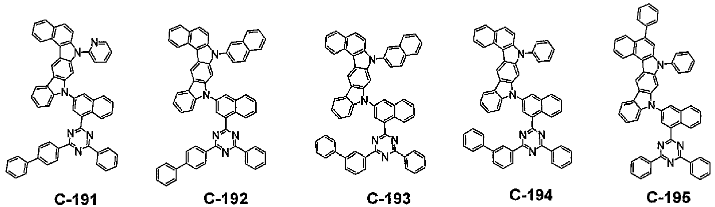

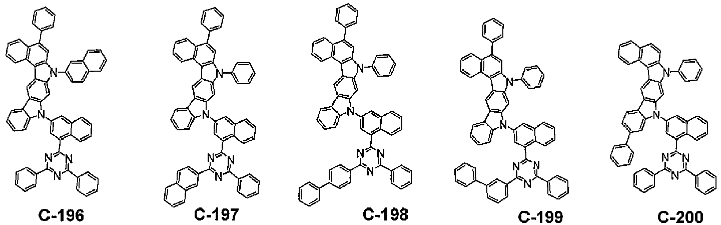

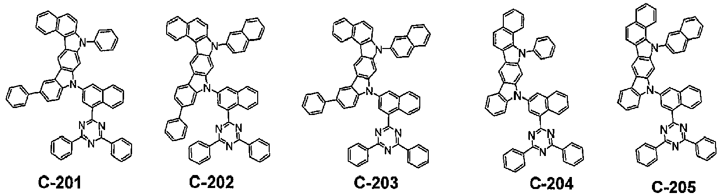

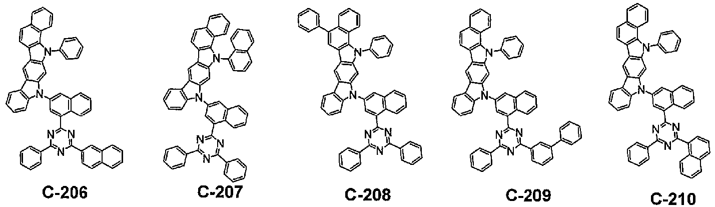

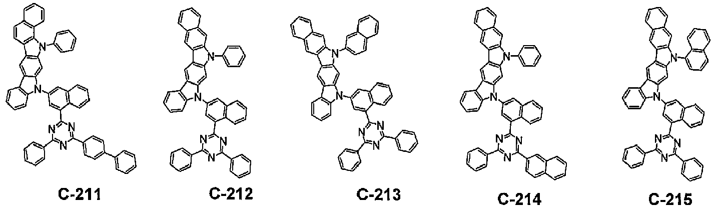

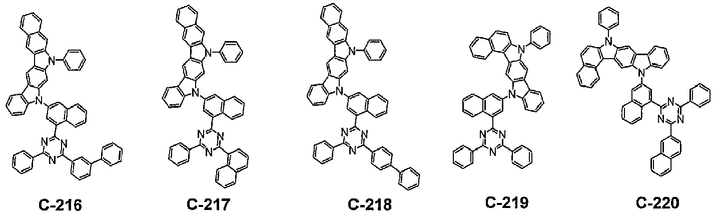

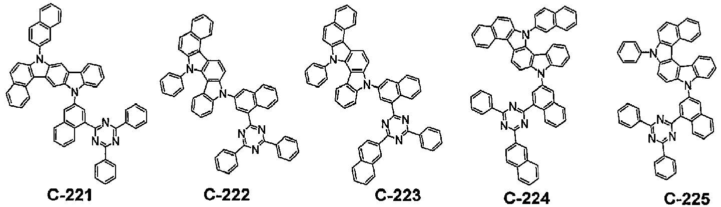

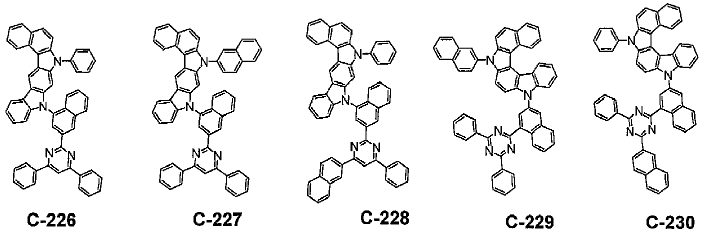

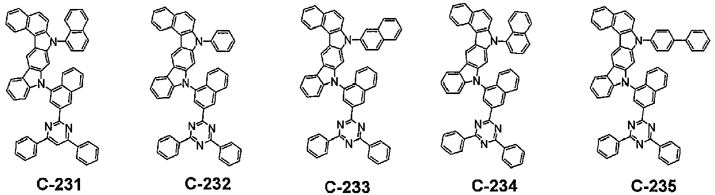

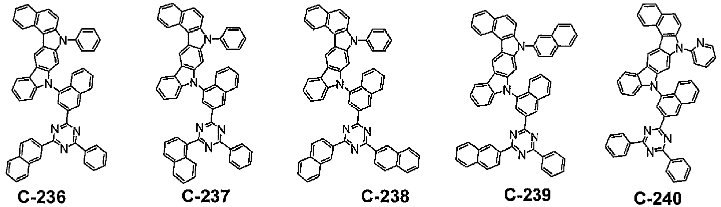

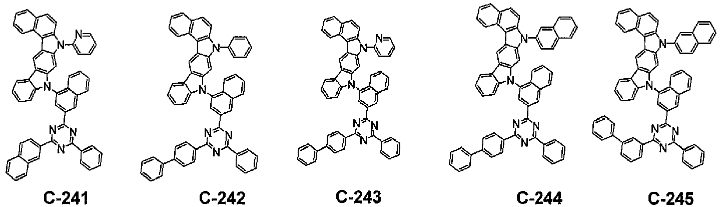

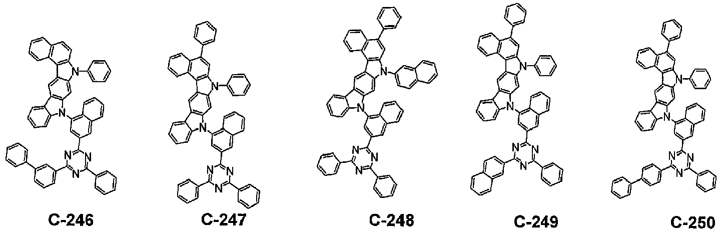

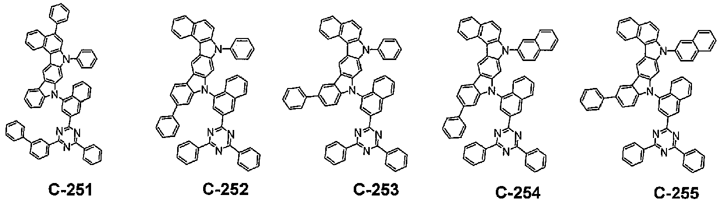

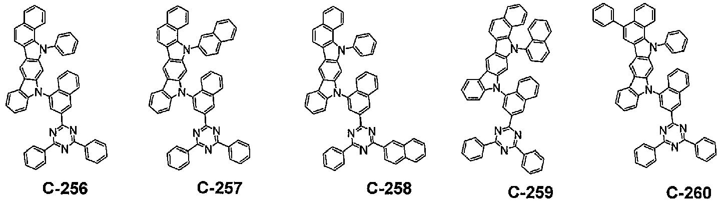

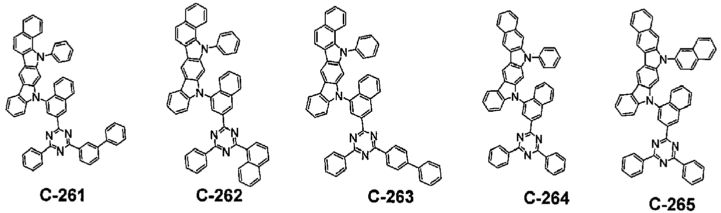

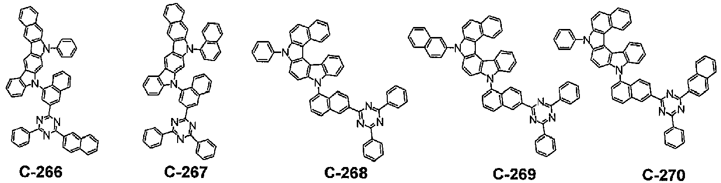

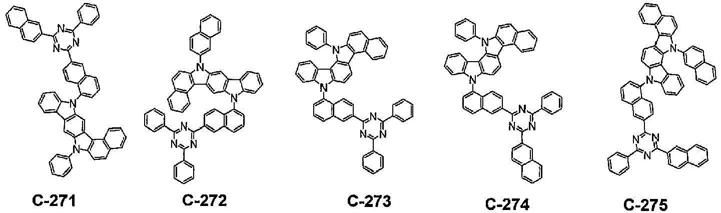

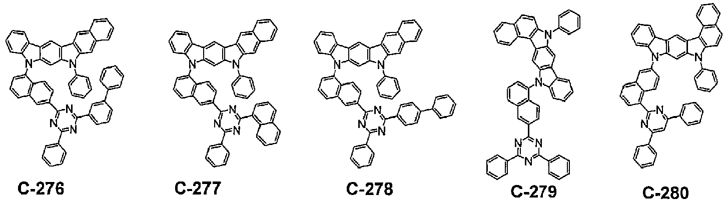

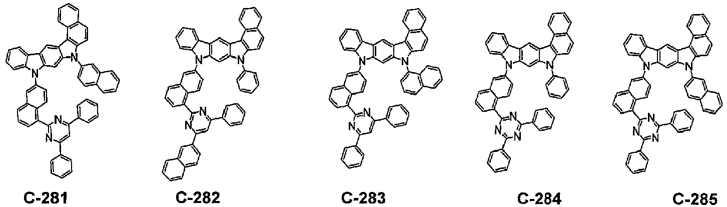

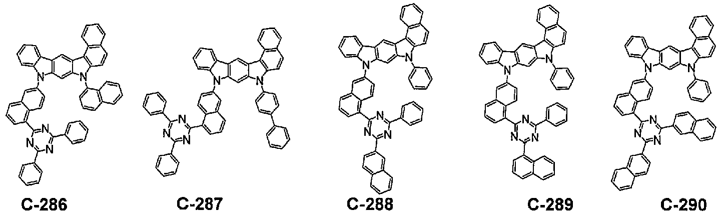

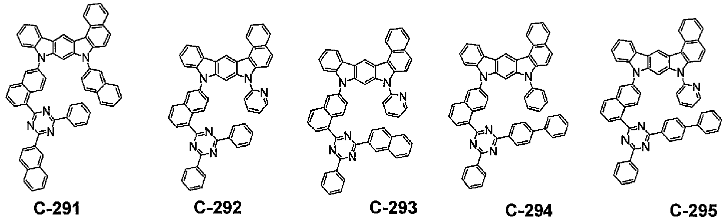

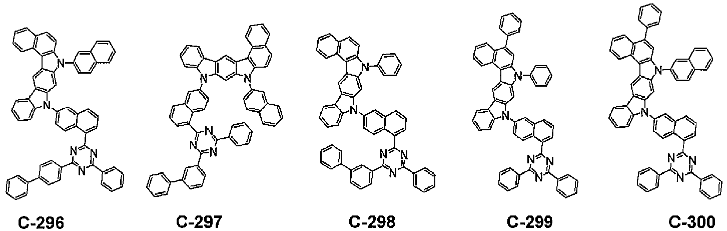

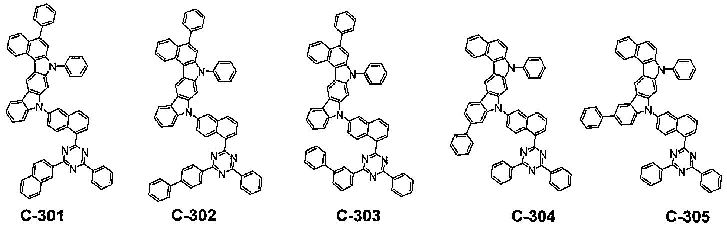

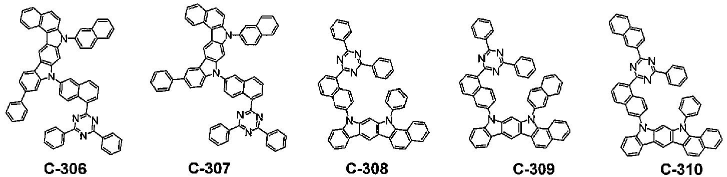

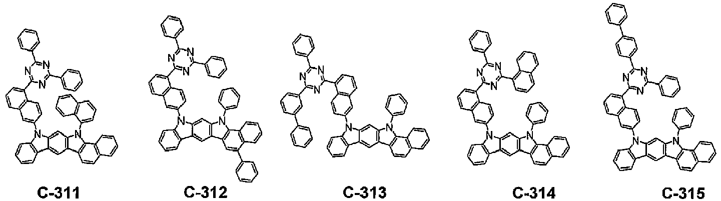

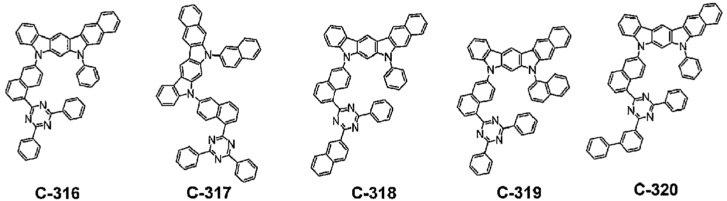

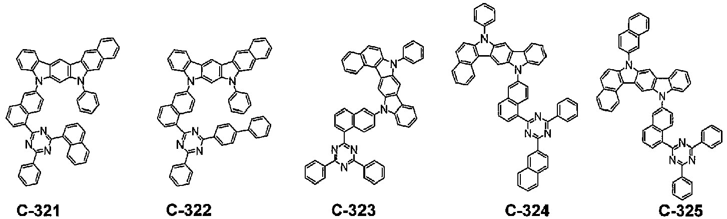

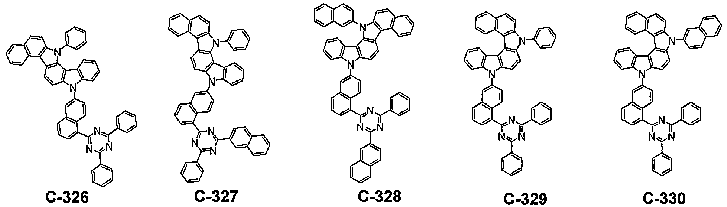

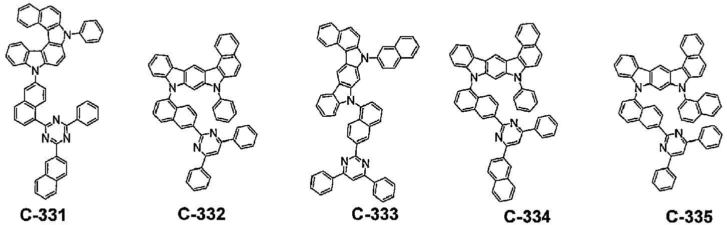

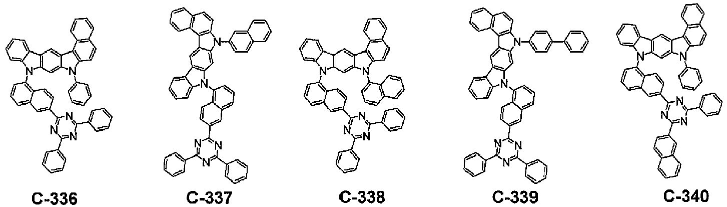

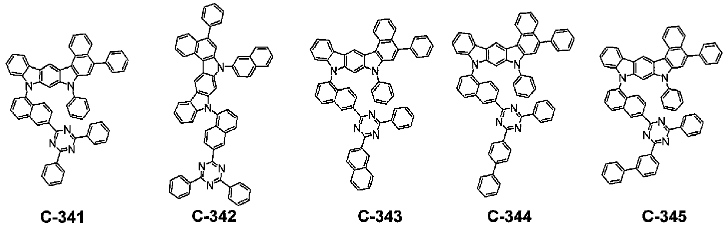

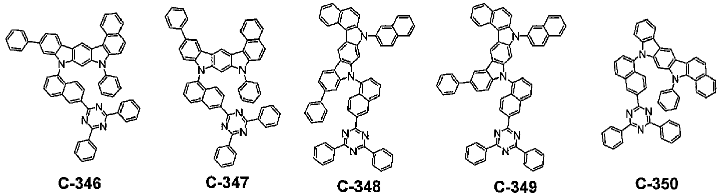

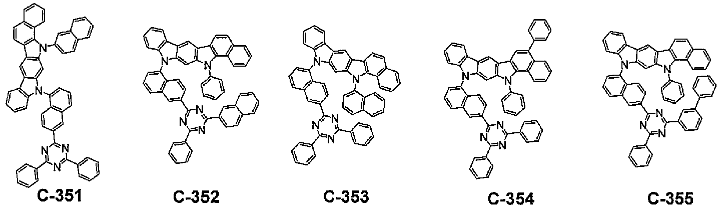

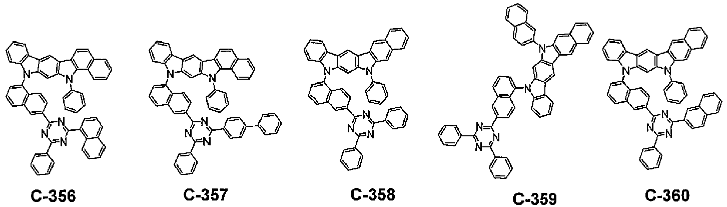

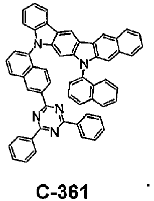

9. An organic electroluminescent material comprising the organic electroluminescent compound according to claim 1.

10. The organic electroluminescent material according to claim 9, wherein the organic electroluminescent material is a host material comprising a plurality of host compounds, and at least one of the host compounds is the organic electroluminescent compound represented by formula 1.

## Patentansprüche

1. Organische elektrolumineszente Verbindung, dargestellt durch die folgende Formel 1: 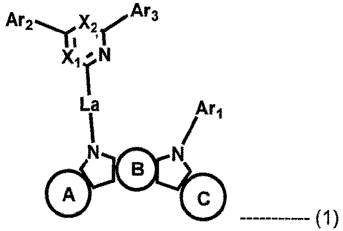 worin
X₁ und X₂ jeweils unabhängig dargestellt werden durch N oder CH mit der Maßgabe, dass mindestens eines von X₁ und X₂ N darstellt,
La einen substituierten oder unsubstituierten, zweiwertigen aromatischen (C10-C30)-Ring darstellt, bei dem es sich um einen kondensierten Ring handelt,
Ring A und Ring C jeweils unabhängig einen substituierten oder unsubstituierten Benzolring oder einen substituierten oder unsubstituierten Naphthalenring mit der Maßgabe darstellen, dass mindestens einer von Ring A und Ring C einen substituierten oder unsubstituierten Naphthalenring darstellt,
Ring B einen substituierten oder unsubstituierten Benzolring darstellt,
Ar₁, Ar₂ und Ar₃ jeweils unabhängig Wasserstoff, Deuterium, ein Halogen, ein Cyano, ein substituiertes oder unsubstituiertes (C1-C30)-Alkyl, ein substituiertes oder unsubstituiertes (C6-C30)-Aryl, ein substituiertes oder unsubstituiertes (3- bis 30-gliedriges) Heteroaryl, ein substituiertes oder unsubstituiertes (C3-C30)-Cycloalkyl, ein substituiertes oder unsubstituiertes (C1-C30)-Alkoxy, ein substituiertes oder unsubstituiertes Tri(C1-C30)-Alkylsilyl, ein substituiertes oder unsubstituiertes Di-(C1-C30)-Alkyl(C6-C30)-Arylsilyl, ein substituiertes oder unsubstituiertes (C1-C30)-Alkyl-di-(C6-C30)-Arylsilyl, ein substituiertes oder unsubstituiertes Tri-(C6-C30)-Arylsilyl, ein substituiertes oder unsubstituiertes Mono- oder Di-(C1-C30)-Alkylamino, ein substituiertes oder unsubstituiertes Mono- oder Di-(C6-C30)-Arylamino oder ein substituiertes oder unsubstituiertes (C1-C30)-Alkyl-(C6-C30)-Arylamino darstellen, und
das Heteroaryl mindestens ein Heteroatom enthält, das ausgewählt ist aus B, N, O, S, Si und P.

2. Organische elektrolumineszente Verbindung nach Anspruch 1, wobei die Substituenten des substituierten (C1-C30)-Alkyls, des substituierten (C6-C30)-Aryls, des substituierten (3- bis 30-gliedrigen) Heteroaryls, des substituierten (C3-C30)-Cycloalkyls, des substituierten (C1-C30)-Alkoxys, des substituierten Tri-(C1-C30)-Alkylsilyls, des substituierten Di-(C1-C30)-Alkyl-(C6-C30)-Arylsilyls, des substituierten (C1-C30)-Alkyl-di-(C6-C30)-Arylsilyls, des substituierten Tri-(C6-C30)-Arylsilyls, des substituierten Mono- oder Di-(C1-C30)-Alkylaminos, des substituierten Mono- oder Di-(C6-C30)-Arylaminos, des substituierten (C1-C30)-Alkyl-(C6-C30)-Arylaminos, des substituierten, zweiwertigen aromatischen (C10-C30)-Rings, des aromatischen Benzolrings und des substituierten Naphthalenrings in La, Ar₁, Ar₂, Ar₃, Ring A, Ring B und Ring C jedes unabhängig voneinander mindestens eines der folgenden ist, das ausgewählt ist aus der Gruppe bestehend aus Deuterium; einem Halogen; einem Cyano; einem Carboxyl; einem Nitro; einem Hydroxy; einem (C1-C30)-Alkyl; einem Halogen-(C1-C30)-Alkyl; einem (C2-C30)-Alkenyl; einem (C2-C30)-Alkinyl; einem (C1-C30)-Alkoxy; einem (C1-C30)-Alkylthio; einem (C3-C30)-Cycloalkyl; einem (C3-C30)-Cycloalkenyl; einem (3- bis 7-gliedrigen) Heterocycloalkyl; einem (C6-C30)-Aryloxy; einem (C6-C30)-Arylthio; einem (5- bis 30-gliedrigen) Heteroaryl, das unsubstituiert oder substituiert ist mit einem (C6-C30)-Aryl; einem (C6-C30)-Aryl, das unsubstituiert oder substituiert ist mit einem (5- bis 30-gliedrigen) Heteroaryl; einem Tri-(C1-C30)-Alkylsilyl; einem Tri-(C6-C30)-Arylsilyl; einem Di-(C1-C30)-Alkyl-(C6-C30)-Arylsilyl; einem (C1-C30)-Alkyldi-(C6-C30)-Arylsilyl; einem Amino; einem Mono- oder Di-(C1-C30)-Alkylamino; einem Mono- oder Di-(C6-C30)-Arylamino, das unsubstituiert oder substituiert ist mit einem (C1-C30)-Alkyl; einem (C1-C30)-Alkyl-(C6-C30)-Arylamino; einem (C1-C30)-Alkylcarbonyl; einem (C1-C30)-Alkoxycarbonyl; einem (C6-C30)-Arylcarbonyl; einem Di-(C6-C30)-Arylboronyl; einem Di-(C1-C30)-Alkylboronyl; einem (C1-C30)-Alkyl-(C6-C30)-Arylboronyl; einem (C6-C30)-Aryl-(C1-C30)-Alkyl; und einem (C1-C30)-Alkyl-(C6-C30)-Aryl.

3. Organische elektrolumineszente Verbindung nach Anspruch 1,
wobei Formel 1 dargestellt wird durch eine der folgenden Formeln 2 bis 5: 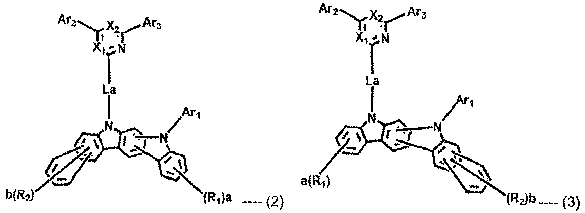 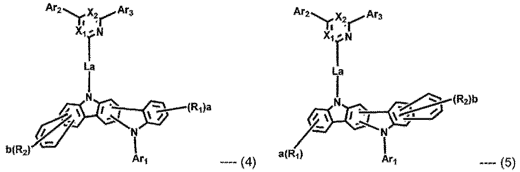 worin
R₁ und R₂ jeweils unabhängig voneinander Deuterium, ein Halogen, ein Cyano, ein substituiertes oder unsubstituiertes (C1-C30)-Alkyl, ein substituiertes oder unsubstituiertes (C6-C30)-Aryl, ein substituiertes oder unsubstituiertes (3- bis 30-gliedriges) Heteroaryl, ein substituiertes oder unsubstituiertes (C3-C30)-Cycloalkyl, ein substituiertes oder unsubstituiertes (C1-C30)-Alkoxy, ein substituiertes oder unsubstituiertes Tri-(C1-C30)-Alkylsilyl, ein substituiertes oder unsubstituiertes Di-(C1-C30)-Alkyl-(C6-C30)-Arylsilyl, ein substituiertes oder unsubstituiertes (C1-C30)-Alkyldi-(C6-C30)-Arylsilyl, ein substituiertes oder unsubstituiertes Tri-(C6-C30)-Arylsilyl, ein substituiertes oder unsubstituiertes Mono-oder Di-(C1-C30)-Alkylamino, ein substituiertes oder unsubstituiertes Mono- oder Di-(C6-C30)-Arylamino oder ein substituiertes oder unsubstituiertes (C1-C30)-Alkyl-(C6-C30)-Arylamino darstellen,
a eine ganze Zahl von 0 bis 4 darstellt und b eine ganze Zahl von 0 bis 6 darstellt;
wobei, wenn a und b jeweils unabhängig voneinander eine ganze Zahl von 2 oder mehr darstellen, jedes von R₁ und R₂ gleich oder verschieden sein kann,
das Heteroaryl mindestens ein Heteroatom enthält, das ausgewählt ist aus B, N, O, S, Si und P, und
X₁, X₂, La, Ar₁, Ar₂ und Ar₃ wie in Anspruch 1 festgelegt sind.

4. Organische elektrolumineszente Verbindung nach Anspruch 1,
wobei die Formel 1 dargestellt wird durch eine der folgenden Formeln 6 bis 17: 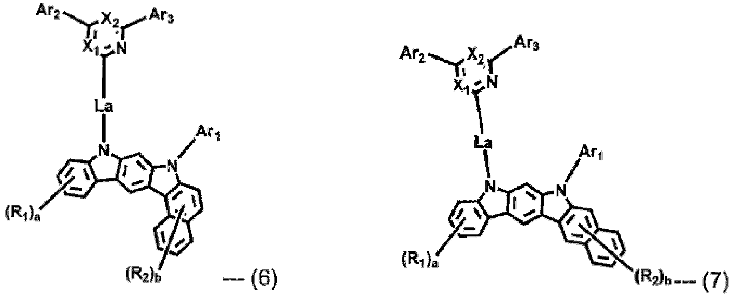 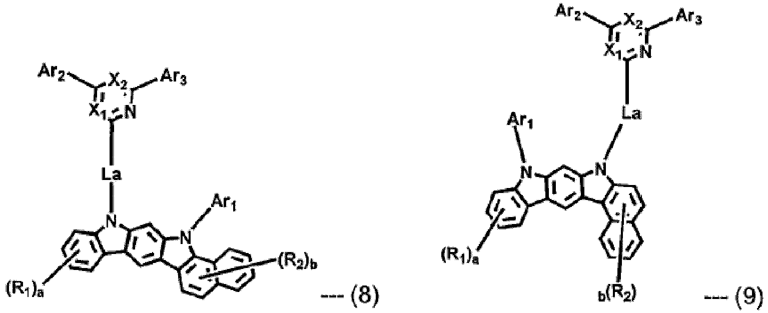 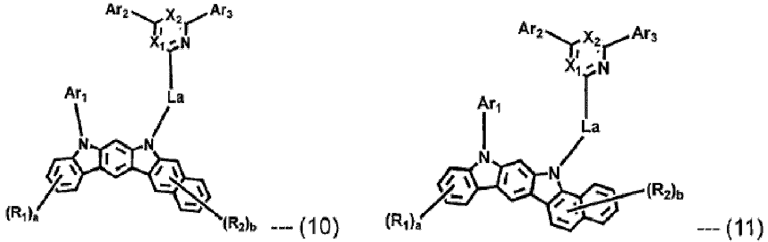 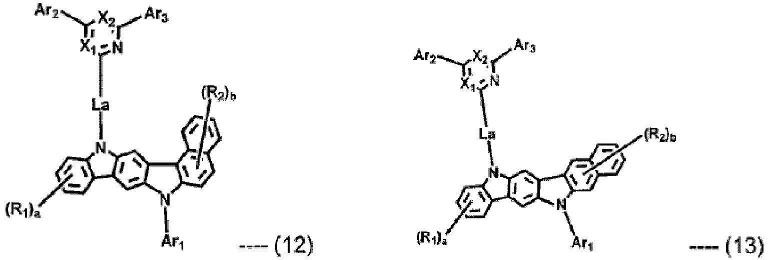 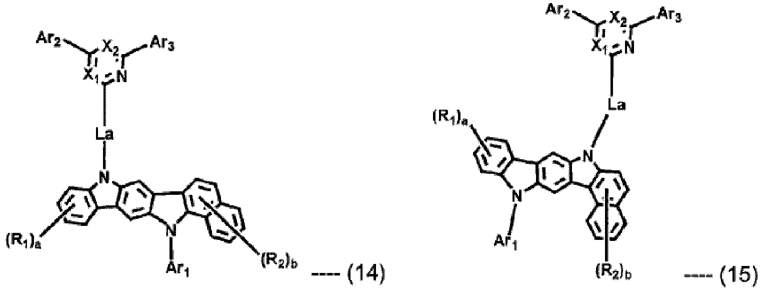 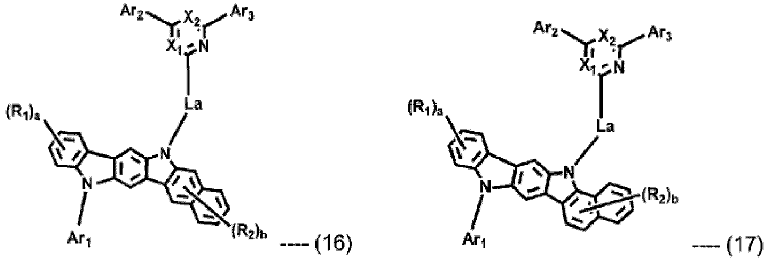 worin
R₁ und R₂ unabhängig voneinander darstellen: Deuterium, ein Halogen, ein Cyano, ein substituiertes oder unsubstituiertes (C1-C30)-Alkyl, ein substituiertes oder unsubstituiertes (C6-C30)Aryl, ein substituiertes oder unsubstituiertes (3- bis 30-gliedriges) Heteroaryl, ein substituiertes oder unsubstituiertes (C3-C30)-Cycloalkyl, ein substituiertes oder unsubstituiertes (C1-C30)-Alkoxy, ein substituiertes oder unsubstituiertes Tri-(C1-C30)-Alkylsilyl, ein substituiertes oder unsubstituiertes Di-(C1-C30)-Alkyl-(C6-C30)-Arylsilyl, ein substituiertes oder unsubstituiertes (C1-C30)-Alkyldi-(C6-C30)-Arylsilyl, ein substituiertes oder unsubstituiertes Tri-(C6-C30)-Arylsilyl, ein substituiertes oder unsubstituiertes Mono- oder Di-(C1-C30)-Alkylamino, ein substituiertes oder unsubstituiertes Mono- oder Di-(C6-C30)-Arylamino oder ein substituiertes oder unsubstituiertes (C1-C30)-Alkyl-(C6-C30)-Arylamino,
a eine ganze Zahl von 0 bis 4 darstellt und b eine ganze Zahl von 0 bis 6 darstellt;
wobei, wenn a und b jeweils unabhängig voneinander eine ganze Zahl von 2 oder mehr darstellen, jedes von R₁ und R₂ gleich oder verschieden sein kann,
das Heteroaryl mindestens ein Heteroatom enthält, das ausgewählt ist aus B, N, O, S, Si und P enthält, und
X₁, X₂, La, Ar₁, Ar₂ und Ar₃ wie in Anspruch 1 festgelegt sind.

5. Organische elektrolumineszente Verbindung nach Anspruch 1,
wobei La ein Naphthylen, ein Anthracenylen, ein Triphenylenylen, ein Dimethylbenzofluorenylen oder ein Phenanthrylen darstellt.

6. Organische elektrolumineszente Verbindung nach Anspruch 1,
wobei beliebig einer von Ring A und Ring C einen substituierten oder unsubstituierten Naphthalenring darstellt und der andere einen substituierten oder unsubstituierten Benzolring darstellt; und Ar₁ bis Ar₃ jeweils unabhängig ein substituiertes oder unsubstituiertes (C6-C25)-Aryl oder ein substituiertes oder unsubstituiertes (5-bis 25-gliedriges) Heteroaryl darstellen.

7. Organische elektrolumineszente Verbindung nach Anspruch 3,
wobei R₁ und R₂ jeweils unabhängig voneinander ein substituiertes oder unsubstituiertes (C1-C20)-Alkyl, ein substituiertes oder unsubstituiertes (C6-C25)-Aryl oder ein substituiertes oder unsubstituiertes (5- bis 25-gliedriges) Heteroaryl darstellen; und a und b jeweils unabhängig voneinander für 0 oder 1 betragen.

8. Organische elektrolumineszente Verbindung nach Anspruch 1,
wobei die durch Formel 1 dargestellte Verbindung ausgewählt ist aus der Gruppe bestehend aus:

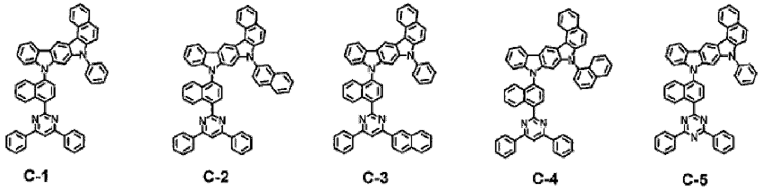

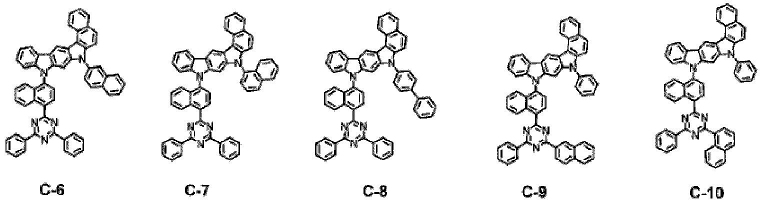

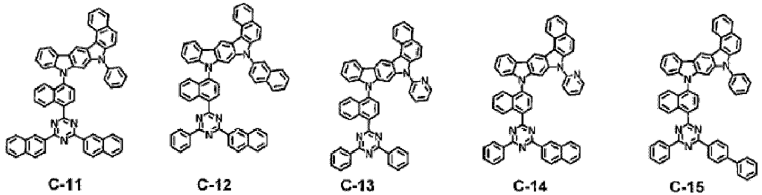

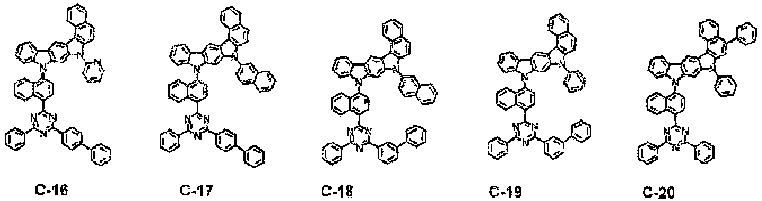

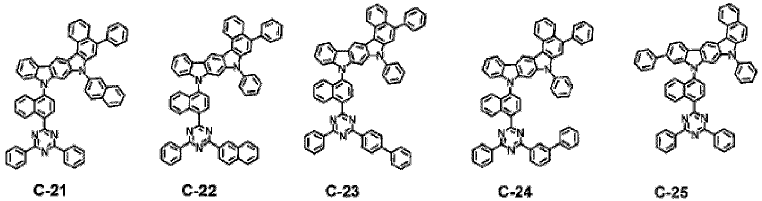

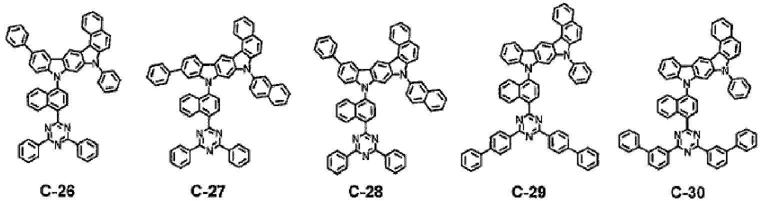

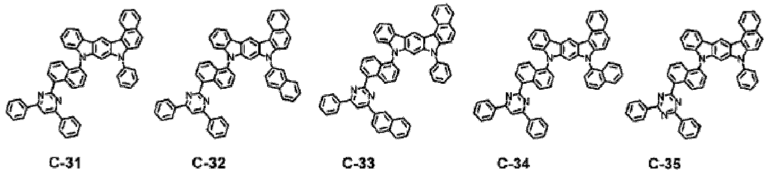

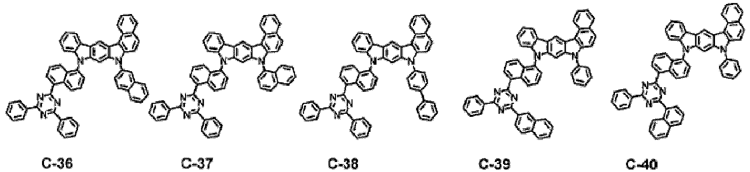

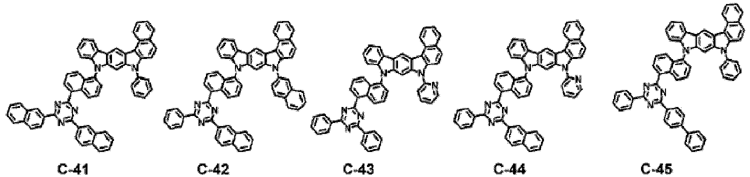

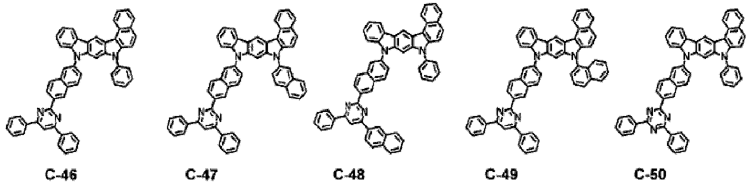

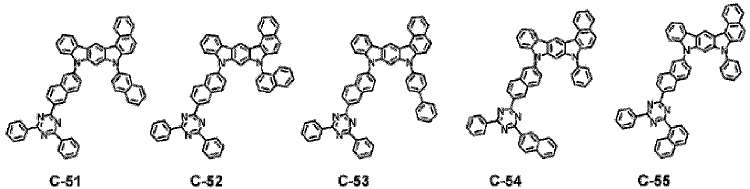

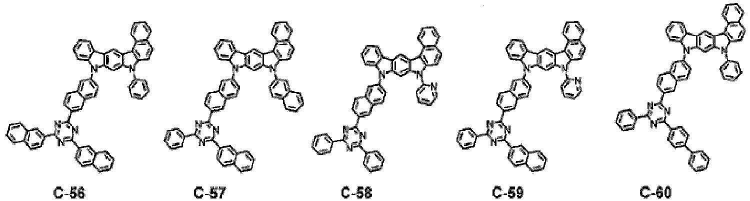

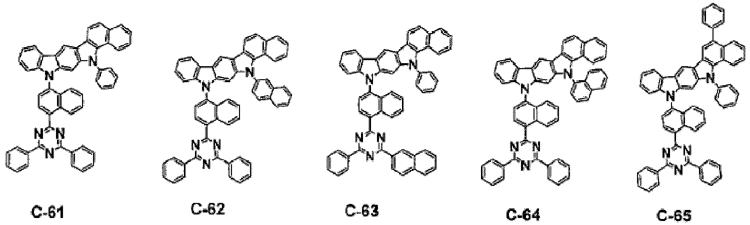

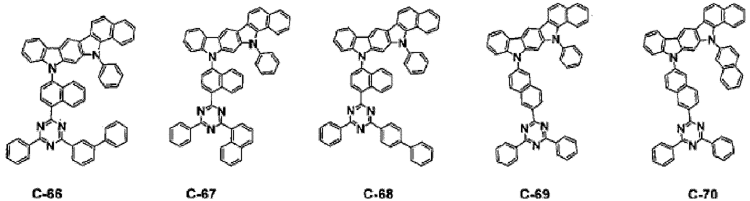

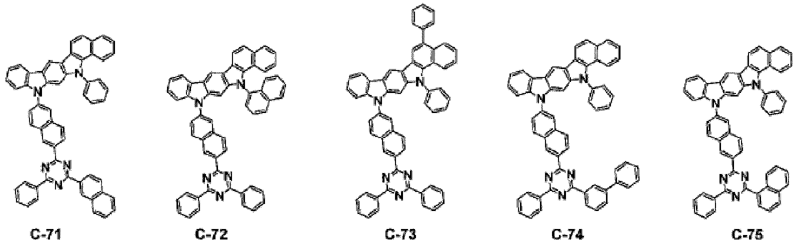

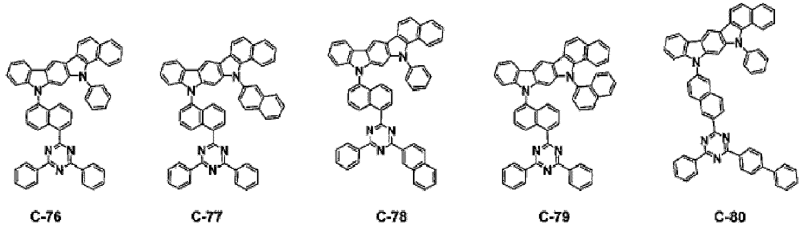

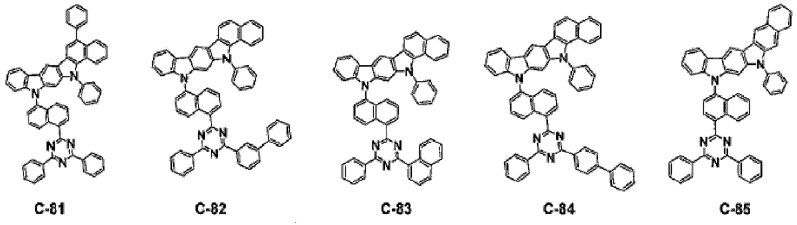

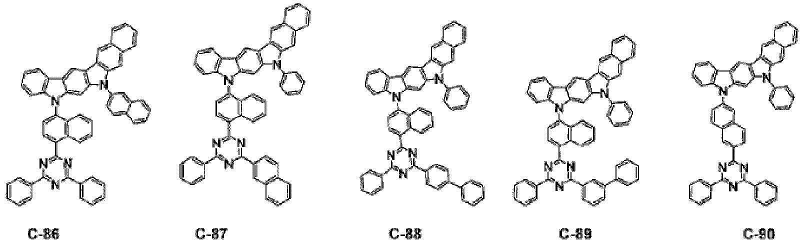

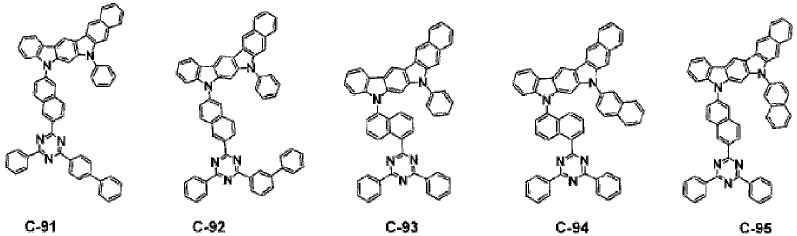

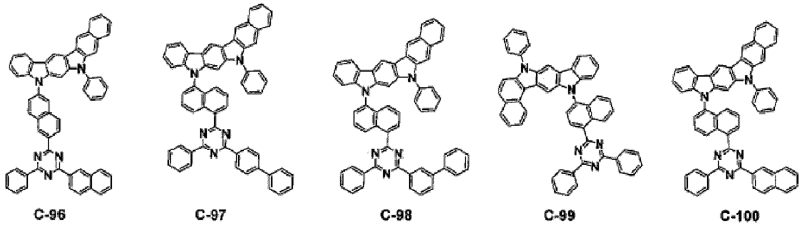

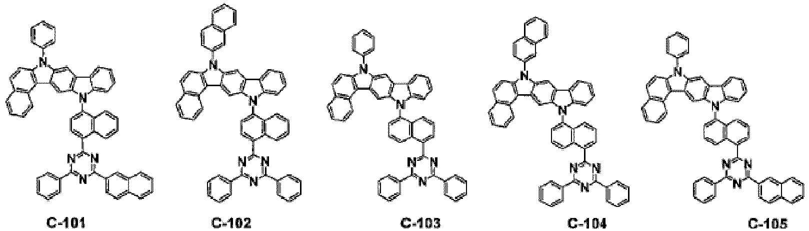

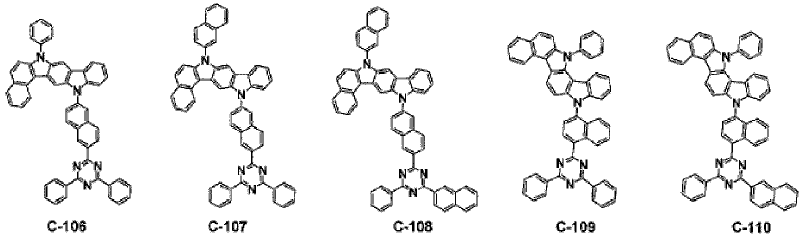

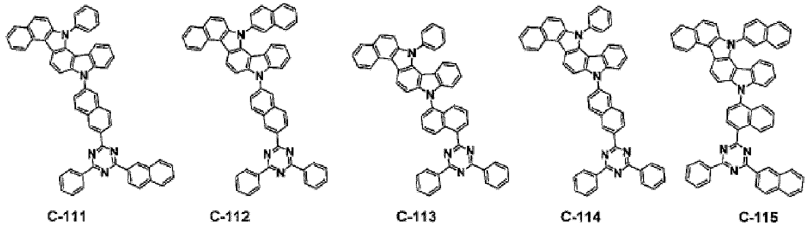

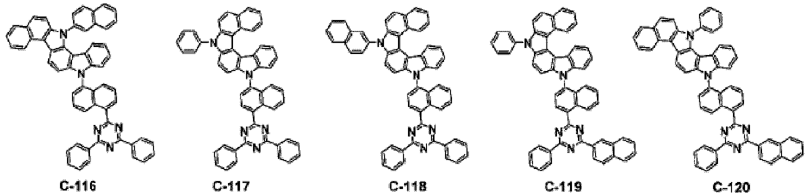

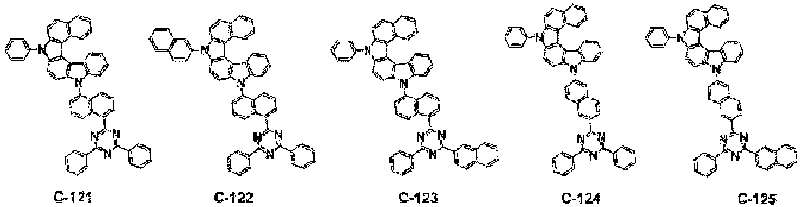

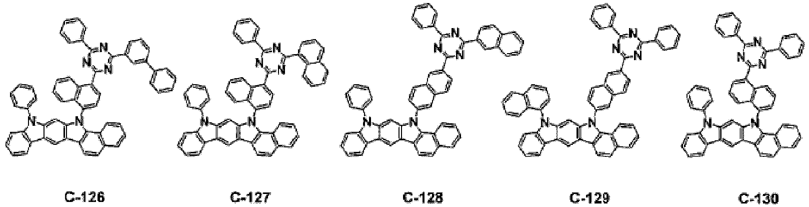

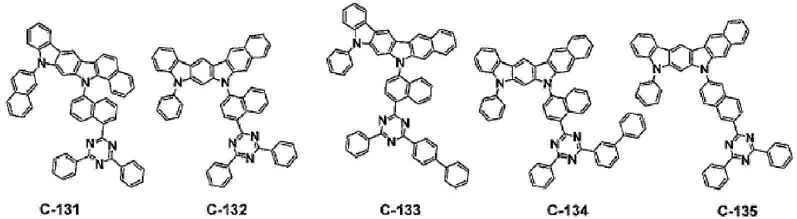

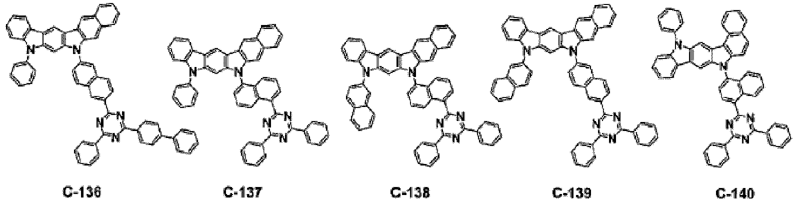

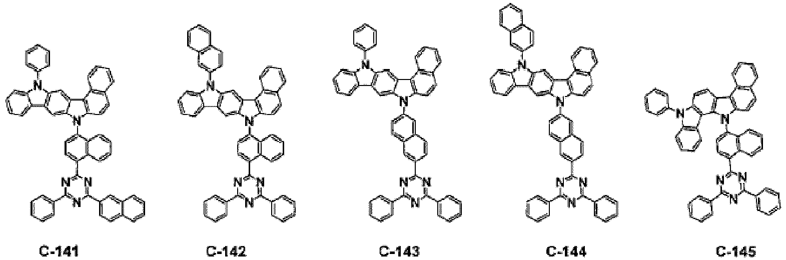

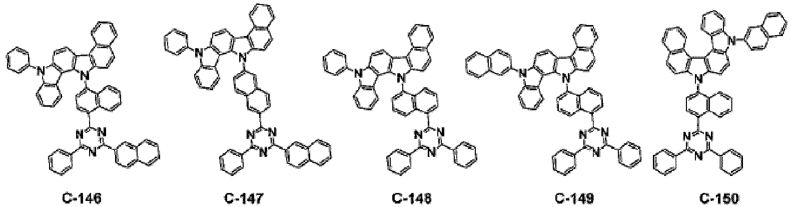

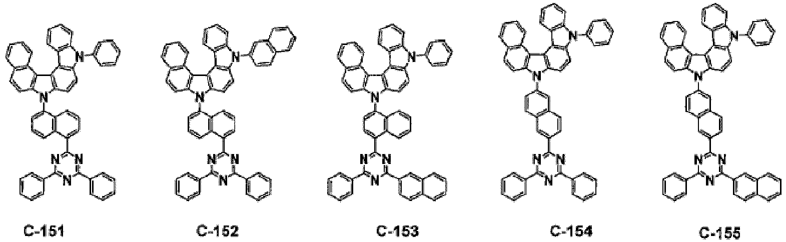

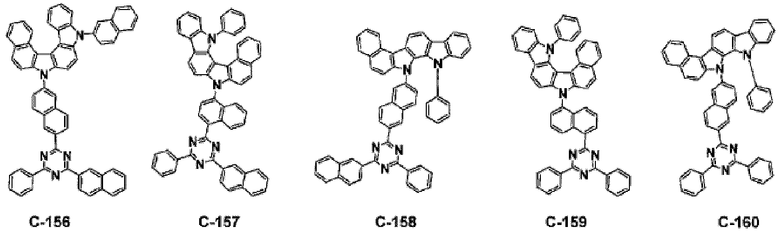

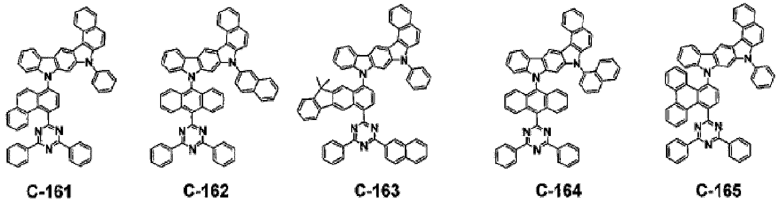

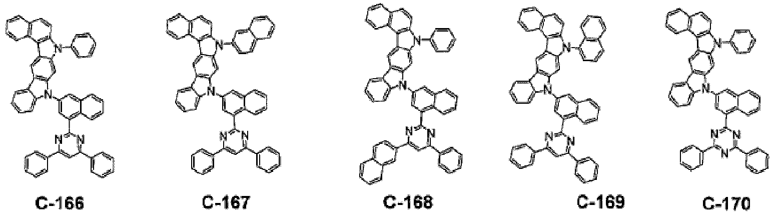

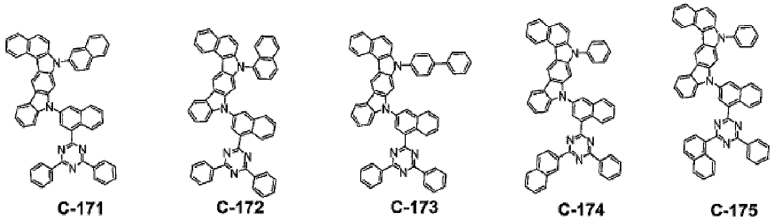

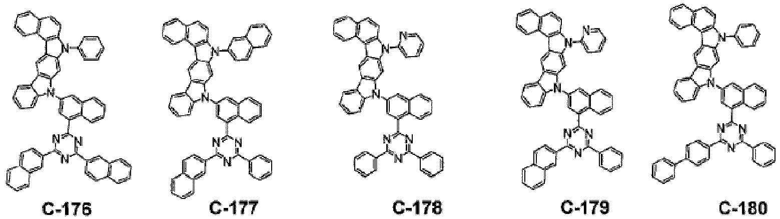

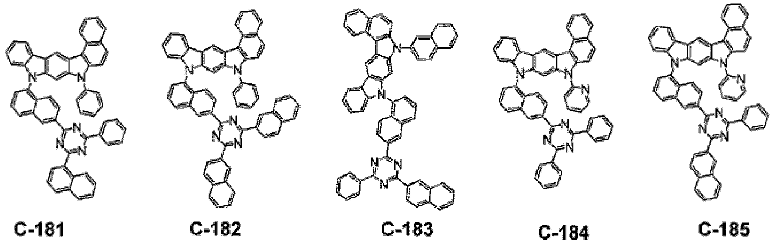

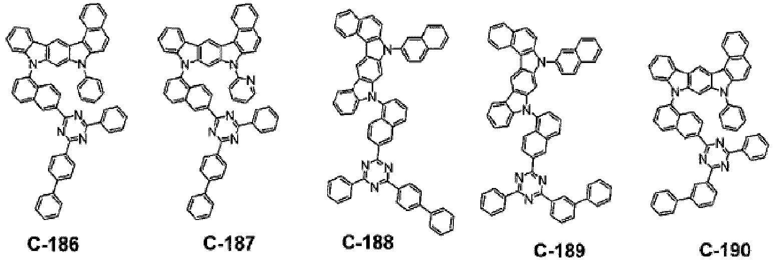

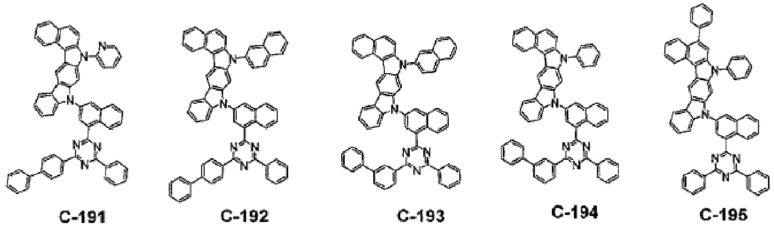

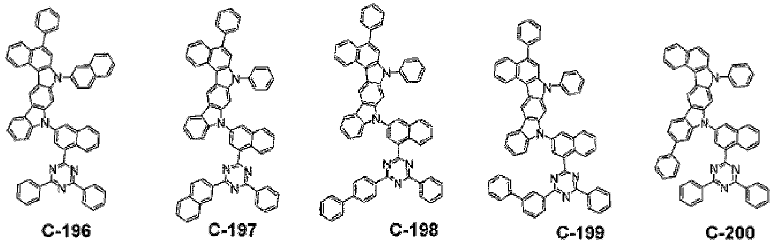

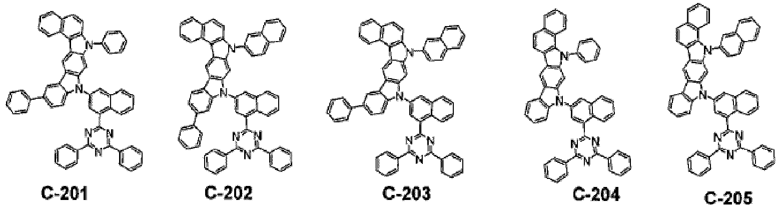

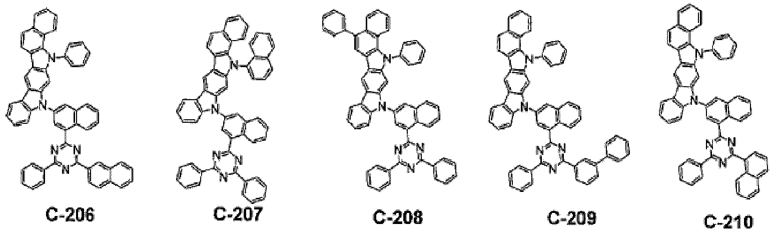

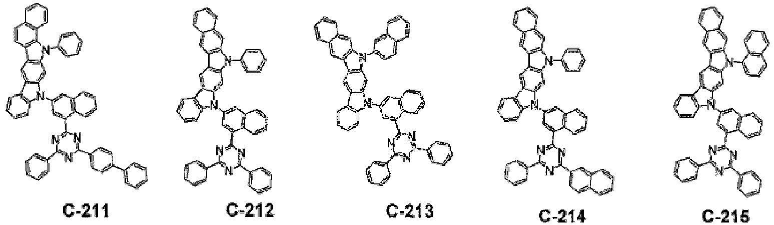

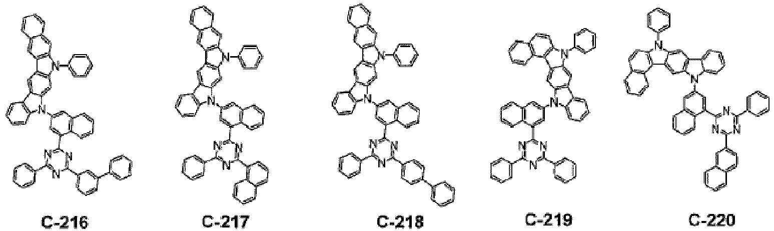

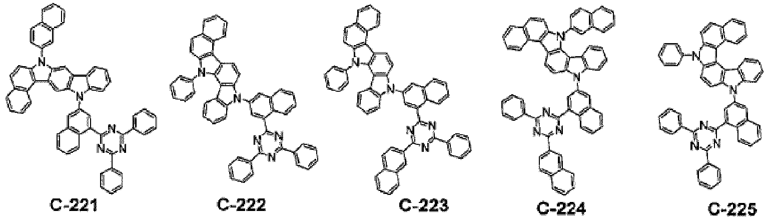

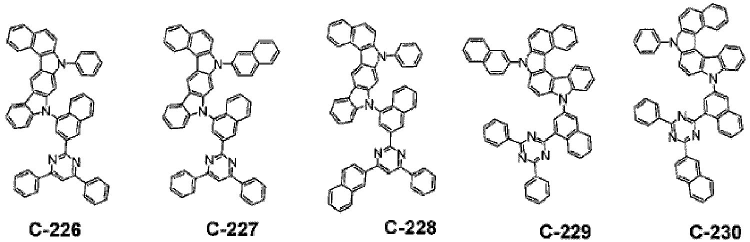

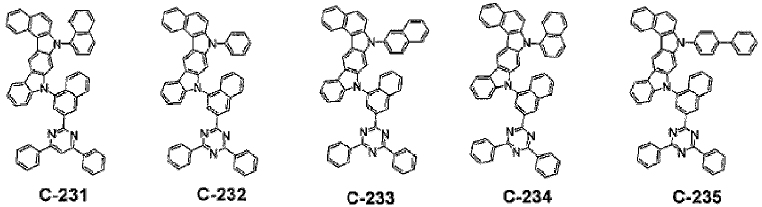

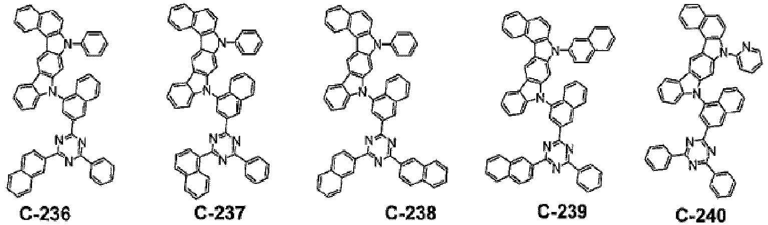

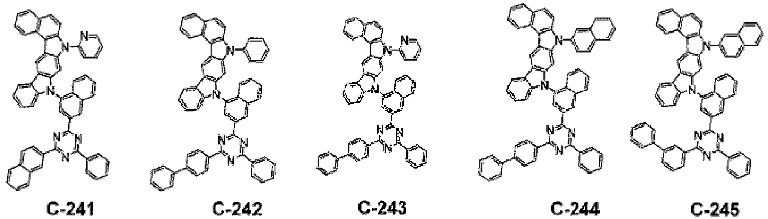

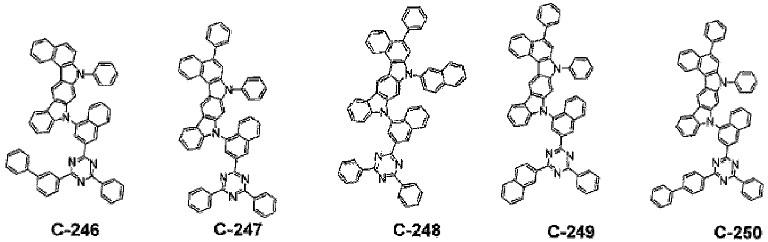

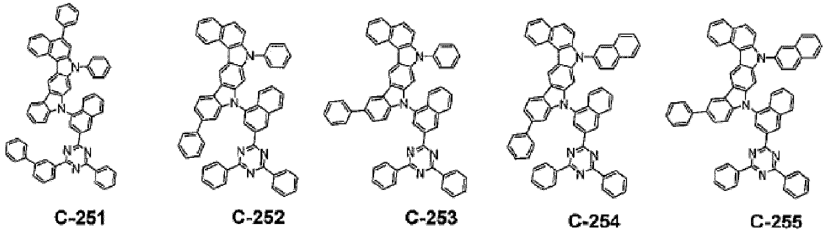

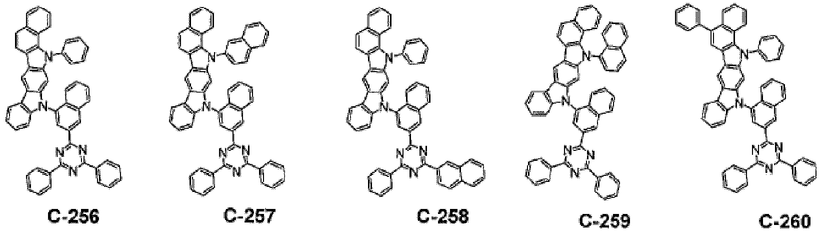

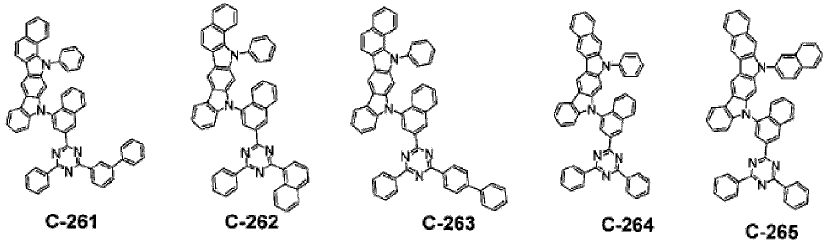

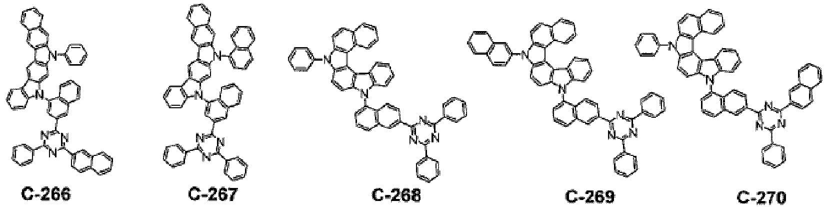

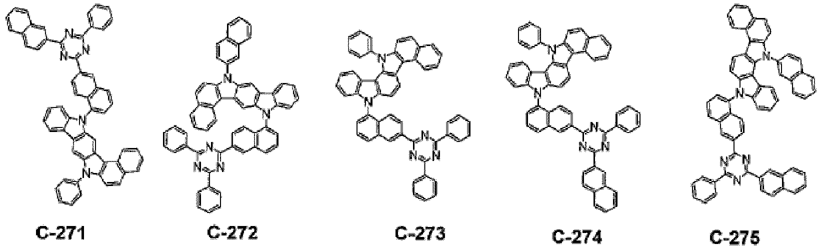

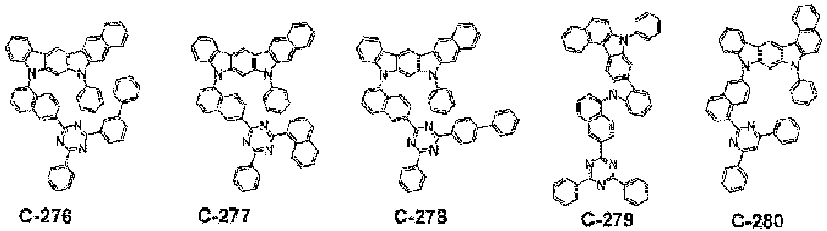

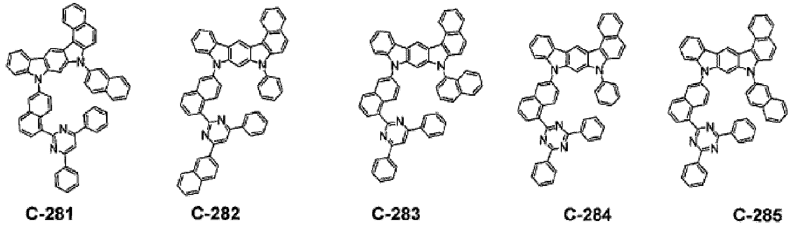

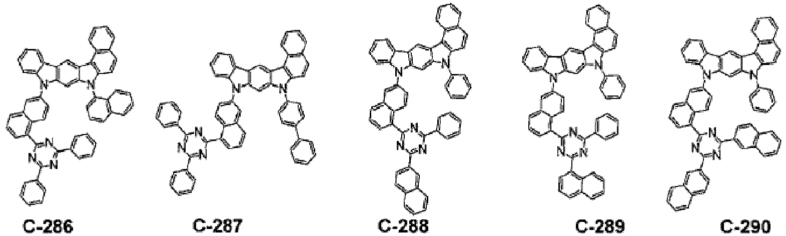

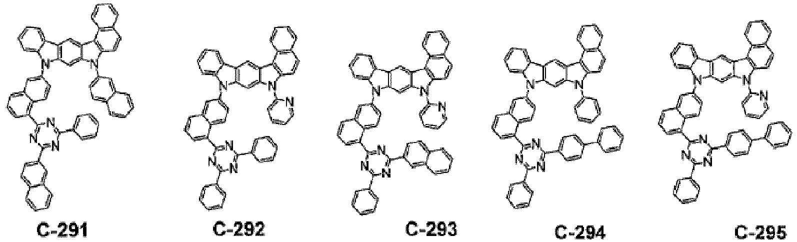

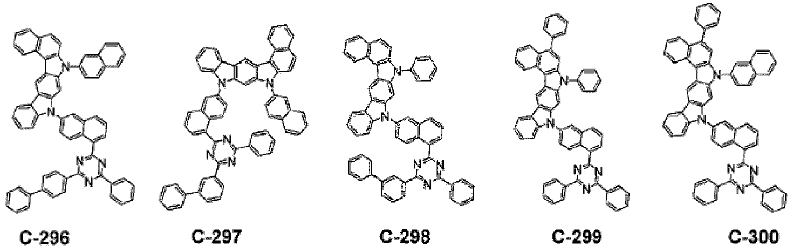

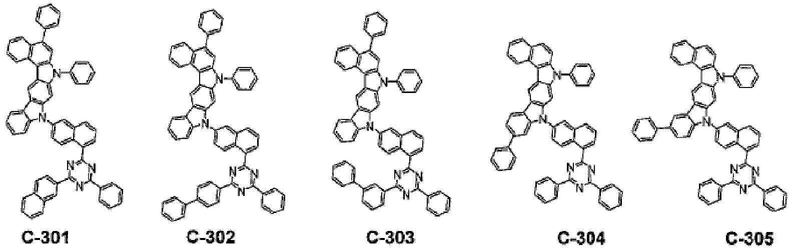

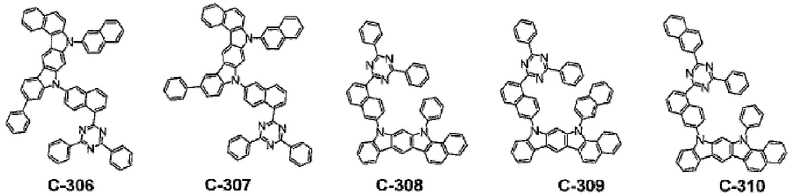

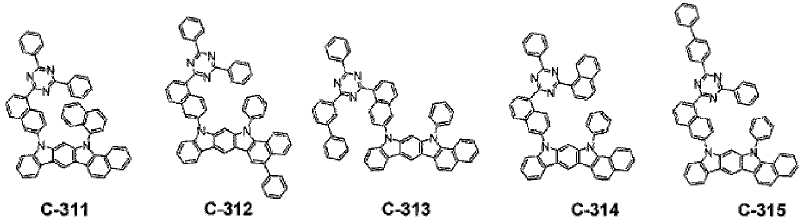

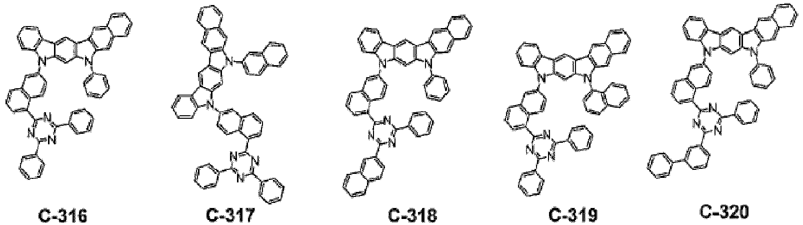

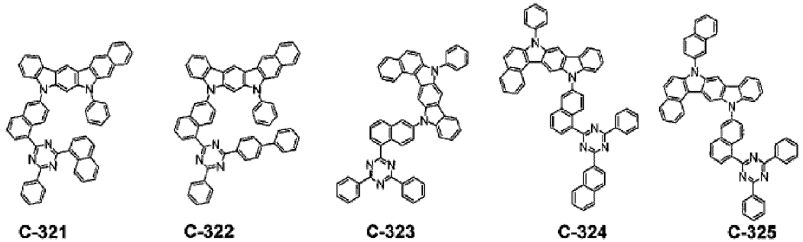

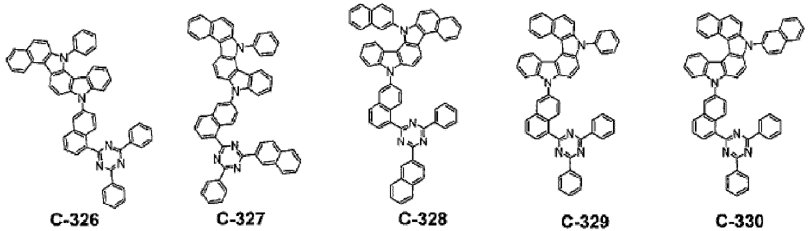

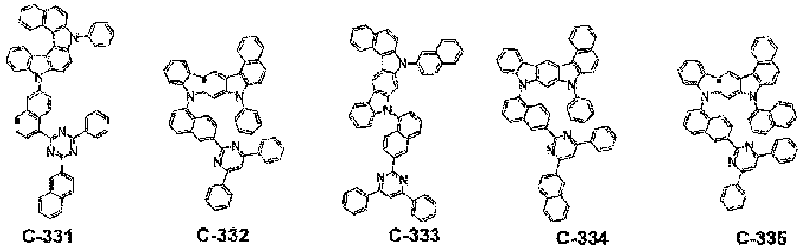

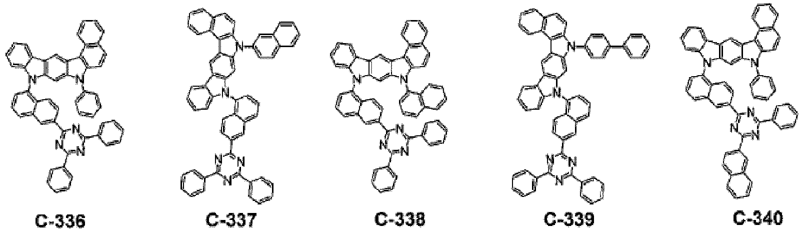

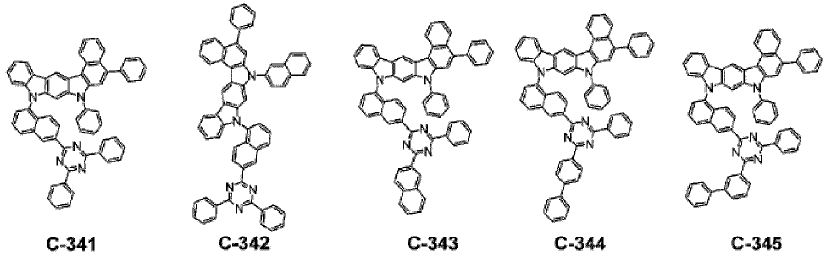

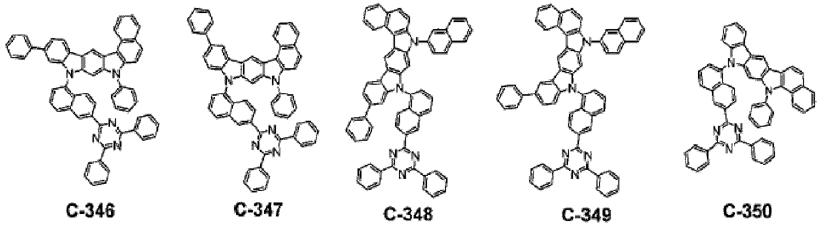

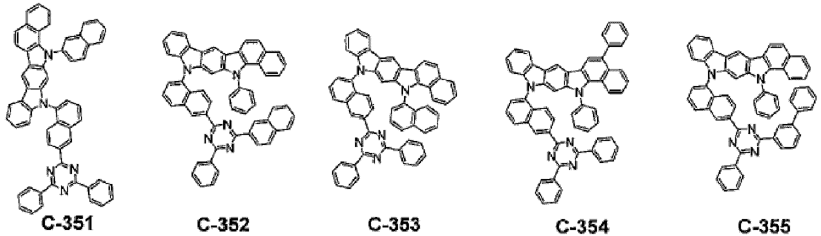

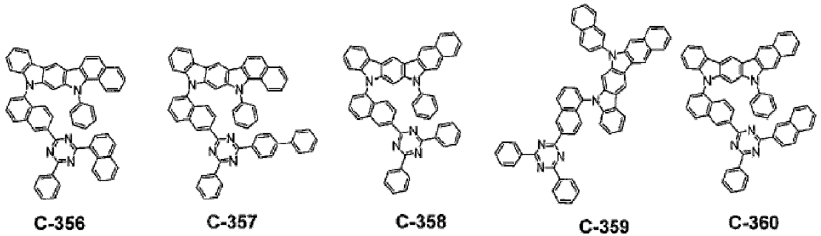

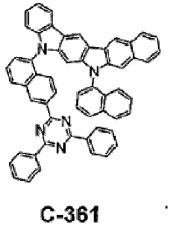

9. Organisches elektrolumineszentes Material, umfassend die organische elektrolumineszente Verbindung nach Anspruch 1.

10. Organisches elektrolumineszentes Material nach Anspruch 9, wobei das organische elektrolumineszente Material ein Wirtsmaterial ist, das eine Mehrzahl von Wirtsverbindungen umfasst, und mindestens eine der Wirtsverbindungen die organische elektrolumineszente Verbindung ist, die dargestellt ist durch Formel 1.

## Revendications

1. Composé organique électroluminescent représenté par la formule 1 suivante: 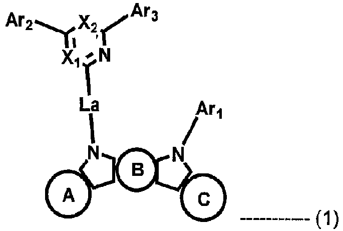 dans lequel:
X₁ et X₂, chacun indépendamment, représentent N ou CH, sous réserve qu'au moins un de X₁ et X₂ représente N,
La représente un cycle aromatique (C10-C30) divalent substitué ou non substitué, qui est un cycle condensé,
le cycle A et le cycle C, chacun indépendamment, représentent un cycle de benzène substitué ou non substitué ou un cycle de naphtalène substitué ou non substitué, sous réserve qu'au moins un du cycle A et du cycle C représente un cycle de naphtalène substitué ou non substitué,
le cycle B représente un cycle de benzène substitué ou non substitué,
Ar₁, Ar₂ et Ar₃, chacun indépendamment, représentent un hydrogène, un deutérium, un halogène, un cyano, un (C1-C30)alkyle substitué ou non substitué, un (C6-C30)aryle substitué ou non substitué, un hétéroaryle (de 3 à 30 membres) substitué ou non substitué, un (C3-C30)cycloalkyle substitué ou non substitué, un (C1-C30)alcoxy substitué ou non substitué, un tri(C1-C30)alkylsilyle substitué ou non substitué, un di(C1-C30)alkyl(C6-C30)arylsilyle substitué ou non substitué, un (C1-C30)alkyldi(C6-C30)arylsilyle substitué ou non substitué, un tri(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué, et
l'hétéroaryle contient au moins un hétéroatome choisi parmi B, N, O, S, Si et P.

2. Composé organique électroluminescent selon la revendication 1, dans lequel les substituants du (C1-C30)alkyle substitué, du (C6-C30)aryle substitué, de l'hétéroaryle (de 3 à 30 membres) substitué, du (C3-C30)cycloalkyle substitué, du (C1-C30)alcoxy substitué, du tri(C1-C30)alkylsilyle substitué, du di(Cl-C30)alkyl(C6-C30)arylsilyle substitué, du (C1-C30)alkyldi(C6-C30)arylsilyle substitué, du tri(C6-C30)arylsilyle substitué, du mono- ou di-(C1-C30)alkylamino substitué, du mono- ou di-(C6-C30)arylamino substitué, du (C1-C30)alkyl(C6-C30)arylamino substitué, du cycle aromatique (C10-C30) divalent substitué, du cycle de benzène substitué et du cycle de naphtalène substitué dans La, Ar₁, Ar₂, Ar₃, le cycle A, le cycle B et le cycle C, chacun indépendamment, sont au moins un choisi dans le groupe constitué de: un deutérium; un halogène; un cyano; un carboxyle; un nitro; un hydroxyle; un (C1-C30)alkyle; un halo(C1-C30)alkyle; un (C2-C30)alcényle; un (C2-C30)alcynyle; un (C1-C30)alcoxy; un (C1-C30)alkylthio; un (C3-C30)cycloalkyle; un (C3-C30)cycloalcényle; un hétérocycloalkyle (de 3 à 7 membres); un (C6-C30)aryloxy; un (C6-C30)arylthio; un hétéroaryle (de 5 à 30 membres) non substitué ou substitué avec un (C6-C30)aryle; un (C6-C30)aryle non substitué ou substitué avec un hétéroaryle (de 5 à 30 membres); un tri(C1-C30)alkylsilyle; un tri(C6-C30)arylsilyle; un di(Cl-C30)alkyl(C6-C30)arylsilyle; un (C1-C30)alkyldi(C6-C30)arylsilyle; un amino; un mono- ou di-(C1-C30)alkylamino; un mono- ou di-(C6-C30)arylamino non substitué ou substitué avec un (C1-C30)alkyle; un (C1-C30)alkyl(C6-C30)arylamino; un (C1-C30)alkylcarbonyle; un (C1-C30)alcoxycarbonyle; un (C6-C30)arylcarbonyle; un di(C6-C30)arylboronyle; un di(C1-C30)alkylboronyle; un (C1-C30)alkyl(C6-C30)arylboronyle; un (C6-C30)aryl(Cl-C30)alkyle; et un (C1-C30)alkyl(C6-C30)aryle.

3. Composé organique électroluminescent selon la revendication 1,
dans lequel la formule 1 est représentée par l'une quelconque des formules 2 à 5 suivantes: 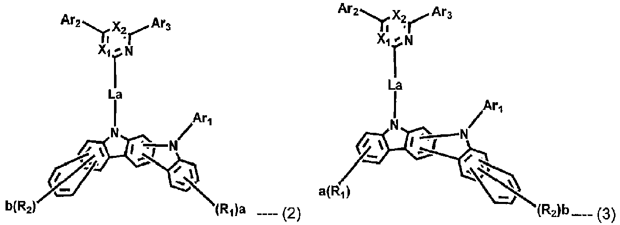 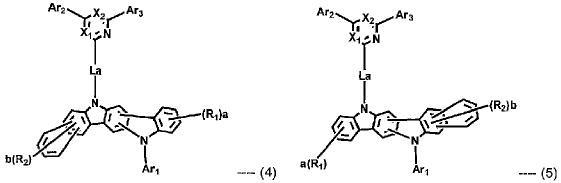 dans lequel:
R₁ et R₂, chacun indépendamment, représentent un deutérium, un halogène, un cyano, un (C1-C30)alkyle substitué ou non substitué, un (C6-C30)aryle substitué ou non substitué, un hétéroaryle (de 3 à 30 membres) substitué ou non substitué, un (C3-C30)cycloalkyle substitué ou non substitué, un (C1-C30)alcoxy substitué ou non substitué, un tri(C1-C30)alkylsilyle substitué ou non substitué, un di(C1-C30)alkyl(C6-C30)arylsilyle substitué ou non substitué, un (C1-C30)alkyldi(C6-C30)arylsilyle substitué ou non substitué, un tri(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué,
a représente un nombre entier de 0 à 4, et b représente un nombre entier de 0 à 6;
où si a et b, chacun indépendamment, représentent un nombre entier de 2 ou plus, R₁ et R₂ peuvent chacun être identiques ou différents,
l'hétéroaryle contient au moins un hétéroatome choisi parmi B, N, O, S, Si et P, et X₁, X₂, La, Ar₁, Ar₂ et Ar₃ sont tels que définis dans la revendication 1.

4. Composé organique électroluminescent selon la revendication 1,
dans lequel la formule 1 est représentée par l'une quelconque des formules 6 à 17 suivantes: 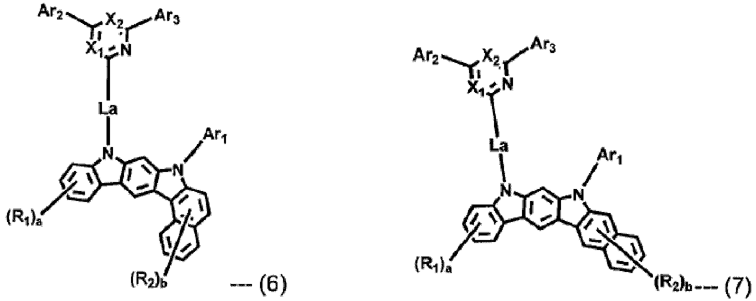 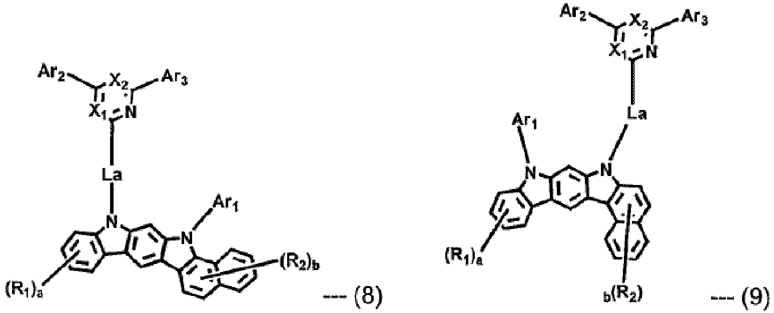 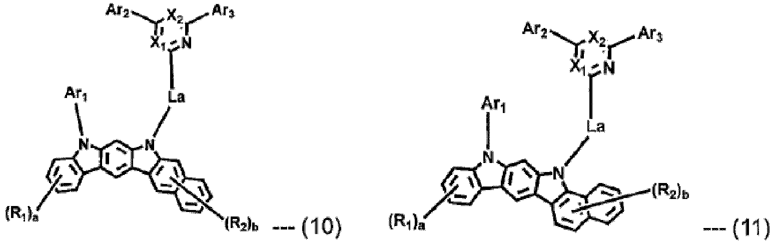 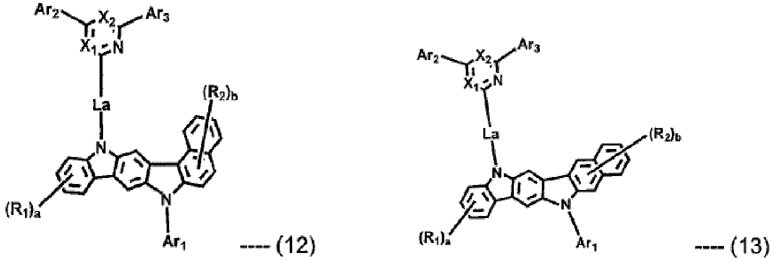 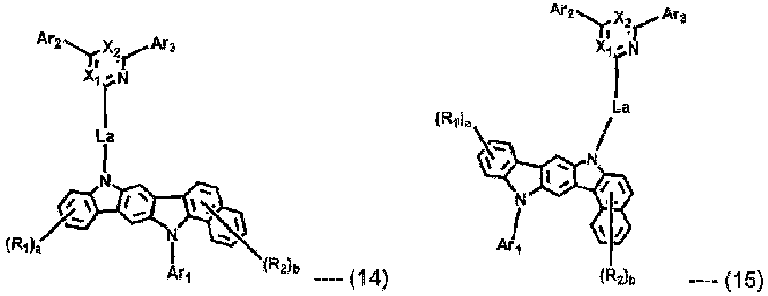 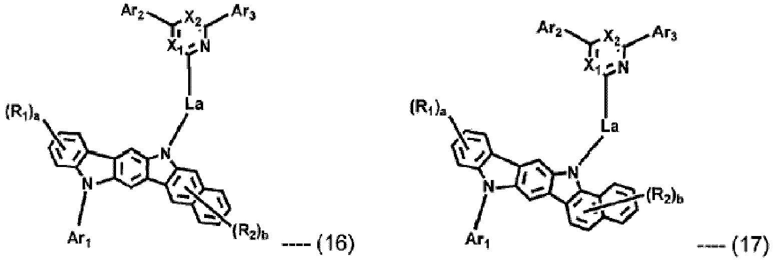 dans lequel:
R₁ et R₂, chacun indépendamment, représentent un deutérium, un halogène, un cyano, un (C1-C30)alkyle substitué ou non substitué, un (C6-C30)aryle substitué ou non substitué, un hétéroaryle (de 3 à 30 membres) substitué ou non substitué, un (C3-C30)cycloalkyle substitué ou non substitué, un (C1-C30)alcoxy substitué ou non substitué, un tri(C1-C30)alkylsilyle substitué ou non substitué, un di(C1-C30)alkyl(C6-C30)arylsilyle substitué ou non substitué, un (C1-C30)alkyldi(C6-C30)arylsilyle substitué ou non substitué, un tri(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué,
a représente un nombre entier de 0 à 4, et b représente un nombre entier de 0 à 6;
où si a et b, chacun indépendamment, représentent un nombre entier de 2 ou plus, R₁ et R₂ peuvent chacun être identiques ou différents,
l'hétéroaryle contient au moins un hétéroatome choisi parmi B, N, O, S, Si et P, et X₁, X₂, La, Ar₁, Ar₂ et Ar₃ sont tels que définis dans la revendication 1.

5. Composé organique électroluminescent selon la revendication 1,
dans lequel La représente un naphtylène, un anthracénylène, un triphénylénylène, un diméthylbenzofluorénylène ou un phénanthrénylène.

6. Composé organique électroluminescent selon la revendication 1,
dans lequel l'un quelconque du cycle A et du cycle C représente un cycle de naphtalène substitué ou non substitué et l'autre représente un cycle de benzène substitué ou non substitué; et Ar₁ à Ar₃, chacun indépendamment, représentent un (C6-C25)aryle substitué ou non substitué ou un hétéroaryle (de 5 à 25 membres) substitué ou non substitué.

7. Composé organique électroluminescent selon la revendication 3,
dans lequel R₁ et R₂, chacun indépendamment, représentent un (C1-C20)alkyle substitué ou non substitué, un (C6-C25)aryle substitué ou non substitué ou un hétéroaryle (de 5 à 25 membres) substitué ou non substitué; et a et b, chacun indépendamment, représentent 0 ou 1.

8. Composé organique électroluminescent selon la revendication 1,
dans lequel le composé représenté par la formule 1 est choisi dans le groupe constitué de:

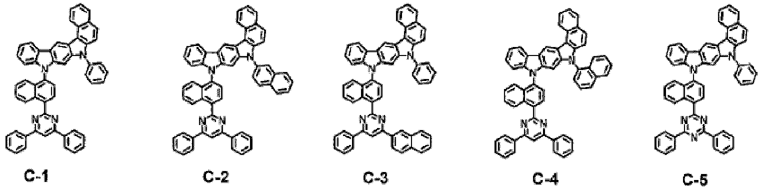

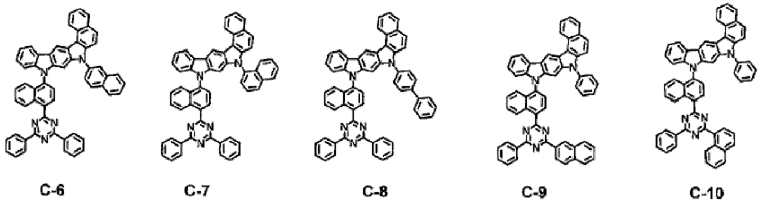

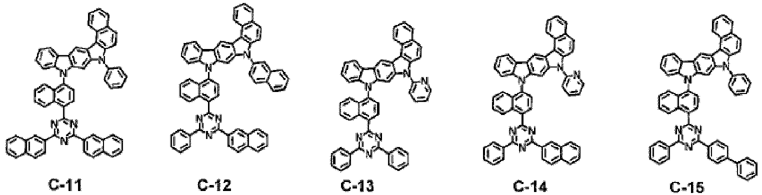

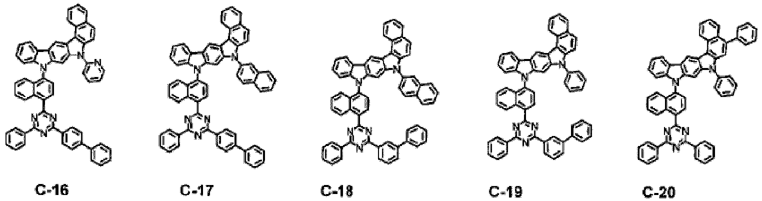

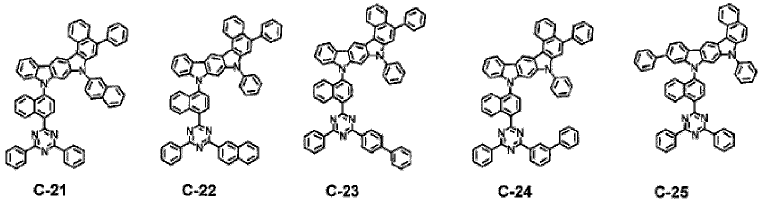

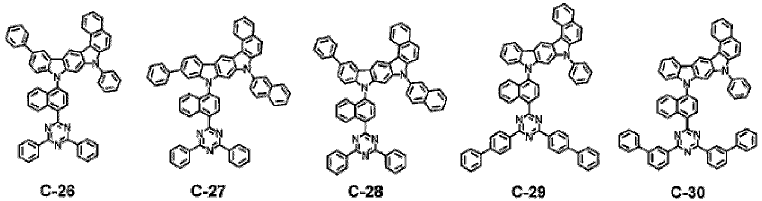

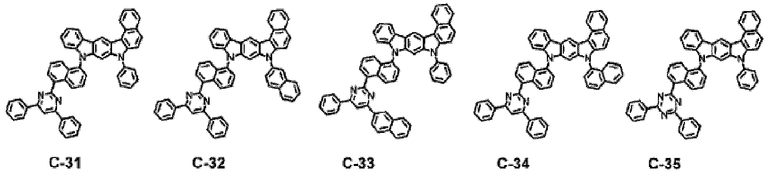

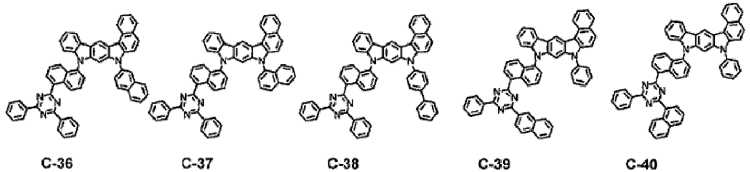

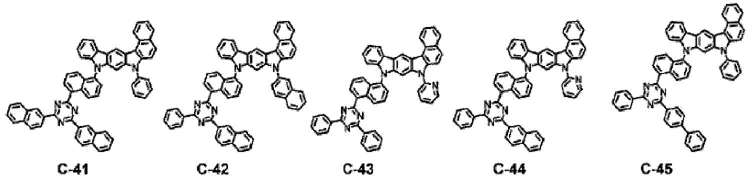

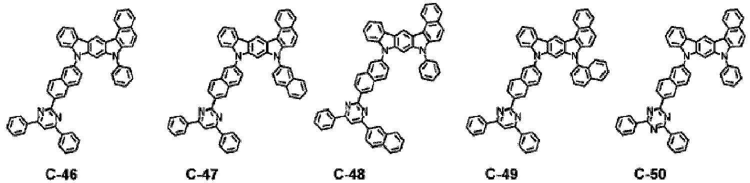

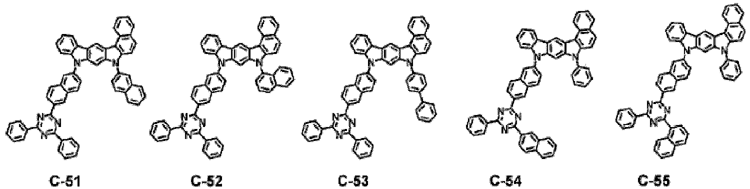

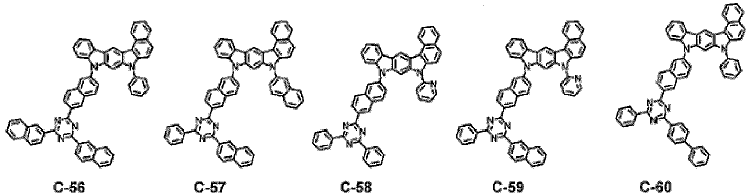

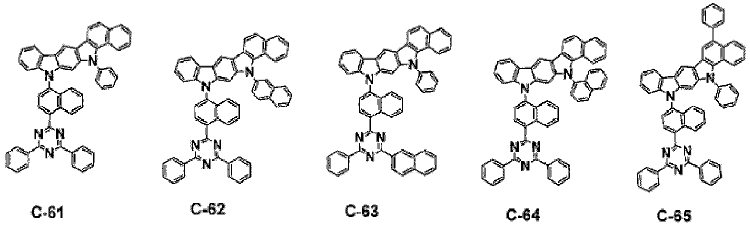

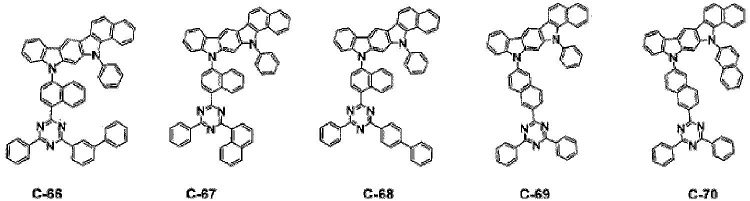

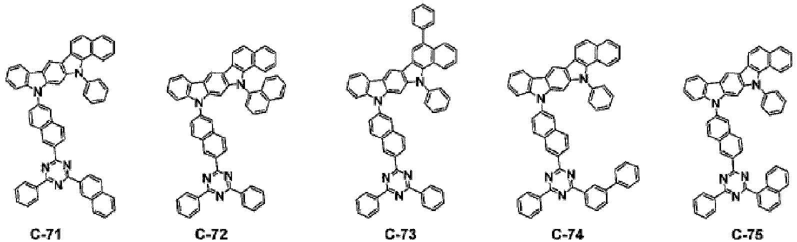

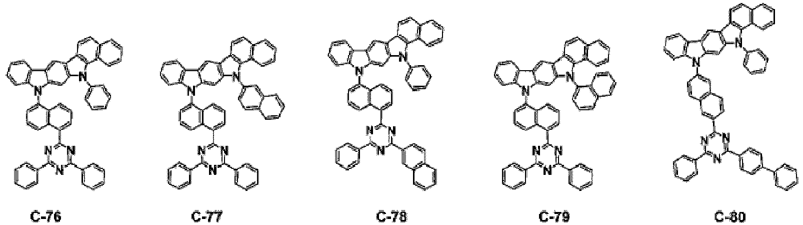

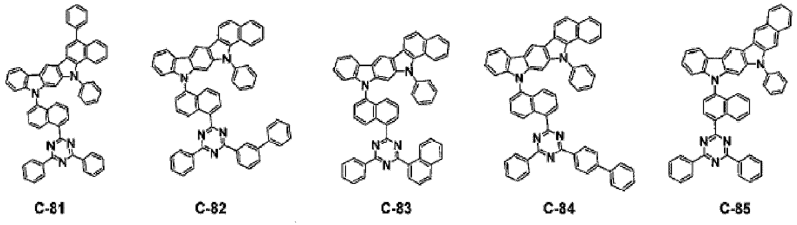

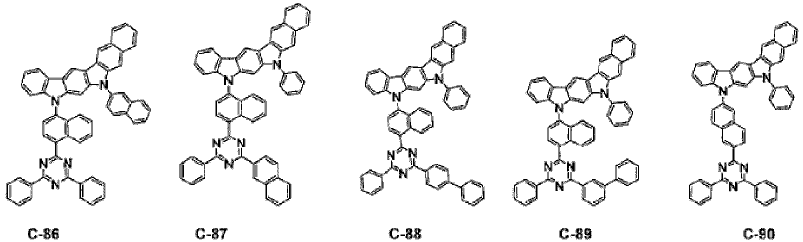

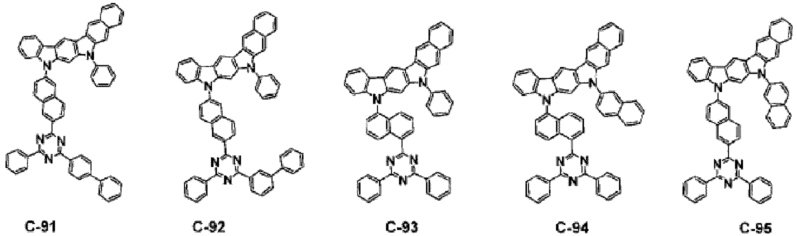

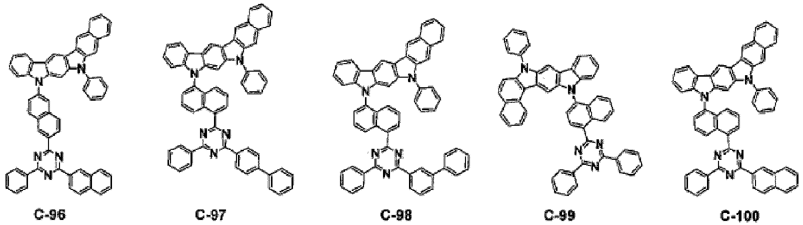

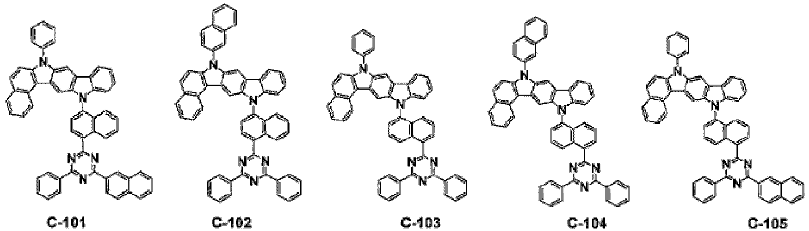

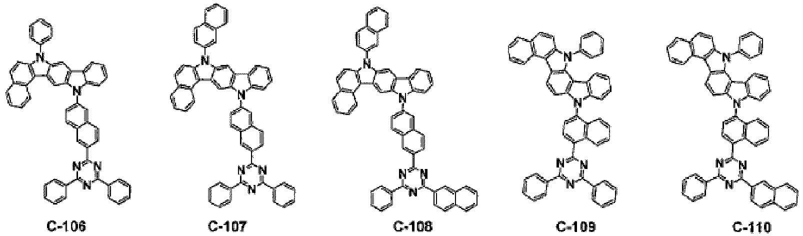

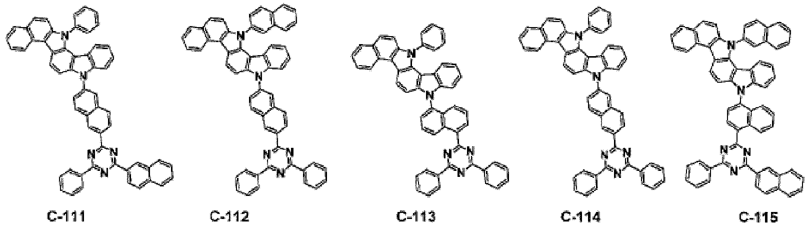

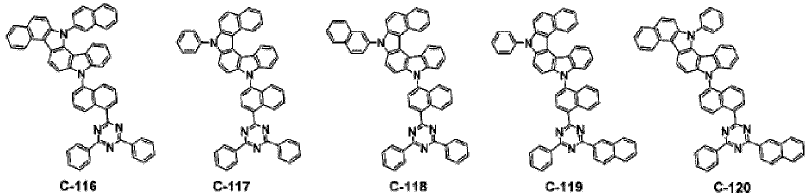

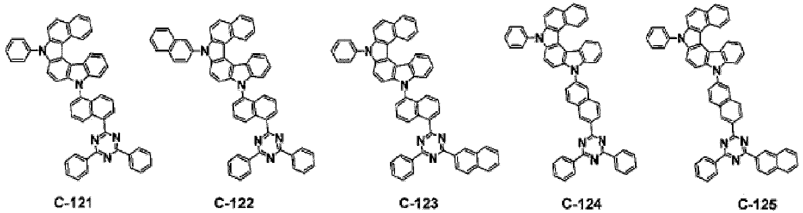

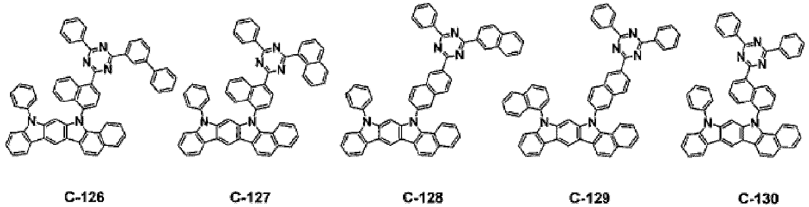

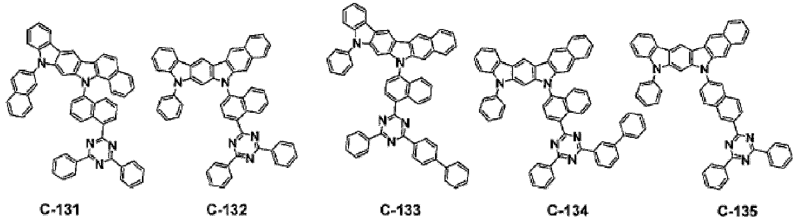

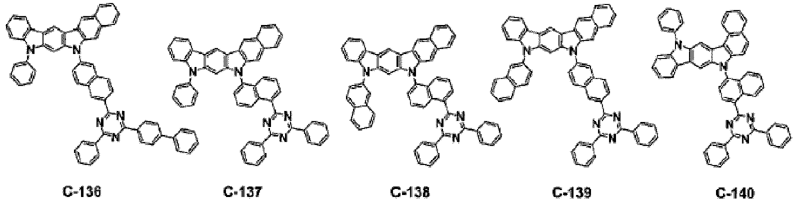

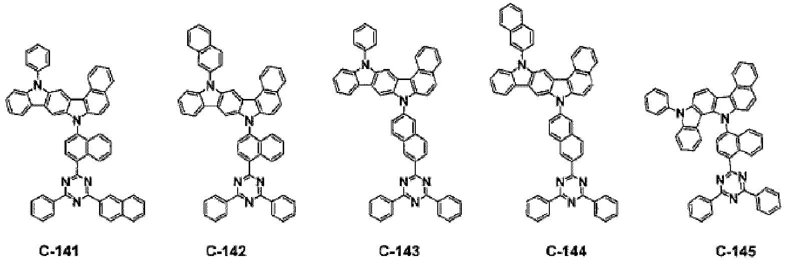

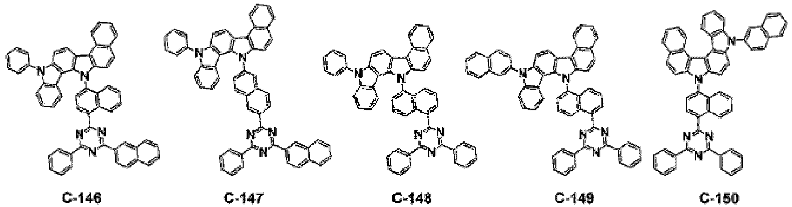

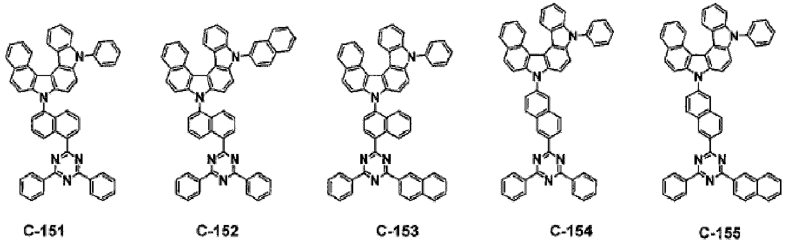

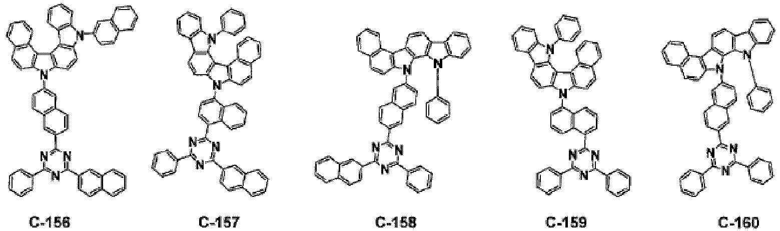

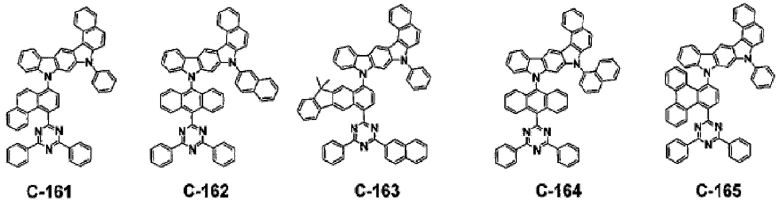

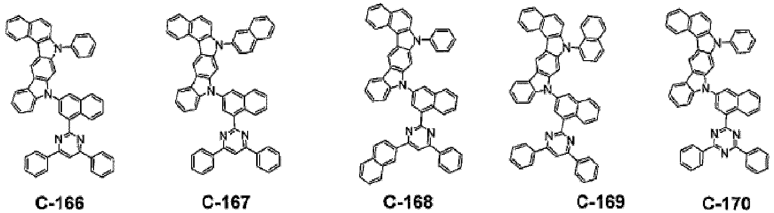

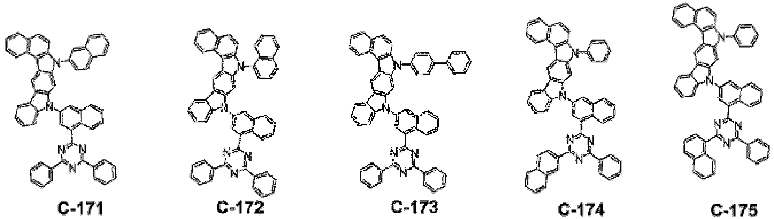

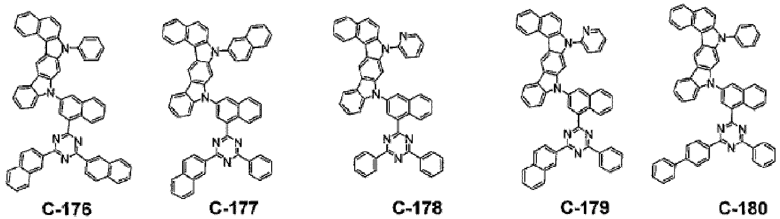

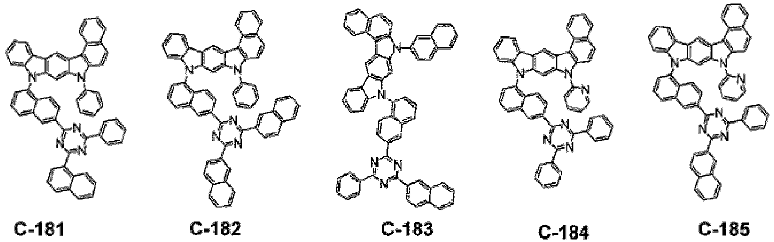

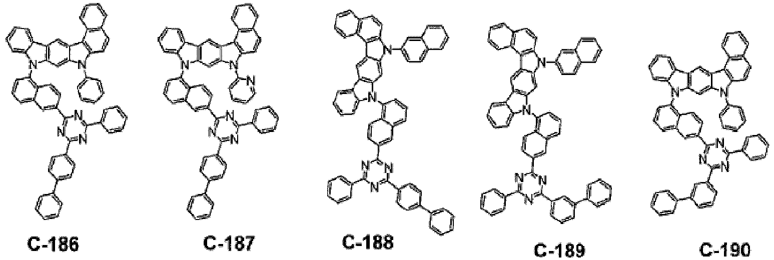

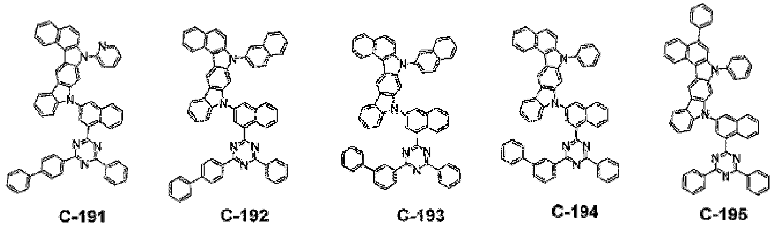

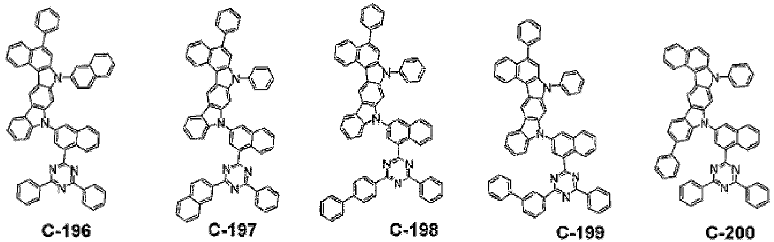

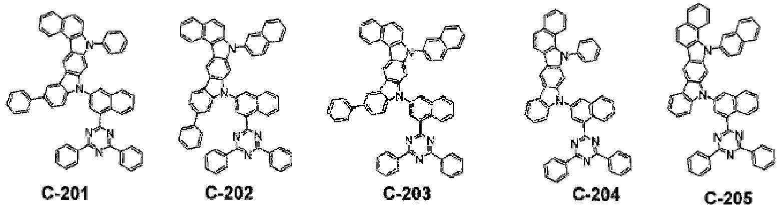

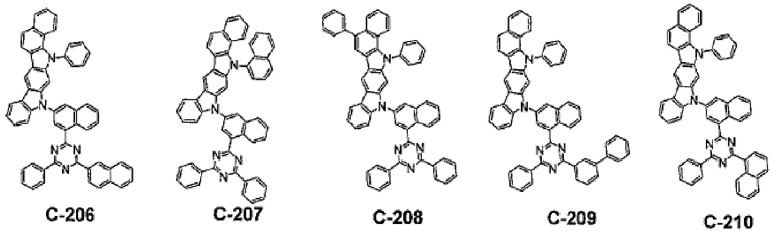

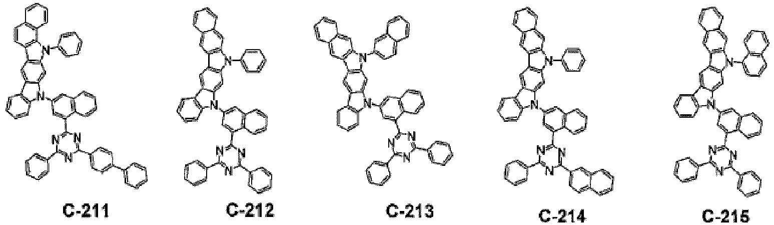

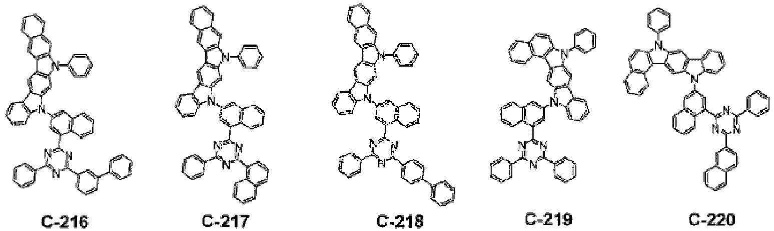

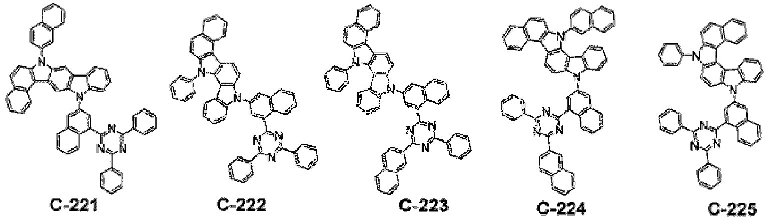

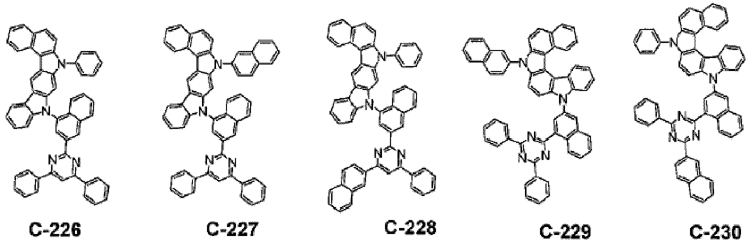

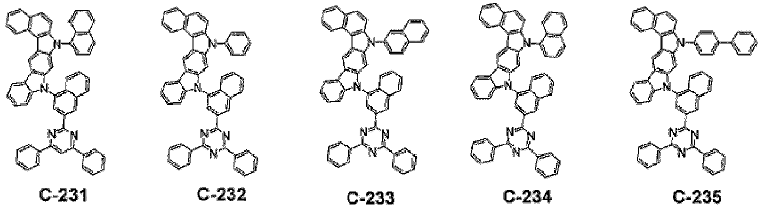

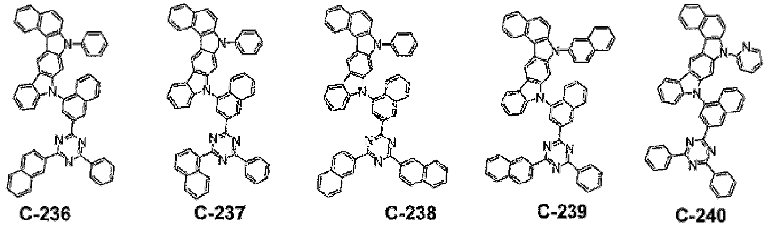

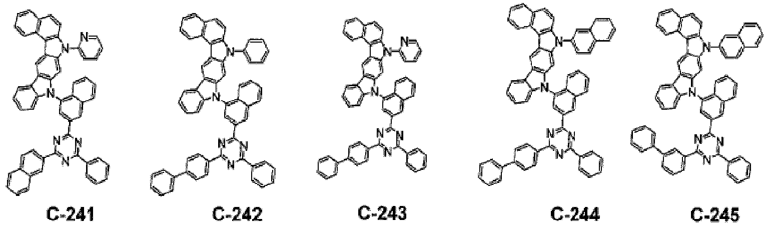

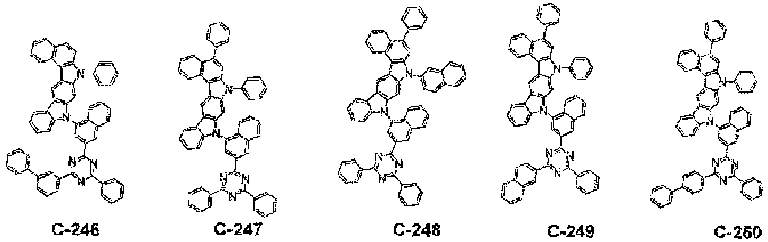

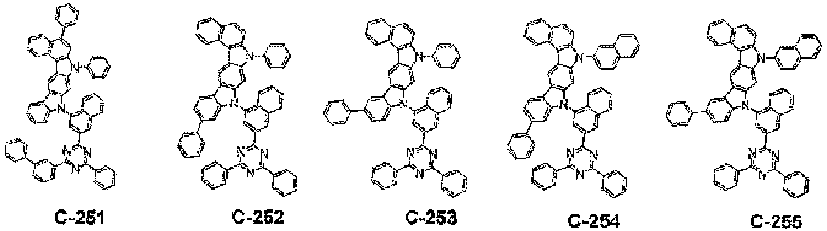

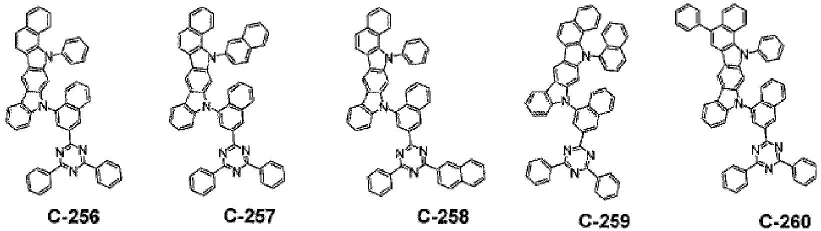

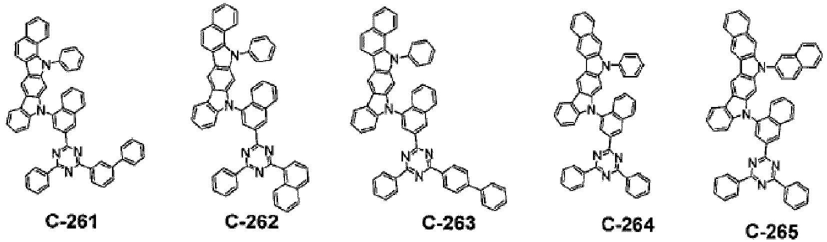

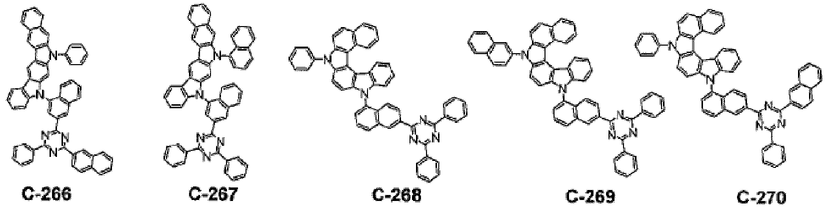

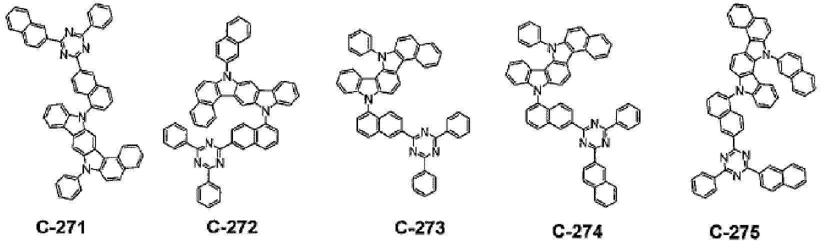

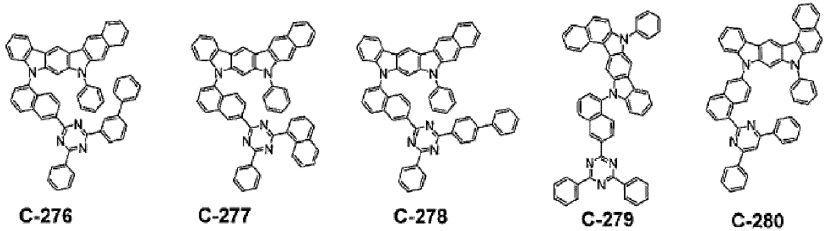

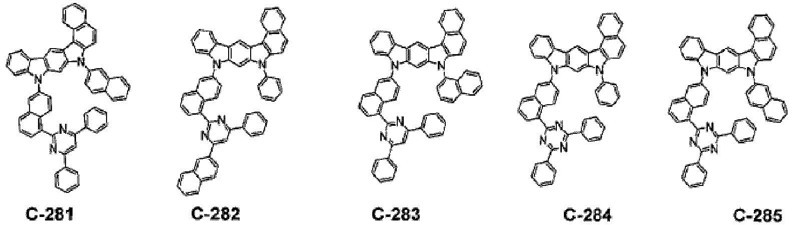

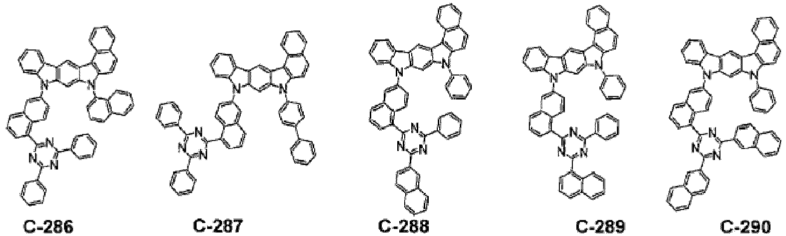

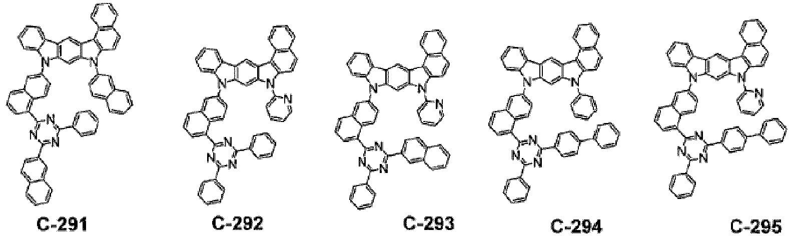

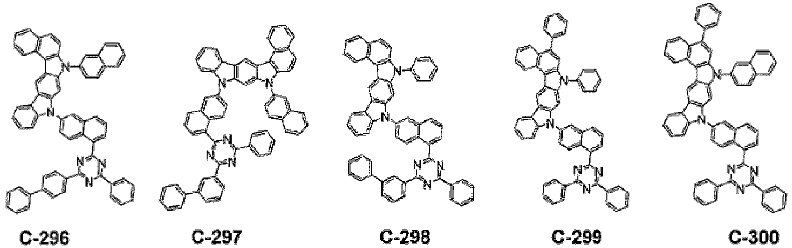

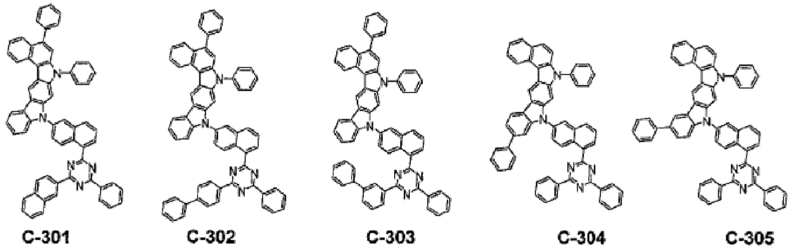

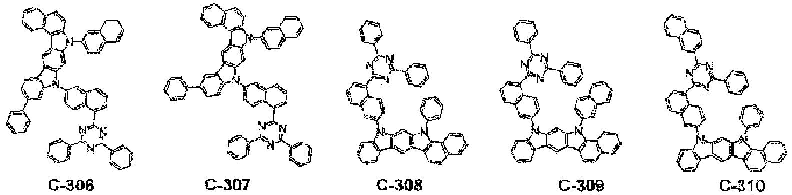

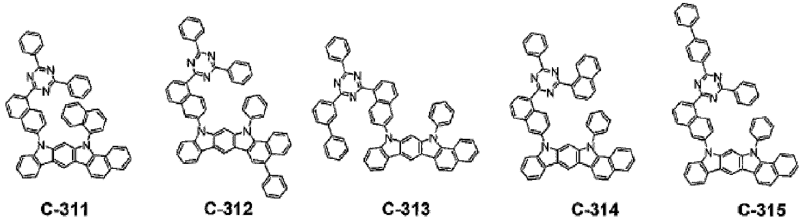

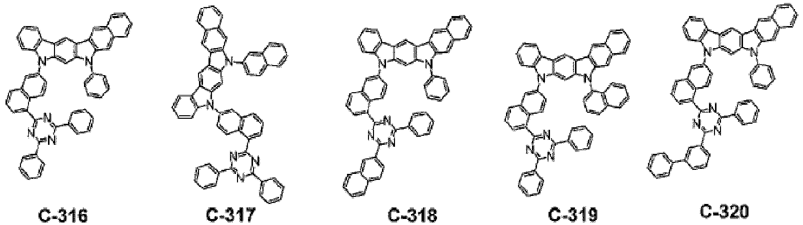

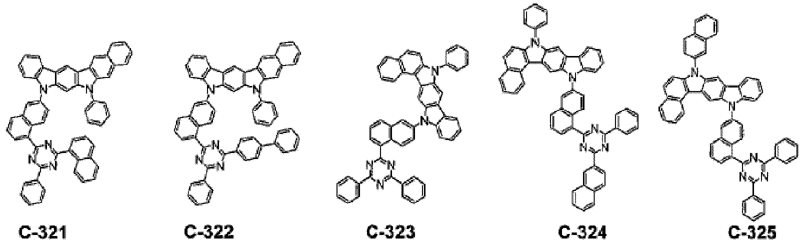

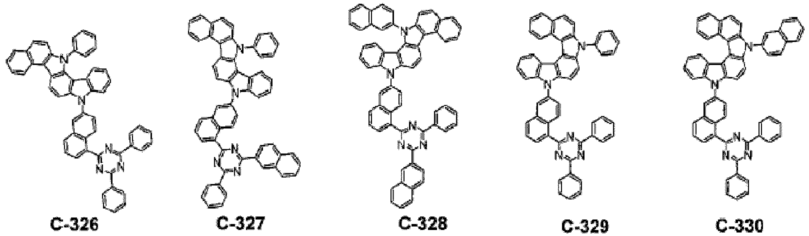

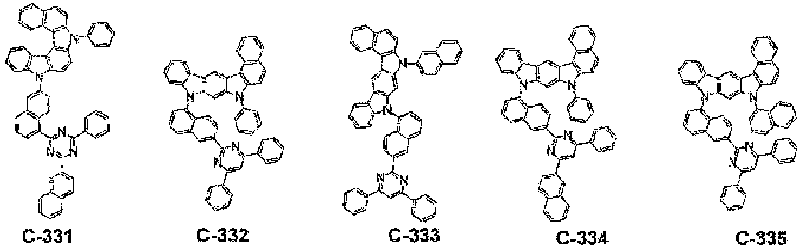

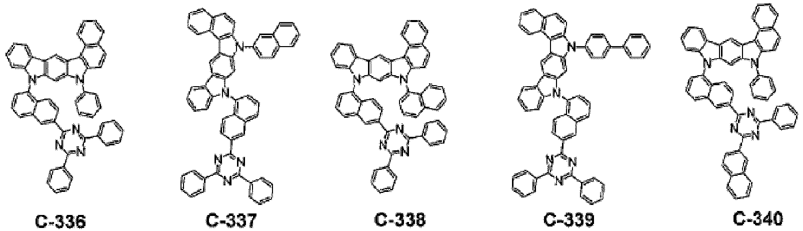

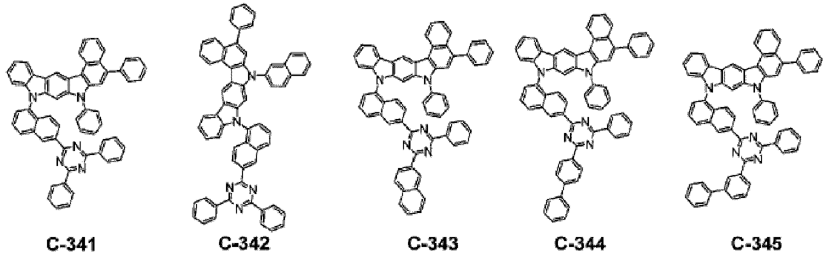

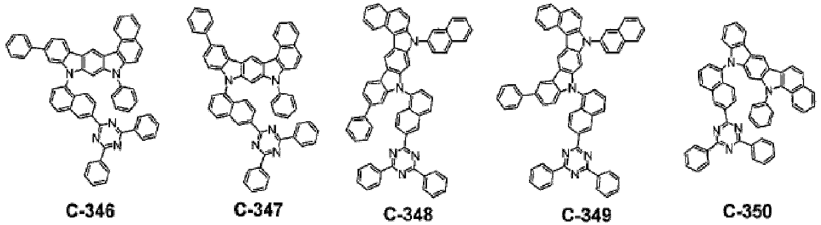

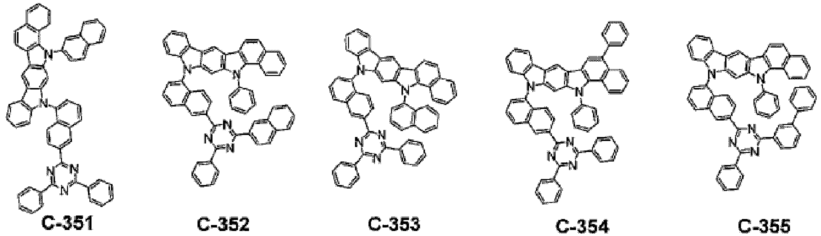

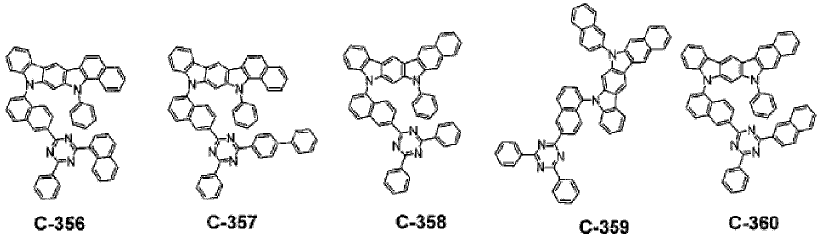

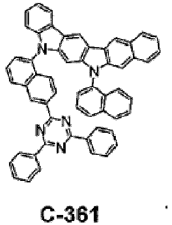

9. Matériau organique électroluminescent comprenant le composé organique électroluminescent selon la revendication 1.

10. Matériau organique électroluminescent selon la revendication 9, dans lequel le matériau organique électroluminescent est un matériau hôte comprenant une pluralité de composés hôtes et au moins un des composés hôtes est le composé organique électroluminescent représenté par la formule 1.
